# EUROPEAN PATENT APPLICATION

(11) **EP 4 668 282 A1**
(43) Date of publication of application: **24.12.2025**
(21) Application number: 24843565.3
(22) Date of filing: 19.07.2024
(51) Int. Cl.: G16C 20/70, G16C 20/30, G16C 20/40, G16C 20/50, G16C 20/80, G06N 20/00

(54) **METHOD AND SYSTEM FOR GENERATING ANSWERS**

(30) Priority: 19.07.2023 KR 20230093644
(71) Applicant: LG Management Development Institute Co., Ltd., Seoul 07336 (KR)
(72) Inventor: HORMAZABAL, Rodrigo, Incheon 21022 (KR); BERTENS, Paul, Seoul 07788 (KR); HAN, Se Hui, Seoul 07665 (KR); RYOO, Kwang Rok, Seoul 07336 (KR); KIM, Ji Ye, Seoul 07336 (KR); LEE, Su Min, Seoul 07336 (KR); JEONG, Dae Woong, Seoul 07336 (KR); KO, Sung Moon, Seoul 07336 (KR); JO, Ah Ra, Seoul 07336 (KR); LEE, Seung Jun, Seoul 07336 (KR)
(74) Representative: BCKIP Part mbB
(86) International application number: PCT/KR2024/010507
(87) International publication number: WO 2025/018854

(57) **Abstract**

The present invention relates to an answer generation method and system. More specifically, the present invention relates to an answer generation method and system using an ultra-large foundation model. The present invention relates to an answer generation platform based on an ultra-large foundation model. In addition, the present invention relates to a chemical reaction prediction system, a control method thereof, and a learning method of a chemical reaction prediction system. More specifically, the present invention relates to a chemical reaction prediction system that performs forward reaction prediction based on an electron flow, a control method thereof, and a learning method of a chemical reaction prediction system.

## Description

### [Technical Field]

Various embodiments disclosed in the present document relate to an answer generation method and system, and provides an answer generation method and system using a generative model or a foundation model. The present invention relates to an answer generation platform based on an ultra-large foundation model. In addition, the present invention relates to a chemical reaction prediction system, a control method thereof, and a learning method of a chemical reaction prediction system. More specifically, the present invention relates to a chemical reaction prediction system that performs forward reaction prediction based on an electron flow, a control method thereof, and a learning method of a chemical reaction prediction system.

### [Background Art]

Recently, there has been a rapid increase in cases where artificial intelligence, especially deep learning, which extracts data characteristics using deep neural network structures, has achieved excellent results in various fields such as voice recognition, image recognition, natural language processing, and autonomous driving.

With the development of such deep learning technology, generative artificial intelligence (generative AI) technology is recently receiving attention. More specifically, generative AI models may generate new data in various forms, such as text, images, and voices, from given data, and provide different levels of application potential from simply classifying or predicting existing data.

In other words, as sentences, images, voices, etc., that were previously created by humans may be automatically generated using generative artificial intelligence models, services (e.g., ChatGPT) using generative artificial intelligence has shown greater activity and accuracy than the existing chatbot services and is receiving great attention worldwide.

Meanwhile, attempts are continuously being made to solve various scientific problems in the field of natural sciences (e.g., physics, chemistry, biology, etc.). For example, researches are actively being conducted to design new materials or develop new drugs, and these researches are playing an important role in future technological advancement and industrial innovation.

In this regard, organic synthesis is one of the important tasks in new drug development and materials science, and predicting the results of chemical reactions is very important in designing new molecules. However, it takes a lot of time and cost for related researchers to directly perform chemical synthesis such as molecular synthesis.

Accordingly, researches are actively being conducted on methods for increasing the efficiency of natural science research based on generative artificial intelligence.

### [Disclosure]

### [Technical Problem]

The present invention provides an answer generation method and system capable of suggesting an optimal research method to researchers in the field of natural sciences.

More specifically, the present invention provides an answer generation method and system capable of minimizing the risk of failure in natural science research to increase the efficiency of natural science research.

In addition, the present invention provides an answer generation method and system capable of solving time and cost problems required for material research and development and increasing the efficiency of material research and development.

In addition, the present invention provides a chemical reaction prediction model capable of predicting results of various types of chemical reactions by understanding chemical reaction mechanisms.

More specifically, the present invention is to provide a chemical reaction prediction model based on an electron flow using graph diffusion in which both input and output structures are formed in a graph space.

In addition, the present invention provides a learning method of a chemical reaction prediction model capable of understanding chemical reaction mechanisms well and generating more accurate and predicted results of interpretable chemical reactions.

### [Technical Solution]

An answer generation method performed by cooperation of a memory and at least one processor according to various embodiments disclosed in the present document may include: specifying a plurality of molecular structures to be predicted based on user input received through a service page; storing information on the plurality of molecular structures in the memory; acquiring molecular graphs for the plurality of molecular structures by converting atoms into nodes and bonds between the atoms into edges based on the plurality of molecular structures stored in the memory; receiving a user query for chemical reaction prediction related to the plurality of molecular structures through the service page; processing the molecular graphs for the plurality of molecular structures as an input of a chemical reaction prediction model so that a chemical reaction corresponding to the user query is predicted; performing chemical reaction prediction on the plurality of molecular structures in the chemical reaction prediction model; acquiring a product of the chemical reaction prediction for the plurality of structures from the chemical reaction prediction model; and generating an answer to the user query using the predicted product of the chemical reaction.

In an embodiment, the chemical reaction prediction model may include a first module that receives the molecular structure and predicts a graph-based chemical reaction, and a second module that analyzes information related to chemical reaction prediction related to the plurality of molecular structures from text data, and in the performing of the chemical reaction prediction, the product according to the chemical reaction prediction may be generated using the predicted result of the first module and the analysis result of the second module.

In an embodiment, in the performing of the chemical reaction prediction, the product predicted through the first module may be verified using the output data analyzed in the second module.

In an embodiment, the plurality of molecular structures may include a first molecular structure and the second molecular structure, in the acquiring of the molecular graphs for the plurality of molecular structures, atoms constituting the first molecular structure may be converted into nodes and a bond relationship between atoms constituting the first molecular structure may be converted into edges to acquire a first molecular graph including the nodes and edges corresponding to the first molecular structure, and atoms constituting the second molecular structure may be converted into nodes and a bond relationship between atoms constituting the second molecular structure may be converted into edges to acquire the second molecular graph including the nodes and edges corresponding to the second molecular structure.

In an embodiment, the answer generation method may further include performing labeling so that different labels are assigned to each of the plurality of molecular structures, and storing, in the memory, information on the first molecular graph acquired through the process of acquiring the molecular graph and a label assigned to the first molecular graph, and information on the second molecular graph acquired through the process of acquiring the molecular graph and a label assigned to the second molecular graph.

In an embodiment, when the user query including the plurality of molecular structures to which the different labels are assigned is received, in the generating of the answer, the plurality of molecular structures to which the different labels are assigned may be processed as an input of the chemical reaction prediction model, the product of the chemical reaction prediction acquired from the chemical reaction prediction model may be used to generate the answer to the user query, and when the answer to the user query includes a specific molecular structure generated through the chemical reaction prediction model, the label may be assigned to the specific molecular structure.

In an embodiment, the answer generation method may further include providing, to a region of the service page where the user query is received, a plurality of graphic objects corresponding to each of the plurality of molecular structures to which the different labels are assigned, in which each of the plurality of graphic objects may include the first molecule graph and the second molecule graph.

In an embodiment, based on receiving the user input for selecting one of the plurality of graphic objects, the service page may provide detailed information corresponding to a graphic object selected according to the user input.

In an embodiment, in the performing of the chemical reaction prediction on the plurality of molecular structures, an embedding vector corresponding to the molecular graph may be acquired using the information on the plurality of molecular structures, an attention operation related to an interaction between the atoms of the plurality of molecular structures may be performed, and the embedding vector may be updated based on the operation, bond prediction and atom prediction predicted as a chemical reaction of the plurality of molecular structures may be performed using the updated embedding vector, and the product of the chemical reaction prediction predicted from the chemical reaction of the plurality of molecular structures may be acquired using a result of the bond prediction and a result of the atom prediction.

In an embodiment, the updated embedding vector may be updated by adding different biases according to a bond type between the nodes constituting the first molecular graph and the second molecular graph to an attention score calculated according to the result of performing the attention operation.

In an embodiment, the bond prediction may be performed by performing a dot-product using the updated embedding vector, the atom prediction may predict atom properties of the atoms corresponding to the updated embedding vector using an atom property probability distribution of each of the atoms corresponding to the updated embedding vector, and the atom properties may include a charge state of the atoms that change during a chemical reaction process of the plurality of molecular structures.

In an embodiment, the product of the chemical reaction prediction may correspond to a final product stabilized through a diffusion feedback process.

In an embodiment, the service page may include at least one of a first region in which the plurality of graphic objects corresponding to each of the plurality of molecular structures assigned with the different labels and detailed information on the plurality of molecular structures are provided, a second region in which the answer to the user query is provided, and a third region in which the user query is received, and the detailed information on the plurality of molecular structures may include at least one of a molecular structure image, a name, a property, and a string according to a SMILES notation of each of the plurality of molecular structures, the detailed information may extracted from the user input or acquired from at least one pre-trained prediction model, and the pre-trained prediction model may include at least one of a chemical reaction prediction reaction model that predicts the chemical reaction between the molecular structures and a molecular property prediction model that predicts a property of the molecular structure.

In an embodiment, the answer generation method may further include receiving an editing request about the plurality of molecular structures through the service page to which the answer to the user query is provided, and providing an editing interface that provides an editing function for the plurality of molecular structures to the service page.

In an embodiment, the molecular graphs corresponding to at least one of the first molecular structure and second molecular structure acquired through the molecular graph transformation may be provided to the editing interface in an editable state.

In an embodiment, the editing of the plurality of molecular structures may be a deletion or position change of at least one of the nodes corresponding to each of the atoms constituting each of the plurality of molecular structures and the edges indicating the bond relationship of the atoms, or an addition of a new node corresponding to a new atom or an addition of a new edge that generates a new bond relationship between the atoms.

In an embodiment, the edited molecular structure among the plurality of molecular structures may be stored in the memory, a new label specifying the edited molecular structure may be assigned to the edited molecular structure, and when a user query including the new label is input to the ultra-large foundation model, the ultra-large foundation model may generate an answer using the edited molecular structure corresponding to the new label.

An answer generation system according to various embodiments disclosed in the present document may include a memory and at least one processor, in which the memory and the processor cooperate to specify a plurality of molecular structures to be predicted based on user input received through a service page, store information on the plurality of molecular structures in the memory, acquire molecular graphs for the plurality of molecular structures by converting atoms into nodes and bonds between the atoms into edges based on the plurality of molecular structures stored in the memory, receive a user query for chemical reaction prediction related to the plurality of molecular structures through the service page, process the molecular graphs for the plurality of molecular structures as an input of a chemical reaction prediction model so that a chemical reaction corresponding to the user query is predicted, perform chemical reaction prediction on the plurality of molecular structures in the chemical reaction prediction model, acquire a product of the chemical reaction prediction for the plurality of structures from the chemical reaction prediction model, and generate an answer to the user query using the product of the chemical reaction prediction.

A program stored on a computer-readable recording medium, executable by one or more processes on an electronic device according to various embodiments disclosed in the present document may include instructions to execute: specifying a plurality of molecular structures to be predicted based on user input received through a service page, storing information on the plurality of molecular structures in the memory, acquiring molecular graphs for the plurality of molecular structures by converting atoms into nodes and bonds between the atoms into edges based on the plurality of molecular structures stored in the memory, receiving a user query for chemical reaction prediction related to the plurality of molecular structures through the service page, processing the molecular graphs for the plurality of molecular structures as an input of a chemical reaction prediction model so that a chemical reaction corresponding to the user query is predicted, performing chemical reaction prediction on the plurality of molecular structures in the chemical reaction prediction model, acquiring a product of the chemical reaction prediction for the plurality of structures from the chemical reaction prediction model, and generating an answer to the user query using the product of the chemical reaction prediction.

A chemical reaction prediction method performed by cooperation of a memory and a processor according to various embodiments disclosed in the present document may include: receiving information related to a plurality of molecular structures as an input of an encoder; acquiring an embedding vector corresponding to the plurality of molecular structures using the information related to the plurality of molecular structures in an embedding layer of the encoder; performing an attention operation related to an interaction between atoms of the plurality of molecular structures in a multi-head self-attention layer and updating the embedding vector based on the operation; storing the embedding vector updated through the updating step in the memory and inputting the updated embedding vector stored in the memory to a decoder; performing bond prediction and atom prediction predicted as a chemical reaction of the plurality of molecular structures using the updated embedding vector in the decoder; and acquiring a final chemical reaction product predicted from the chemical reaction of the plurality of molecular structures using a result of the bond prediction and a result of the atom prediction.

In an embodiment, the acquiring of the chemical reaction product may include sampling an initial chemical reaction result using the result of the bond prediction and the result of the atom prediction, stabilizing the sampled initial chemical reaction result through a diffusion feedback process, and acquiring the final chemical reaction product stabilized through the diffusion feedback process.

In an embodiment, the chemical reaction prediction method may further include acquiring molecular graphs for the plurality of molecular structures by converting atoms into nodes and bonds between the atoms into edges based on the plurality of molecular structures, in which the structure of the plurality of molecules may include a first molecular structure and the second molecular structure, and the acquiring of the molecular graph may include acquiring the first molecular graph including nodes and edges corresponding to the first molecular structure by converting atoms constituting the first molecular structure into the nodes and a bond relationship between atoms constituting the first molecular structure into edges using a pre-specified graph transformation algorithm, and acquiring the second molecular graph including the nodes and edges corresponding to the second molecular structure by converting atoms constituting the second molecular structure into nodes and a bond relationship between atoms constituting the second molecular structure into edges using the pre-specified graph transformation algorithm.

In an embodiment, the information related to the plurality of molecular structures includes information on the nodes and edges corresponding to the first molecular structure and information on the nodes and edges corresponding to the second molecular structure, and the embedding vector includes at least one piece of information on an atom type, an atom charge, the number of hydrogens, the number of radical electrons, and a degree of the node corresponding to the first molecular structure and the node corresponding to the second molecular structure.

In an embodiment, the updating of the embedding vector, different biases may be added to an attention score calculated in the multi-head self-attention layer according to the bond type between the nodes constituting the first molecular graph and the second molecular graph.

In an embodiment, the bond type may include a single bond type, a double bond type, a triple bond type, and an aromatic bond type.

In an embodiment, the chemical reaction prediction method may further include extracting at least one of an adjacency matrix, a bond type matrix, a shortest paths matrix, and K-hop neighbors corresponding to the first molecular graph and the second molecular graph, respectively, using the nodes and edges corresponding to the first molecular graph and the nodes and edges corresponding to the second molecular graph, in which the adjacency matrix may include information on a direct connection between the nodes constituting the first molecular graph and the second molecular graph, the bond type matrix may include information on the bond type between the nodes constituting the first molecular graph and the second molecular graph, the shortest paths matrix may include information on a shortest path length between the nodes constituting the first molecular graph and the second molecular graph, the K-hop neighbors information may include information on neighboring nodes within K-hops for each of the nodes constituting the first molecular graph and the second molecular graph.

In an embodiment, in the updating of the embedding vector, the output vector of the multi-head self-attention layer may be input to a feed-forward neural network layer, in the feed-forward neural network layer, the output vector of the multi-head self-attention layer may updated using at least one of the adjacency matrix, bond type matrix, shortest paths matrix, and K-hop neighbors, and the output vector of the feed-forward neural network layer may be specified as the updated embedding vector.

In an embodiment, the bond prediction may be performed by performing a dot-product using the updated embedding vector, and the dot-product may be performed for each vector corresponding to each atom pair of the atoms corresponding to the updated embedding vector.

In an embodiment, the performing of the bond prediction may include acquiring an inner product value for each of the plurality of bond types for each of the atom pairs based on the dot-product, and the plurality of bond types may be related to at least one of a single bond, a double bond, a bond formation, a bond collapse, and no change.

In an embodiment, the performing of the bond prediction may further include generating a probability distribution for each of the plurality of bond types for each of the atom pairs using the inner product value according to the dot-product, and acquiring a transformation matrix that predicts a change in a bonded state of each of the atom pairs using the probability distribution.

In an embodiment, in the generating of the probability distribution, for each atom pair, a Softmax function may be applied to the inner product values acquired for the plurality of bond types to generate the probability distribution for the plurality of bond types for each atom pair.

In an embodiment, the performing of the atom prediction may further include generating an atom property probability distribution for each atom corresponding to the updated embedding vector using a Softmax output layer, and predicting atom properties of the atoms corresponding to the updated embedding vector using the probability distribution, and the atom properties may include a charge state of the atoms that change during the chemical reaction process of the structure of the plurality of molecules.

In an embodiment, the diffusion feedback process may repeatedly evaluate each bond transformation of the initial chemical reaction product, and remove or modify an unstable bond.

In an embodiment, the diffusion feedback process may evaluate the predicted bond transformation at each step repeatedly performed using a transformation probability matrix and a target transformation matrix to predict the change in the bonded state of the initial chemical reaction product, and generate the final chemical reaction product using an interpolation factor.

A chemical reaction prediction method performed by cooperation of a memory and a processor according to various embodiments disclosed in the present document may include: receiving information related to a plurality of molecular structures as an input of an encoder; acquiring a molecular graph using atoms as nodes and bonds as edges based on the plurality of molecular structures; acquiring an embedding vector corresponding to the molecular graph using the information related to the plurality of molecular structures in the embedding layer of the encoder; performing an attention operation related to an interaction between the atoms of the plurality of molecular structures in a multi-head self-attention layer and updating the embedding vector based on the operation; storing the embedding vector updated through the updating step in the memory and inputting the updated embedding vector stored in the memory into a decoder; performing bond prediction and atom prediction predicted as a chemical reaction of the plurality of molecular structures using the updated embedding vector in the decoder; acquiring a final chemical reaction product predicted from the chemical reaction of the plurality of molecular structures using a result of the bond prediction and a result of the atom prediction; calculating a loss function between the final chemical reaction product and label data including the actually bonded state and atom state corresponding to the plurality of molecular structures; and optimizing a parameter of at least one of the encoder and decoder to minimize the loss function.

A chemical reaction prediction system according to various embodiments disclosed in the present document may include a memory, an encoder, a decoder, and at least one processor, in which the encoder may receive information related to a plurality of molecular structures, acquires a molecular graph using atoms as nodes and bonds as edges based on the plurality of molecular structures, acquire an embedding vector corresponding to the molecular graph in an embedding layer of the encoder, perform an attention operation related to an interaction between atoms of the plurality of molecular structures in a multi-head self-attention layer of the encoder, and update the embedding vector based on the operation, the processor may store the embedding vector updated through the updating step in the memory and inputs the updated embedding vector stored in the memory to a decoder, and the decoder may perform bond prediction and atom prediction predicted as a chemical reaction of the plurality of molecular structures using the updated embedding vector, and acquire a final chemical reaction product predicted from a chemical reaction of the plurality of molecular structures using a result of the bond prediction and a result of the atom prediction.

A program stored on a computer-readable recording medium, executable by one or more processes on an electronic device according to various embodiments disclosed in the present document may include instructions to execute: receiving information related to a plurality of molecular structures as an input of an encoder; acquiring a molecular graph using atoms as nodes and bonds as edges based on the plurality of molecular structures; acquiring an embedding vector corresponding to the molecular graph in an embedding layer of the encoder; performing an attention operation related to an interaction between atoms of the plurality of molecular structures in a multi-head self-attention layer and updating the embedding vector based on the operation; storing the embedding vector updated through the updating step in a memory and inputting the updated embedding vector stored in the memory to a decoder; performing bond prediction and atom prediction predicted as a chemical reaction of the plurality of molecular structures using the updated embedding vector in the decoder; and performing a final chemical reaction product predicted from the chemical reaction of the plurality of molecular structures using a result of the bond prediction and a result of the atom prediction.

### [Advantageous Effects]

As described above, according to the answer generation method and system according to the present invention, by generating and providing the answer suitable for the user query based on the data extracted from the document, it is possible for the user to minimize the risk of research failure by receiving suggestions for the optimal research method.

In addition, according to the answer generation method and system according to the present invention, it is possible to provide the answer to the user query using the data that is extracted from the document or generated from the pre-trained prediction model. Accordingly, the user can quickly and accurately be provided with his/her required information and reduce the time and/or cost of research and/or development.

Furthermore, according to the answer generation method and system according to the present invention, it is possible to generate the answer to the user query using the predicted results from the pre-trained prediction model and provide the generated answer to the user. Accordingly, it is possible for the user to shorten the time required for research and/or development and reduce the number of trial and errors in research and/or development.

Furthermore, according to the answer generation method and system according to the present invention, by visualizing and providing the extracted molecular structure and related data through the user interface, it is possible for the user to intuitively recognize his/her required information and understand the information more quickly, thereby increasing the accuracy and efficiency of the research.

Meanwhile, according to the chemical reaction prediction system and the control method thereof, and the learning method of a chemical reaction prediction system according to the present invention, by modeling the movement of electrons through the graph structure of molecules, it is possible to better understand the chemical reaction mechanisms and accurate prediction of the results of the chemical reaction.

In addition, according to the chemical reaction prediction system and the control method thereof according to the present invention, and the learning method of a chemical reaction prediction system, by providing the chemical reaction prediction model that can accurately predict the results of the chemical reaction and better understand the chemical reaction mechanisms, it is possible for a user to reduce the time and cost required for experiments. Accordingly, it is possible to reduce the research and development costs and shorten the time to market for new products.

Meanwhile, according to the chemical reaction prediction system, the control method thereof, and the learning method of a chemical reaction prediction system according to the present invention, by performing both the input of the molecular structure and the output of the predicted product in the graph space, it is possible to perform the forward reaction prediction regardless of the SMILES permutation and order.

Furthermore, according to the chemical reaction prediction system, the control method thereof, and the learning method of a chemical reaction prediction system according to the present invention, by simultaneously sampling multiple interdependent transformations that occur in parallel within the molecular graph, it is possible to secure the consistency between the transformations.

Furthermore, according to the chemical reaction prediction system, the control method thereof, and the learning method of a chemical reaction prediction system according to the present invention, in order to solve problems that may occur due to the symmetrical structure, by breaking the symmetry and forming the valid output structure by including the noise or sampling mechanisms, it is possible to prevent the occurrence of the invalid configuration.

### [Description of Drawings]

FIG. 1 is a conceptual diagram for describing an answer generation system to which a chemical reaction prediction system and a control method thereof, and a learning method of a chemical reaction prediction system according to the present invention are applied.
FIG. 2A to 2F are conceptual diagrams for describing a chemical reaction prediction model according to the present invention.
FIG. 3 is a flowchart for describing a learning method of a chemical reaction prediction model according to the present invention.
FIGS. 4A to 4D are conceptual diagrams for describing the chemical reaction prediction model according to the present invention.
FIGS. 5A to 5E, 6, and 7 are conceptual diagrams for describing examples of utilization in the answer system to which the chemical reaction prediction model according to the present invention is applied.
FIGS. 8 and 9 are conceptual diagrams for describing an ultra-large foundation model according to the present invention.
FIG. 10 is a flowchart for describing an answer generation method according to the present invention.
FIGS 11 to 32 are conceptual diagrams for describing the answer generation method according to the present invention.

### [Mode for Disclosure]

Hereinafter, embodiments disclosed in this specification will be described in detail with reference to the accompanying drawings, but the same or similar components will be denoted by the same reference numerals independent of the drawing numerals, and an overlapping description of the same or similar components will be omitted. In addition, the terms "module" and "unit" for components used in the following description are used only to easily make the disclosure. Therefore, these terms do not have meanings or roles that distinguish from each other in themselves. Further, in describing the embodiments disclosed in this specification, if it is determined that a detailed description of related known technologies may obscure the gist of the embodiments disclosed in this specification, the detailed description thereof is omitted. In addition, it is to be understood that the accompanying drawings are provided only for easy understanding of embodiments disclosed in this specification, and the technical idea disclosed in this specification is not limited by the accompanying drawings, but includes all the modifications, equivalents, and substitutions included in the spirit and the scope of the present invention.

The terms including ordinal numbers such as 'first' and 'second' may be used to describe various components, but these components are not limited by these terms. The terms are used to distinguish one component from another component.

It is to be understood that when one component is referred to as being "connected to" or "coupled to" another component, one component may be connected directly to or coupled directly to another component or be connected to or coupled to another component with the other component interposed therebetween. On the other hand, it is to be understood that when one component is referred to as being "connected directly to" or "coupled directly to" another component, it may be connected to or coupled to another component without the other component interposed therebetween.

Singular forms include plural forms unless the context clearly indicates otherwise.

It will be further understood that the terms "include" or "have" used in the present specification specify the presence of features, numerals, steps, operations, components, parts mentioned in the present specification, or combinations thereof, but do not preclude the presence or addition of one or more other features, numerals, steps, operations, components, parts, or combinations thereof.

The present invention relates to an answer generation method and system. The answer generation system according to the present invention performs answer generation based on generative artificial intelligence (generative AI) or a foundation model, and may also be called an answer generation platform based on an ultra-large foundation model. However, the "ultra-large foundation model" may also be called a generative model, a foundation model, or a large language model (LLM). The answer generation system according to the present invention may be a system that generates property prediction results of a molecular structure or designs a molecule having user's desired characteristics. In addition, the answer generation system according to the present invention may be a system that generates a predicted result of a chemical reaction between a new type of molecules and/or a plurality of molecules. Furthermore, the answer generation system according to the present invention may be a system that generates a predicted result of transformation of existing materials and synthesis of various materials (e.g., a new material, a polymer material, a nano material, a composite material, an organic material, a pharmaceutical material, etc.).

The answer generation system according to the present invention includes an ultra-large foundation model (or an ultra-large foundation artificial intelligence model), and the present invention aims to increase the efficiency of natural science research by minimizing the risk of research failure.

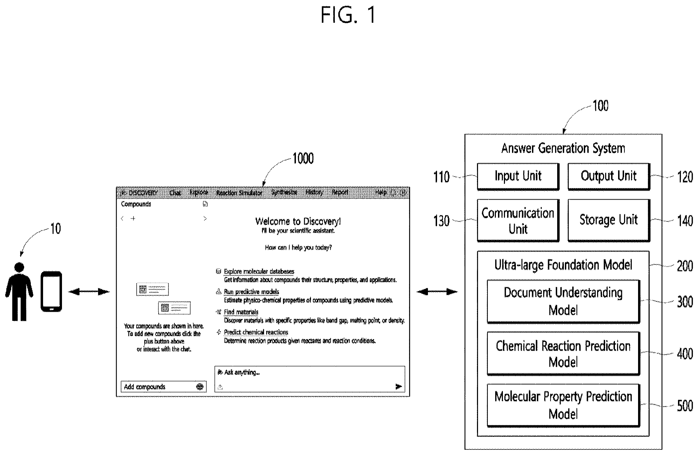

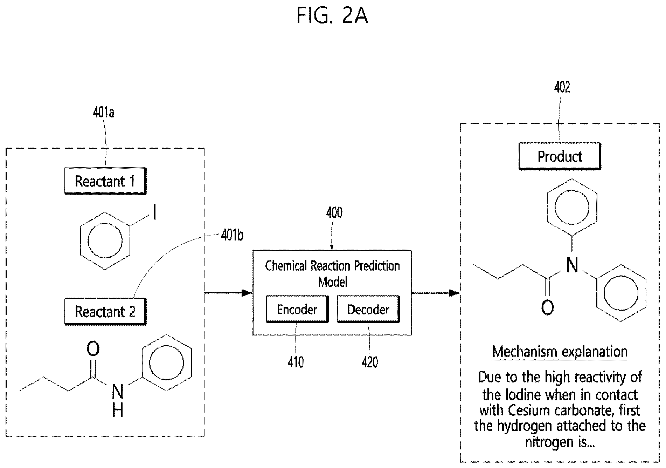

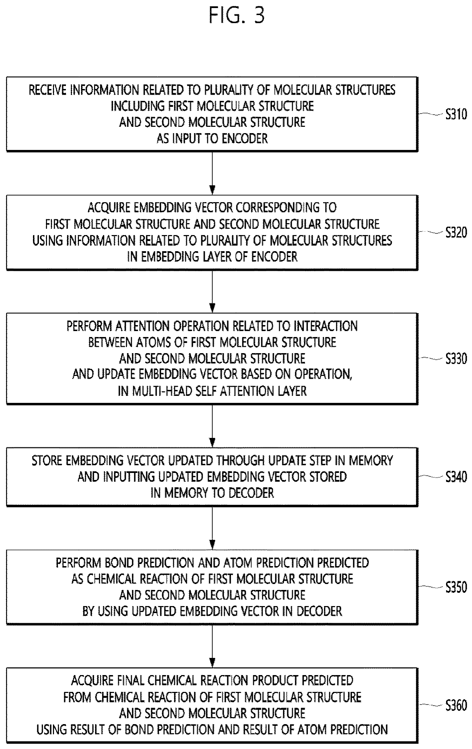

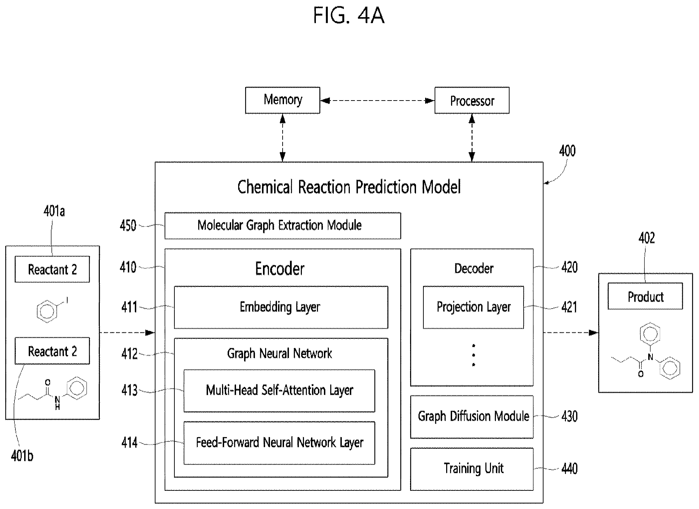

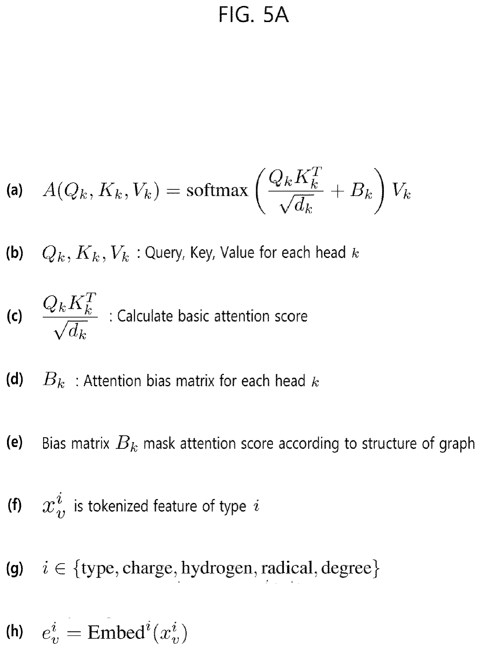

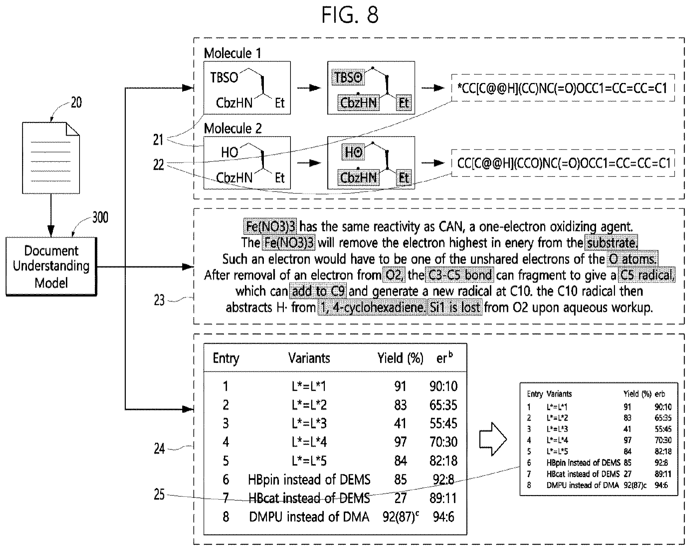

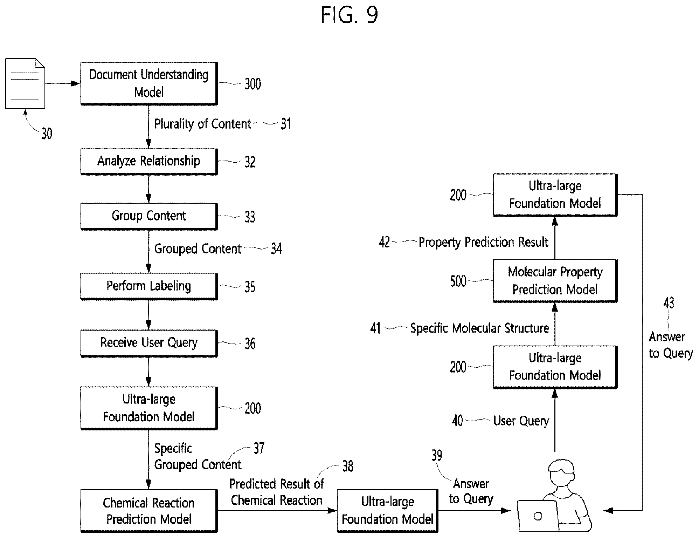

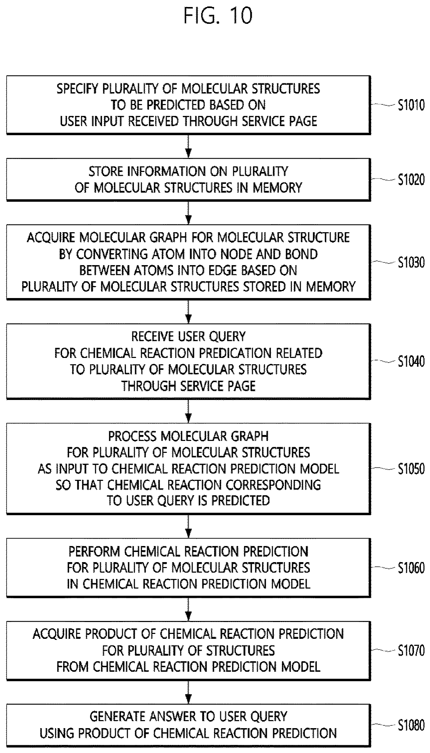

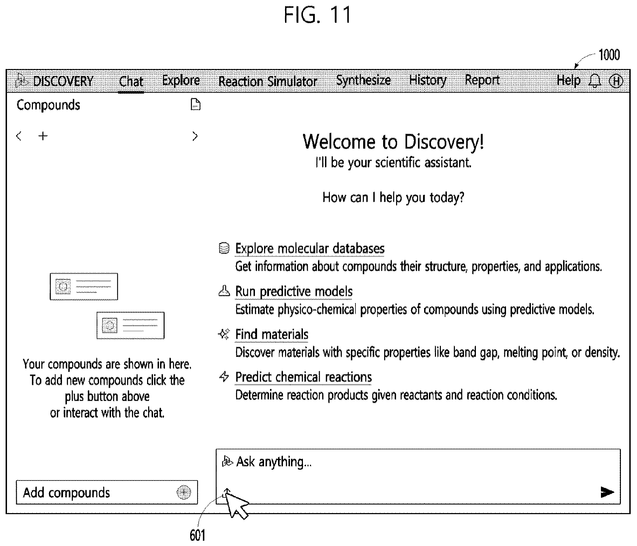

Hereinafter, it will be described in more detail with the attached drawings. FIG. 1 is a conceptual diagram for describing an answer generation system to which a chemical reaction prediction system and a control method thereof, and a learning method of a chemical reaction prediction system according to the present invention are applied, and FIG. 2A to 2F are conceptual diagrams for describing a chemical reaction prediction model according to the present invention. FIG. 3 is a flowchart for describing a learning method of a chemical reaction prediction model according to the present invention, FIGS. 4A to 4D are conceptual diagrams for describing the chemical reaction prediction model according to the present invention, and FIGS. 5A to 5E, 6, and 7 are conceptual diagrams for describing examples of utilization in the answer system to which the chemical reaction prediction model according to the present invention is applied. FIGS. 8 and 9 are conceptual diagrams for describing an ultra-large foundation model according to the present invention, and FIG. 10 is a flowchart for describing an answer generation method according to the present invention. FIGS 11 to 32 are conceptual diagrams for describing the answer generation method according to the present invention.

Meanwhile, as illustrated in FIG. 1, an answer generation system 100 may include at least one of an input unit 110, an output unit 120, a communication unit 130, a storage unit 140, and an ultra-large foundation model 200. Here, an ultra-large foundation model 200 may also be referred to as a foundation model, and the foundation model may mean an ultra-large AI core foundation model trained with a massive dataset.

Although not illustrated, the answer generation system 100 may include one or more processors, and the processors may include one or more general-purpose processors and/or one or more special-purpose processors (e.g., a digital signal processor, a tensor processing unit (TPU), a graphics processing unit (GPU), a neural network processing unit (NPU), an application-specific integrated circuit, an application-specific integrated circuit (ASIC), etc.). The one or more processors may be configured to execute instructions stored (or included) in the storage unit 140, computer-readable instructions, and/or other instructions described herein. The answer generation system and method may perform data processing described below by cooperation of a memory and at least one processor. The processor may perform a series of operations and data processing using data and information stored in the memory. In this case, the memory may be a configuration of the storage unit 140.

Meanwhile, the input unit 110 is a means for data input, and may be configured in various types. For example, the input unit 110 may be configured to receive user input. The input unit 110 may be configured to receive the user input from the user terminal 10. Here, the "receiving input" may mean receiving an input signal (or selection signal) corresponding to user input based on input performed by a user through the configuration of the input unit provided in the user terminal 10.

The input unit 110 may also be referred to as a user interface module. The input unit 110 may include a touch screen, a computer mouse, a keyboard, a keypad, a touch pad, a trackball, a joystick, a voice recognition module, or other similar devices. However, the present invention does not limit the type of the input unit 110. In addition, the input unit 110 in the present invention does not necessarily mean a hardware means, and may be understood as a passage for receiving input from a user.

Here, the user input may include documents, texts, images (or videos), voices, etc. In this case, the answer generation system 100 may further include a module for converting voice into text.

Next, the output unit 120 may output information through the configuration of the output unit (e.g., a display unit, a touch screen, a speaker, etc.) provided in the user terminal 10 linked to the answer generation system 100. For example, the output unit 120 may output a page (or a service page 1000) linked to the answer generation system 100 to the display unit of the user terminal 10. In addition, the output unit 120 does not necessarily mean a hardware means, and may be understood as a passage for outputting results to the user.

Next, the communication unit 130 may be connected to the user terminal 10, a server (e.g., a central server, an external server, etc.), a device, and at least one network, etc., through a wireless or wired network, and may be configured to receive or transmit the overall data and information necessary for the operation of the answer generation system 100.

Here, the mobile terminal 10 may include at least one of a mobile phone, a smart phone, a notebook computer, a laptop computer, a slate PC, a tablet PC, an ultrabook, a desktop computer, a digital broadcasting terminal, personal digital assistants (PDA), a portable multimedia player (PMP), navigation, a wearable device (e.g., a smartwatch, a smart glass, and a head mounted display (HMD)), and the like.

Furthermore, the communication unit 130 may support various communication methods according to the communication standards of the communicating device.

For example, the communication unit 130 may be configured to communicate with a communication target using at least one of wireless LAN (WLAN), Wireless-Fidelity (Wi-Fi), Wireless Fidelity (Wi-Fi) direct, digital living network alliance (DLAN), Wireless Broadband, World Interoperability for Microwave Access (WiMAX), High Speed Downlink Packet Access (HSDPA), High Speed Uplink Packet Access (HSUPA), Long Term Evolution (LTE), Long Term Evolution-Advanced (LTE-A), 5th Generation (5G) Mobile Telecommunication, Bluetooth^{™} Radio Frequency Identification (RFID), Infrared Data Association; IrDA), Ultra-Wideband (UWB), ZigBee, Near Field Communication (NFC), Wi-Fi Direct, and Wireless Universal Serial Bus (USB) technologies.

Meanwhile, the storage unit 140 serves to store various data related to the present invention and may include one or more non-transitory computer-readable storage media that may be read and/or accessed by at least one of the one or more processors 140.

The one or more computer-readable storage media may include volatile and/or nonvolatile storage components such as optical, magnetic, organic or other memory or disk storage devices. In some examples, the storage unit 140 may be implemented using a single physical device (e.g., one optical, magnetic, organic, or other memory or disk storage device), while in other examples, the storage unit 140 may be implemented using two or more physical devices.

The storage unit 140 may include computer-readable instructions and additional data. The storage unit 140 may include storage necessary to perform at least some of methods, scenarios and techniques described herein and/or at least some of the functions of the devices and networks.

Furthermore, at least a portion of the storage unit 140 may be a cloud storage or a cloud server. The storage unit 140 may store at least some of data corresponding to the user input received from the input unit 110 and training data.

That is, the storage unit 140 may be sufficient as a space where information necessary for the operation of the answer generation system 100 is stored, and it may be understood that there is no limitation on the physical space.

Meanwhile, the ultra-large foundation model 200 may be configured to generate property prediction results of a molecular structure or to design a molecule having user's desired characteristics. In addition, the ultra-large foundation model 200 may generate predicted results of a chemical reaction between a new type of molecule and/or a plurality of molecules, or generate predicted results of transformation of the existing materials and synthesis of various materials (e.g., a new material, a polymer material, a nano material, a composite material, an organic material, a pharmaceutical material, etc.).

In this regard, the ultra-large foundation model 200 may include at least one of a document understanding model 300, a chemical reaction prediction model 400, and a molecular property prediction model 500.

The document understanding model 300 may extract various types of content that satisfy preset content criteria from documents (e.g., papers, books, patent documents, reports, etc.). More specifically, the document understanding model 300 may be a model trained to understand structured data, unstructured data, linguistic data (or linguistic elements), non-linguistic data (or non-linguistic elements), etc., included in a document, and extract various content (or data) and knowledge based on the understood contents.

Here, the preset content criteria may be set in various ways and may be determined according to the purpose or utilization purpose of the answer generation system 100 according to the present invention.

For example, when the utilization purpose of the answer generation system 100 is chemistry, bio, new materials, new substances, and new drug development, the document understanding model 300 may be trained to understand and extract the contents related to the chemistry, the bio, the new materials, the new substances, and the new drug development from an analysis target document.

In this case, the preset content criteria may include the contents related to the molecular structures that are related to at least one of the chemistry, the bio, the new materials, the new substances, and the new drug development. Here, the document understanding model 300 may extract the contents related to the chemistry, the bio, the new materials, the new substances, and the new drug development from the analysis target document based on the preset content criteria.

In this specification, for the convenience of description, the preset content criteria are described as being related to at least one of the chemistry, the bio, the new materials, the new substances, and the new drug development, but are not necessarily limited thereto.

Based on the preset content criteria, the document understanding model 300 may extract at least one of text, a molecular structure, a formula, a chart, a table, and an image, which satisfy the preset content criteria, from the analysis target document.

For example, the document understanding model 300 may understand the chemical structure of the molecular structure included in the analysis target document 20, and extract the molecular structure by transforming the molecular structure into a SMILES string expression based on the result of understanding. In addition, the document understanding model 300 may understand the chemical structure of the molecular structure and perform graph transformation corresponding to the molecular structure based on the result of understanding.

Meanwhile, in the present invention, receiving the "molecular structure" may be understood as receiving information that may specify a molecule. In this case, the information that may specify the molecular structure may be in various forms such as a molecular structure formula, a molecular graph, a chemical formula, a molecular structure formula based on a SMILES notation, a molecular structure image, etc.

In addition, the document understanding model 300 may understand text 23 related to a molecular structure 21 among the texts included in the analysis target document 20, and may extract the text 23 as text data related to the molecular structure 21.

Furthermore, the document understanding model 300 may recognize rows and columns that constitute a table 24 related to the molecular structure 21 from the analysis target document 20 and extract structured data 25 by converting the recognized rows and columns into the structured data 25 in a format such as HTML or Excel.

Furthermore, the document understanding model 300 may extract relationship information (or relationship) between the molecular structures included in the document.

As described above, the document understanding model 300 may extract various types of data included in a document by converting the data into data (e.g., machine-readable data) in a form that the ultra-large foundation model 200 may understand. The data extracted using the document understanding model 300 may be sorted in units of pages or documents and stored in the storage unit 140 (or memory). In the present invention, the document understanding model 300 may also be called a deep document understanding model.

Next, the chemical reaction prediction model 400 may be configured to predict a chemical reaction between input molecular structures (or compounds or reactants) and output (or generate) the predicted results (or products) of chemical reaction. For example, as illustrated in FIG. 2A, when information on molecular structures 401a and 401b of a specific compound is input, the chemical reaction prediction model 400 may predict a product 402 formed by the chemical reaction between molecular structures 401a and 401b of the specific compound.

The chemical reaction prediction model 400 according to the present invention may model the movement of electrons through a graph structure of molecules. The chemical reaction prediction model 400 according to the present invention may be an electron flow-based prediction model using graph diffusion that may better understand the chemical reaction mechanisms and accurately predict the results of the chemical reaction. The chemical reaction prediction model 400 may express both input and output in a graph space. The chemical reaction prediction model 400 may train the graph transformation of the chemical reaction to output reaction products and/or byproduct prediction results based on the graph structure of the input compound. The chemical reaction prediction model 400 in the present invention may also be called an electron flow model, an electron flow-based forward reaction prediction model, or an electron-flow inspired graph diffusion model for interpretable forward reaction prediction model.

Meanwhile, the chemical reaction prediction model 400 described above may include at least one of an encoder 410 and a decoder 420.

The encoder 410 according to the present invention may be configured to extract features of each atom and bond through a graph representation of a molecular structure and generate an embedding vector based on the extracted features. In addition, by performing an attention operation related to an interaction between the atoms of the plurality of molecular structures through a neural network, the characteristics of the molecular structure may be better understood. The decoder 420 may perform bond prediction and atom prediction, which are predicted as the chemical reaction of the plurality of molecular structures, using the embedding vector acquired from the encoder 410. The chemical reaction prediction model 400 may perform a diffusion feedback process to stabilize the chemical reaction products according to the bond prediction and atom prediction, thereby generating a final chemical reaction product. The composition and detailed process of the chemical reaction prediction model 400 according to the present invention will be described in more detail later.

Meanwhile, the chemical reaction prediction model 400 according to the present invention may use not only the structural information of molecules but also various types of chemical information such as reaction conditions, catalysts, and temperatures in order to predict more accurate chemical reactions. Such chemical information may include structural data (e.g., molecular structure graph) of molecules and descriptive data (e.g., text data). Since the chemical reaction are affected by various factors such as structural changes of molecules, reaction mechanisms, reaction conditions, and reaction environments, more accurate predictions can be possible only when these elements are comprehensively considered. Meanwhile, such chemical information may exist in various literature, textbooks, papers, patent literatures, articles, and academic journals, and the chemical reaction prediction model 400 according to the present invention may perform more accurate chemical reaction predictions by integrating such chemical information.

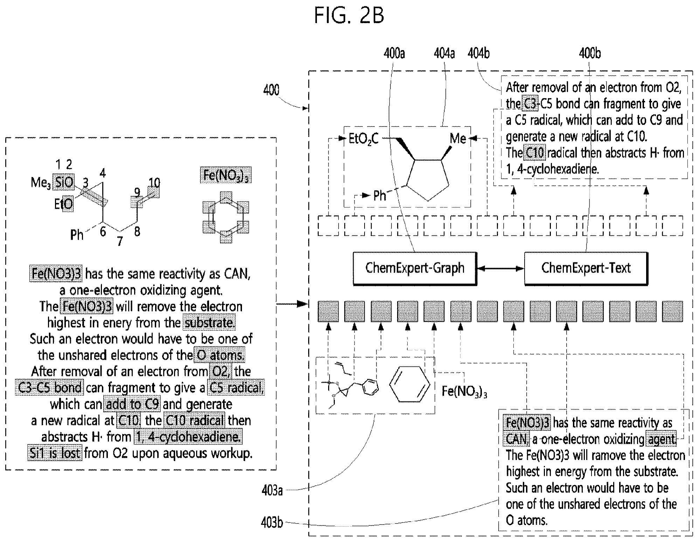

To this end, as illustrated in FIG. 2B, the chemical reaction prediction model 400 may include at least one of a first module 400a and a second module 400b. Here, the first module 400a may also be named as a "ChemExpert-Graph" module, a graph module, a graph processing module, a graph model, a graph processing model, etc., and the second module 400b may also be called as a "ChemExpert-Text" module, a text module, a text processing module, a text model, a text processing model, etc.

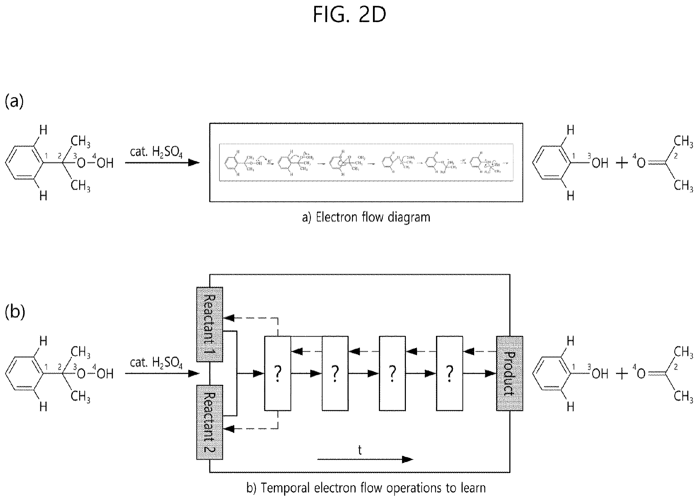

The first module 400a may receive a molecular (or chemical) structure as input and predict a graph-based chemical reaction. For example, referring to (a) of FIG. 2D, an example of the chemical reaction may be confirmed. As illustrated in (b) of FIG. 2D, the first module 400a may include a plurality of layers 400a-1 to predict the graph-based chemical reaction. More specific details regarding the plurality of layers 400a-1 will be described later.

A molecular structure 403a (or molecular structure formula) input to the first module 400a is converted into a molecular graph in the form of a graph, and atoms in the molecular graph may be expressed as nodes and bonds may be expressed as edges.

The molecular structure 403a may correspond to at least one of data extracted from a document 403 including a molecular structure 411 using the document understanding model 300, or information extracted through the storage unit 140 (or memory).

The first module 400a may analyze changes in structural characteristics of a molecule based on the input molecular graph, predict a chemical reaction path and a product to be generated as the results of the chemical reaction, and output the predicted chemical reaction path and product.

In an embodiment, the first module 400a may analyze structural changes of a molecule based on the molecular graph, and predict a process in which a specific bond is separated and a new bond is formed.

In another embodiment, the first module 400a may analyze the interaction between the atoms in the molecule based on the molecular graph, and predict radical formation and bond changes that may occur at each step.

That is, the first module 400a may be configured to receive the molecular graph as input, and output the predicted chemical reaction path and a product 404a based on the molecular graph.

Next, the second module 400b may be configured to process text data 403b to understand and predict a reaction mechanism. In this case, the text data 403b may correspond to at least one of data extracted from a document including the molecular structure 403a using the document understanding model 300, or information extracted through the storage unit 140 (or memory) related to the molecular structure 411. The second module 400b may be a model that has pre-trained vast data related to the chemical reaction.

In an embodiment, the text data 403b input to the second module 400b is data including a description of the molecular structure 403a, and may include at least one of chemical reaction conditions, chemical reaction mechanisms (or reaction paths), and chemical characteristics of the molecular structure 403a.

The second module 400b may analyze the input text data 403b to understand and predict the chemical reaction mechanisms. More specifically, the second module 400b may analyze the input text data 403b and output at least one of the chemical reaction conditions, chemical reaction mechanisms, and chemical characteristics that are predicted based on the text data 403b.

In an embodiment, the second module 400b may analyze the text data 403b using a natural language processing (NLP) technology and extract at least one text of the chemical reaction conditions, chemical reaction mechanisms (or reaction paths), chemical characteristics, and experimental data included in the text data 403b.

In another embodiment, the second module 400b may predict the chemical reaction mechanisms (e.g., how a specific catalyst or condition affects the reaction) based on the text extracted through the analysis of the text data 403b, and output the predicted chemical reaction mechanisms and chemical characteristics.

The second module 400b may analyze the information related to the chemical reaction prediction, which is related to the plurality of molecular structures, from the text data.

The chemical reaction prediction model 400 may combine the output data 404a of the first module 400a and the output data 404b of the second module 400b to output predicted results (e.g., product, chemical reaction path, chemical reaction mechanisms, etc.) of a final chemical reaction.

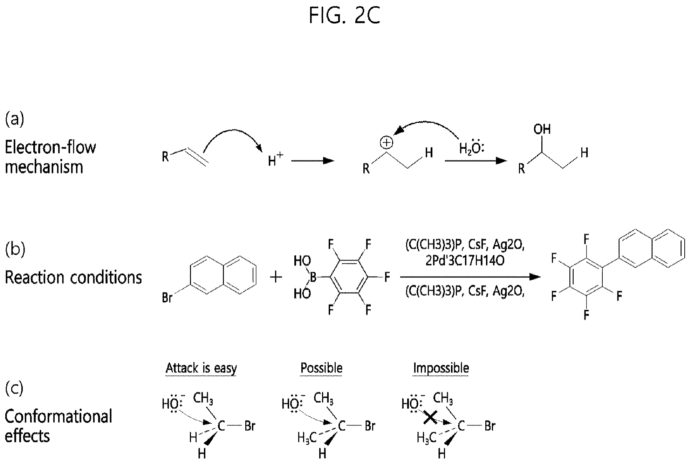

In an embodiment, as illustrated in (a) to (c) of FIG. 2C, the chemical reaction prediction model 400 may generate electron flow, reaction conditions, and structural effects of a molecular structure (or chemical structure) using the output data output from the first module 400a and the second module 400b. In this case, the electron flow, the reaction conditions, and the structural effects may be expressed together as the graph and text, the molecular structure reflecting the position before and after the electron moves may be generated, or the molecular structure of the product generated according to the reaction conditions may be generated.

That is, the chemical reaction prediction model 400 can make more accurate predictions than using only a single data source by fusing the output data 404a and 404b output from the first module 400a and the second module 400b, respectively, and may enable users to intuitively recognize various elements related to chemical reactions.

In addition, the chemical reaction prediction model 400 may verify the chemical reaction products predicted through the first module 400a using the output data analyzed in the second module 400b. That is, the second module 400b may acquire at least one of the chemical reaction conditions, chemical reaction mechanisms, and chemical characteristics analyzed based on the text data 403b. The chemical reaction prediction model 400 may verify whether the chemical reaction products predicted and acquired in the first module 400a match the experimental data or theoretical expectations based on the data analyzed in the second module 400b.

Meanwhile, the chemical reaction prediction model 400 according to the present invention is characterized by performing the chemical transformation based on the molecular graph, and for understanding, the configuration of the molecular structure graph will be schematically described.

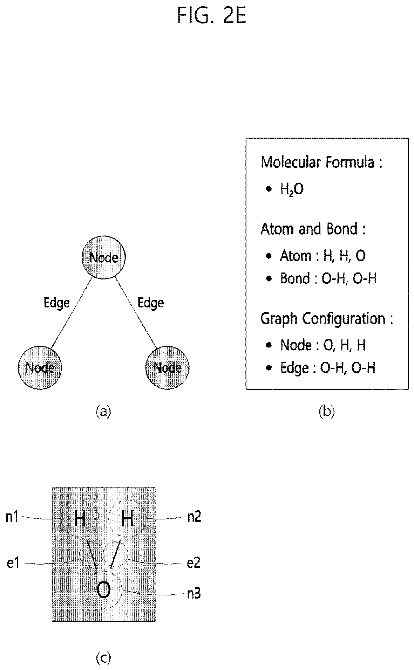

(a) of FIG. 2E illustrates nodes and edges, and in the molecular graph, atoms may be expressed as nodes and bonds between atoms may be expressed as edges. For example, as illustrated in (b) of FIG. 2E, a water molecule has a molecular formula of "H2O". In this case, atoms are composed of H, H, and O. In this case, the number of nodes is three, and as illustrated in (c) of FIG. 2E, the atoms H, H, and O may be expressed as nodes n1, n2, and n3, respectively. In addition, there may be two bonds between atoms, O-H and O-H, and their bonds may be expressed as edges e1 and e2, as illustrated in (c) of FIG. 2E. In this way, when converting the molecular structure into the molecular graph, the unique positional relationship and topological relationship of the molecular structure may be preserved, so it is possible to implement more accurate prediction. In the present invention, the molecular structure to be predicted is converted into the molecular graph and input to the encoder 410 to predict the chemical reaction.

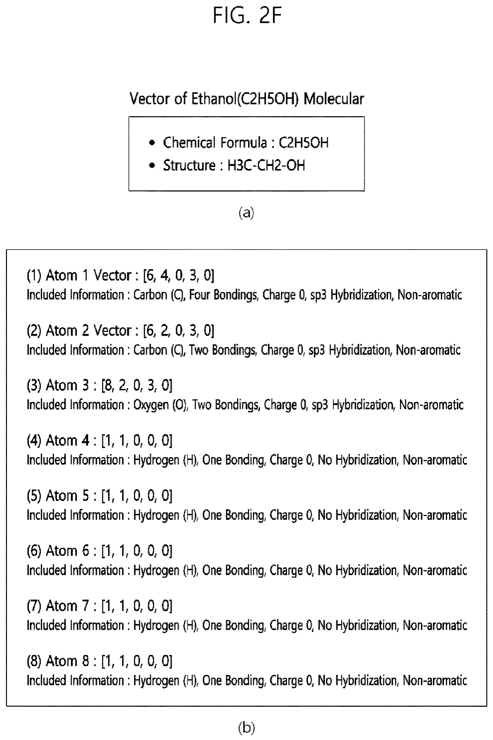

Meanwhile, in the present invention, the encoder 410 may embed the molecular structure into a vector using the molecular graph. In this case, briefly describing the vector, as illustrated in (a) of FIG. 2F, an ethanol (C2H5OH) molecule may represent ethanol through a vector having a specific dimension, as illustrated in (b) of FIG. 2F. For example, a vector corresponding to each atom of ethanol may be expressed as illustrated in (b) of FIG. 2F. In this case, the dimension of the vector and the included information may be set in various ways. For example, (b) of FIG. 2F illustrates a five-dimensional vector, and each vector may include atom type, binding information, charge information, hybridization information, directionality information, etc. Meanwhile, although not illustrated, the encoder 410 may embed a vector for the binding information of the molecule and utilize the embedded vector for analysis.

Hereinafter, a method of predicting a chemical reaction based on a molecular graph and acquiring the chemical reaction products according to the chemical reaction prediction model 400 according to the present invention will be examined in more detail. The description below may be performed in the first module(ex: the encoder 410) illustrated in FIG. 2B.

The chemical reaction prediction and organic synthesis are one of the important challenges in new drug development and/or material science, and it is very important to predict the results of the chemical reaction in designing new molecules, which may greatly contribute to shortening a product development cycle in various industrial fields. Conventionally, a sequence-based model using a SMILES string was utilized to predict chemical reactions and/or retrosynthetic path. The SMILES strings encode chemical structures (or molecules or molecular structures) into ASCII strings, and thus, may be processed as text data through natural language processing technology.

However, the SMILES string does not match the natural graph representation of molecules, so learning efficiency is low, and chemically invalid transformations (or modifications) may be generated. In particular, in the case of the chemical reactions, the transformations in the SMILES space may not directly correspond to valid molecular graphs, so chemically infeasible outputs may occur. That is, there is a limitation that such transformation does not always lead to a valid modification in the graph space, which is a more natural representation of molecules.

To solve this problem, various graph-based (or centric) approaches that represent molecules as graphs have been proposed. The graph representations may accurately represent the structure of molecules, increase the interpretability of chemical reaction mechanisms, and facilitate more accurate chemical reaction predictions based on electron flow theory.

Accordingly, the present invention provides a chemical reaction prediction model based on an electron flow using graph diffusion that models the movement of electrons through the graph structure of molecules to better understand the chemical reaction mechanisms and accurately predict the results of the chemical reaction. The prediction method according to the present invention may also be named an electron-flow inspired graph diffusion model for interpretable forward reaction prediction method.

The chemical reaction prediction model described in the present invention operates in the graph space for both inputs and outputs using the graph diffusion, which maintains invariance of the permutation and order of the SMILES strings while providing more interpretable transformations to users. The chemical reaction prediction model according to the present invention may train a conversion process starting from an initial graph set rather than starting from random graph sampling and leading to a final product.

Hereinafter, the present invention will be described in more detail with reference to the accompanying drawings.

As illustrated in FIG. 4A, the chemical reaction prediction model 400 according to the present invention may include the encoder 410, the decoder 420, a graph diffusion module 430, and a training unit 440. As illustrated, the chemical reaction prediction model 400 according to the present invention may perform a series of data processing by cooperation of a memory and at least one processor. Various types of information may be stored in the memory. For example, the memory may store various types of information such as information input to the chemical reaction prediction model 400, products generated according to a series of data processing in the chemical reaction prediction model 400, information on intermediate products, and information on the final product. The processor may cooperate with each component of the chemical reaction prediction model to allow the process for chemical reaction prediction according to the present invention to proceed in each component and a neural network(or a neural network layer).

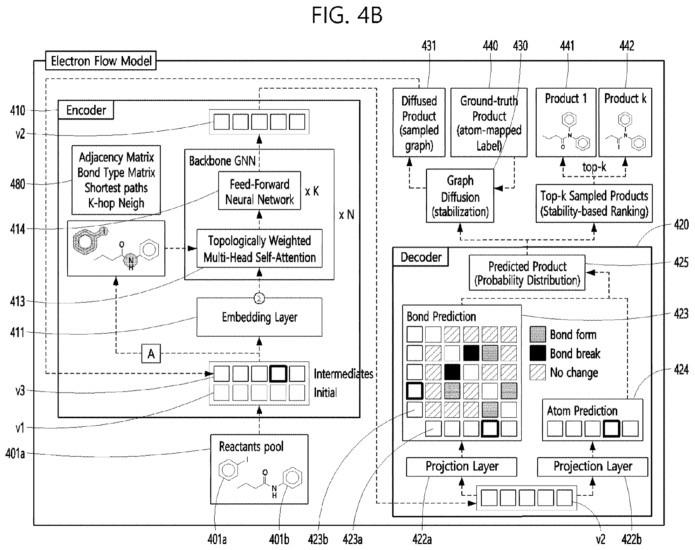

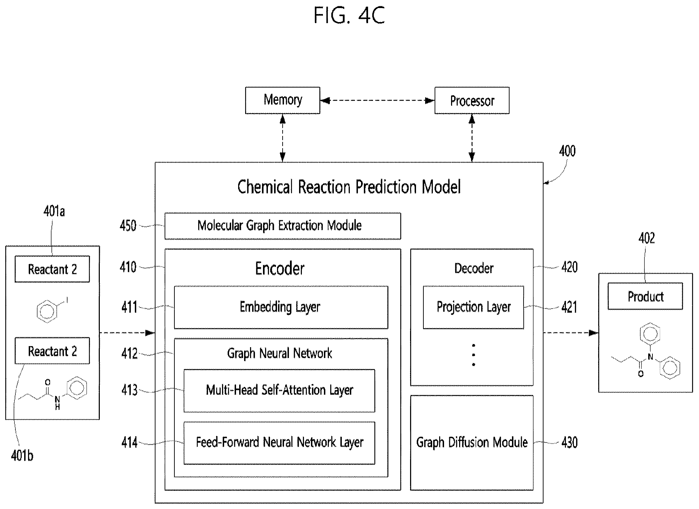

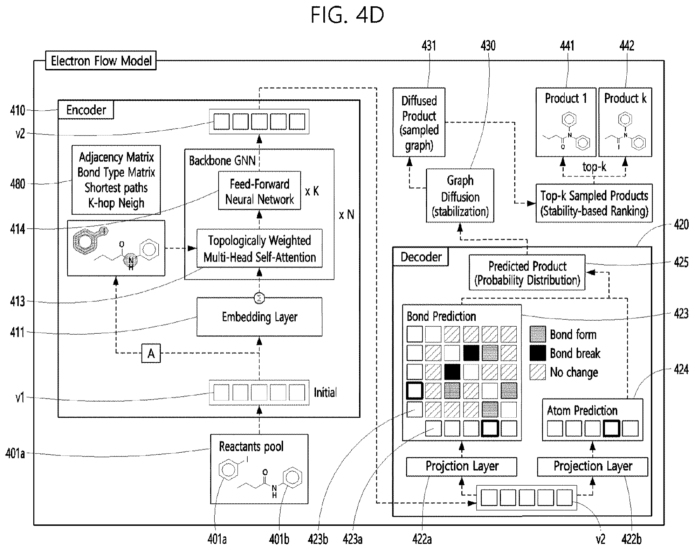

The chemical reaction prediction model 400 according to the present invention has the training unit 440 and a ground-truth product as illustrated in FIGS. 4A and 4B in the training step, but after the training is completed, the training unit and the ground-truth product may be excluded as illustrated in FIGS. 4C and 4D.

The encoder 410 according to the present invention may be configured as a backbone encoder. The encoder 410 according to the present invention is configured as an attention-based graph neural network (GNN) and may perform relative position encoding based on the shortest path between the nodes (atoms). Meanwhile, the diffusion process described in the present invention does not depend on the backbone network and may also be implemented with various encoders.

The encoder 410 according to the present invention may include an embedding layer 411 and graph neural networks (GNN) 412 as illustrated. The graph neural network may be the attention-based graph neural network or the backbone neural network.

Meanwhile, the graph neural networks 412 may include a multi-head self-attention layer 413 and a feed-forward neural network layer. The multi-head self-attention layer may also be named a topologically weighted multi-head self-attention layer.

The embedding layer 411 is configured to receive information related to plurality of molecular structures and convert the received information into the embedding vector. The embedding layer 411 may be configured to generate a vector including atoms of the molecular structure and a bond relationship between the atoms. The graph neural networks 412 may receive the embedding vector embedded in the embedding layer 411, perform an attention operation related to the interaction between the atoms of the plurality of molecular structures, and update the embedding vector based on the operation. In this case, the embedding layer may receive the molecular graph and acquire the embedding vector based on the molecular graph.

Meanwhile, the node features contained in the embedding vector output by the embedding layer 411 may include an atom type, an atom charge, the number of implicit hydrogens, and the number of radical electrons. The embedding layer 411 may generate the embedding vector including information that may be used in the encoder by encoding the nodes according to the molecular graph. In this way, the molecular graph may be completely reconstructed into each molecule, and the degree of the node may also be tokenized as an input function and used to measure the consistency between the bond prediction and the predicted atomic degree later.

Features of an atom v may be represented as (f), (g), and (h) of FIG. 5A, and may be tokenized and embedded through the embedding layer 411.

As illustrated in (g) of FIG. 5A, the features of the atom v are tokenized and embedded through the embedding layer 411, and the tokenized features of type i according to (f) of FIG. 5A may have features as illustrated in (g) of FIG. 5A. i is an index indicating a type of atomic features, and may belong to a set of type, charge, hydrogen, radical, and degree. i may mean at least one of the features of type, charge, hydrogen, radical, and degree. Here, the type may mean an atom type (e.g., carbon, oxygen, etc.), the charge may mean a formal charge of an atom, the hydrogen may mean the number of implicit hydrogens, the radical may mean the number of radical electrons of the atom, and the degree may mean a degree of a node (the number of other atoms bonded to the corresponding atom). These features are embedded through the embedding layer 411, as illustrated in (h) of FIG. 5A, to form the initial embedding of the atom. The initial embedding of the atom v may be an average of all the embeddings.

The embedding layer 411 may perform embedding for the plurality of molecules based on the molecular graph. The molecular graph for the molecular structure may be stored in a memory, a dataset, a database, etc. In contrast, the molecular graph for the molecular structure may be extracted by a molecular graph extraction module 450. The chemical reaction prediction model 400 may further include the molecular graph extraction module 450 or may cooperate with the molecular graph extraction module 450. Furthermore, the molecular graph extraction module 450 may be included as a component of the encoder 410.

The molecular graph may maintain a plurality of molecular structures by expressing the atoms as the nodes and the bonds between the atoms as the edges.

The structure of the plurality of molecules may include a first molecular structure and the second molecular structure, and the first and second molecular structures may be named reactants. In FIG. 4A, they correspond to reference numerals 401a and 401b.

The molecular graph extraction module 450 may acquire the molecular graph for the plurality of molecular structures by converting the atoms into the nodes and the bond relationship between the atoms into the edges based on the plurality of molecular structures using a pre-specified graph transformation algorithm.

The molecular graph extraction module 450 may acquire a first molecular graph including the nodes and edges corresponding to the first molecular structure by converting the atoms constituting the first molecular structure into the nodes and the bond relationship between the atoms constituting the first molecular structure into the edges. Similarly, the molecular graph extraction module 450 may acquire the second molecular graph including the nodes and edges corresponding to the second molecular structure by converting the atoms constituting the second molecular structure into the nodes and the bond relationship between the atoms constituting the second molecular structure into the edges using the pre-specified graph transformation algorithm.

In the embedding layer 411 of the encoder 410, the embedding vector corresponding to the plurality of molecular structures may be acquired using the information related to the plurality of molecular structures. In this case, the information related to the plurality of molecular structures may include the information on the nodes and edges corresponding to the first molecular structure and the information on the nodes and edges corresponding to the second molecular structure. The information related to the molecular structure may be the molecular graph extracted by the molecular graph extraction module 450 or the information extracted from the molecular graph.

The embedding vector may include at least one of the pieces of information on the atom type, the atom charge, the number of hydrogens, the number of radical electrons, and the degree of the node corresponding to the first molecular structure and the node corresponding to the second molecular structure. Furthermore, the embedding vector may be configured to further include the information on the bond relationship between the atoms of the first and second molecular structures.

Meanwhile, when the reactants to be predicted, i.e., the plurality of molecular structures (e.g., the first molecular structure and the second molecular structure 401a and 401b) are specified, the chemical reaction prediction model 400 may extract at least one of an adjacency matrix, a bond type matrix, shortest paths, and K-hop neighbors corresponding to the first molecular graph and the second molecular graph, respectively, using the nodes and edges corresponding to the first molecular graph and the nodes and edges corresponding to the second molecular graph. The time point at which the information 480 is extracted is not limited and may vary.

The adjacency matrix may include information on direct connections between the nodes constituting the first molecular graph and the second molecular graph.

The bond type matrix may include information on the bond types between the nodes constituting the first molecular graph and the second molecular graph.

The shortest paths matrix may include information on lengths of the shortest paths between the nodes constituting the first molecular graph and the second molecular graph.

The K-hop neighbors may include information on neighboring nodes within K steps for each of the nodes constituting the first molecular graph and the second molecular graph.

At least one 480 of the adjacency matrix, the bond type matrix, the shortest paths, and the K-hop neighbors extracted in this manner may be stored in the memory. At least one of the adjacency matrix, bond type matrix, shortest paths, and K-hop neighbors stored in the memory may be input to at least one of the embedding layer 411, the graph neural networks 412, the multi-head self-attention layer 413, and a feed-forward neural network 414.

In the embedding layer 411, when the embedding vector v1 corresponding to the plurality of molecular structures (or reactants, for example, the first molecular structure and the second molecular structure 401a and 401b) is acquired, the acquired embedding vector v1 may be input to the graph neural networks 412. In particular, the embedding vector v1 may be input to the multi-head self-attention layer 413 of the graph neural networks.

The multi-head self-attention layer 413 may perform an attention operation related to the interaction between the atoms of the plurality of molecular structures using the embedded embedding vector v1 based on the molecular graph, and update the embedding vector based on the operation. In this case, the embedding vector v1 to be updated may be a vector output from the embedding layer.

The multi-head self-attention layer 413 may perform global attention-based pooling. In order to consider the molecular graph topology, the multi-head self-attention layer 413 may add a bias to the attention weight score derived from the shortest paths between node pairs. This may act as a relative position encoding between the atoms. As a result, it may be free from the spectrum-based encoding problem (e.g., lack of sign invariance for Laplace matrix eigenvectors) because the distance measurement between the nodes is not absolute. Such encoding has high computation efficiency, and may take into account the entire graph topology while allowing the global message delivery. In this case, the calculation time of the shortest paths may vary.

The global attention-based pooling is one that considers the entire topology of the graph, and the multi-head self-attention layer 413 may globally consider the correlation between each node of the graph by performing the global attention-based pooling.

For example, when the dimension of the input vector from the embedding layer is assumed to be 128, and the number of heads in the multi-head attention is assumed to be 8, a 16-dimensional sub-vector obtained by dividing 128-dimensional input vectors by the number of heads which is 8 may be input to each head.

Each head of the multi-head self-attention layer 413 is configured to independently calculate attention values for 16-dimensional sub-vectors by applying the preset attention mechanism. For example, a first head may mainly reflect information of adjacent nodes in the molecular graph, and a second head may mainly reflect information of a specific bond type in the molecular graph. The 16-dimensional attention vectors calculated from a plurality of heads of the multi-head self-attention layer 413, for example, 8 heads, respectively, may be combined again to become a 128-dimensional vector. This combined vector may have the same dimension as the embedding vector output from the embedding layer 411, but may include richer information by integrating various types of information extracted from each head. In the present invention, this may be expressed as the updated embedding vector.

Meanwhile, in order to reflect the shortest paths between the nodes in the weight, as described above, in the present invention, the shortest paths between all pairs of nodes in the molecular graph may be calculated. The shortest paths may be calculated by a preset algorithm. In the multi-head self-attention layer 413, the relative positions between the nodes may be encoded using the calculated shortest path information. This encoding may be performed based on the shortest path distance between the nodes. The multi-head self-attention layer 413 may use the shortest path information as the bias when calculating the attention weight score between the nodes. For example, the multi-head self-attention layer 413 may adjust the attention weight in such a way that the closer the shortest path distance between two nodes, the higher the attention weight, and the farther the shortest path distance between two nodes, the lower the attention weight. The multi-head self-attention layer 413 may add the bias derived from the shortest path between the node pairs to the attention weight score.

The multi-head self-attention layer 413 encodes each node of the molecular graph using the attention weight adjusted based on the shortest paths in this way, which enables the encoding that considers the topology of the entire molecular graph.

In addition, in the multi-head self-attention layer 413, the bias may be added to the attention score according to the bond type (e.g., single, double, triple, and aromatic bonds).

For example, in the multi-head self-attention layer 413, different biases may be added to the attention score calculated by the multi-head self-attention layer according to the bond type between the nodes constituting the first molecular graph and the second molecular graph.

Here, the bond type may include a single bond type, a double bond type, a triple bond type, and an aromatic bond type.

This may mean that, instead of a single bias matrix for all heads, each head has its own bias matrix. For example, the bias of the shortest path may be added to the first head, and the bias according to the single bond may be added to the second head. The remaining heads may use a mask that focuses on the global attention between the atoms or molecules. In the present invention, in the multi-head self-attention layer 413, such various bias matrices may be added to each layer according to the multi-head. According to the present invention, by adding the bias matrix to each layer corresponding to the multi-head, the attention score of each head may emphasize pieces of information masked by the bias matrix. For example, when the bias matrix according to the bond type is added, the head may be masked according to the bond type and receive information only from neighbors connected by a specific bond type. For example, a specific head may receive information from the nodes connected only by the single bond.

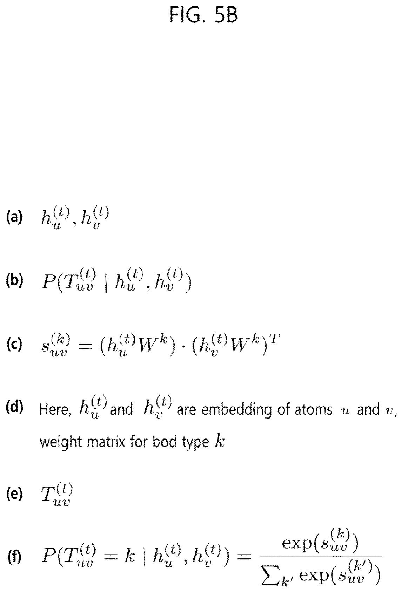

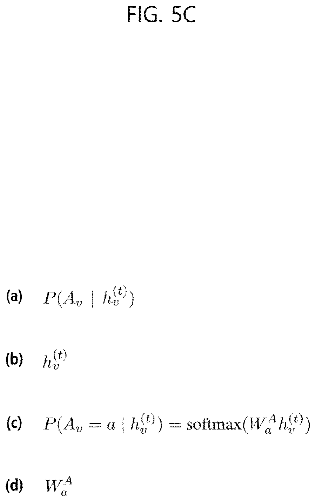

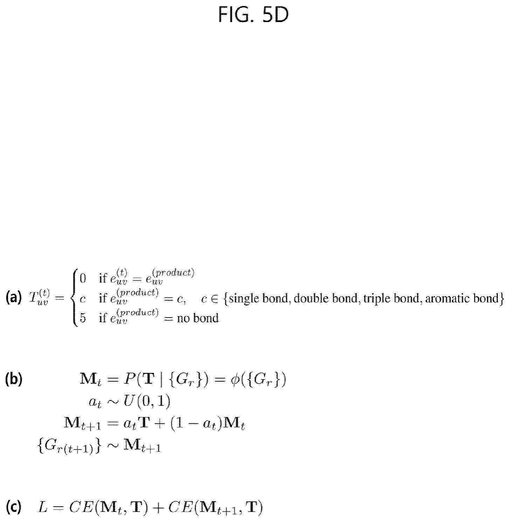

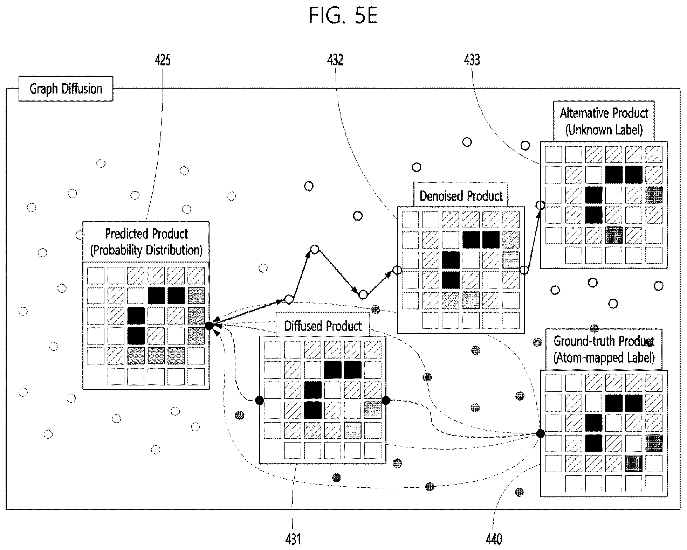

In this way, the weight for the attention score according to the present invention, which adds the bias reflecting various characteristics of the molecular structure, may be calculated according to the formula and meaning illustrated in FIG. 5. Here, A(Qk, Kk, Vk) may mean the attention weight calculated by applying the Softmax function to the attention score.

In this way, in the multi-head self-attention layer 413, when the attention operation related to the interaction between the atoms of the plurality of molecular structures is performed and the embedding vector is updated based on the operation, the updated embedding vector may be stored in the memory through the update step. Then, the updated embedding vector stored in the memory may be input to a feed-forward neural network layer 414. The feed-forward neural network layer 414 may receive the output of the multi-head attention layer 413 and additionally update the updated embedding vector. The feed-forward neural network layer 414 may update the output vector of the multi-head self-attention layer using at least one of the adjacency matrix, bond type matrix, shortest paths, and K-hop neighbors. The feed forward neural network layer 414 may update the output vector of the multi-head self-attention layer so that the structural information on the molecular structures is included more abundantly. The output vector of the feed forward neural network layer may be specified as a final updated embedding vector v2 input to the decoder 420.

Furthermore, the decoder 420 according to the present invention may include at least one projection layer 421. In the decoder 420, the bond prediction and atom prediction based on the plurality of molecular structures (reactants) may be performed based on the updated embedding vector v2 input to the projection layer. The bond prediction is to predict the bonds between the atoms, and the atom prediction is to predict the state change of the atoms. The decoder 420 can include a plurality of projection layers 422a and 422b. In each projection layer 422a and 422b, bond prediction 423 and atom prediction 424 may be performed.

In the decoder 420, the bond prediction may be performed by performing the dot-product using the updated embedding vector. The dot-product may be performed for each vector corresponding to each atom pair of the atoms corresponding to the updated embedding vector.

In the block 423 of the drawing, each box of x-axis and y-axis 423a and 423b conceptually represents each atom of the molecules to be reacted, and these atoms may be represented by the updated embedding vector v2. The decoder 420 may predict the bond type between each atom through the dot-product based on the information included in the updated embedding vector.

The decoder 420 may acquire inner product values for each of the plurality of bond types for each atom pair based on the dot-product. The plurality of bond types may be related to at least one of the single bond, double bond, bond formation, bond collapse, and no change.

The decoder 420 may generate a probability distribution for each of the plurality of bond types for each atom pair using the inner product values according to the dot-product. The decoder 420 may acquire a transformation matrix that predicts the change in the bonded state change of each atom pair using the probability distribution.

The decoder 420 may generate the probability distribution for the plurality of bond types for each atom pair by applying the Softmax function to the inner product values acquired for the plurality of bond types for each atom pair.

The decoder 420 may generate the atom property probability distribution for each atom corresponding to the updated embedding v2 vector using a Softmax output layer. The decoder 420 may predict the atom properties of the atoms corresponding to the updated embedding vector using the probability distribution. The atom properties may include the charge state of the atoms that may change during the chemical reaction process of the structure of the plurality of molecules.

The decoder 420 may perform the inner product between the updated embedding vectors v2 (or atom embedding vector, as illustrated in (a) of FIG. 5B) for the atoms, and calculate a score for potential bond transformation as illustrated in (b) of FIG. 5B. Here, the lowercase letter t may represent the step of the diffusion process

This is based on a standard query-key matrix multiplication of a transformer attention mechanism, but in the present invention, the key and the query may share the same projection head. The setting that allows the key and the query to have the same projection head may require a plurality of projections to accommodate various bond types such as none, the single bond, the double bond, the triple bond, and the aromatic bond. This corresponds to the multi-head attention in the transformer, and if this is expressed in a formula, this may be represented as (c) of FIG. 5B. In this way, bond prediction may be performed in the bond prediction head. This may correspond to the first projection layer 423a illustrated. That is, the bond prediction may be performed in the first projection layer 423a. The first projection layer 423a may be composed of layers that constitute the bond prediction head.

Each bond type prediction may be viewed as a head in the multi-head attention scheme, where Softmax regularization is applied to a state such as (d) of FIG. 5B to acquire the distribution of the bond transformations for each atom pair. Here, (d) of FIG. 5B may mean the bond transformation.

In the bond prediction head, as illustrated in (c) of FIG. 5B, a score S is calculated for each bond type k, where the meanings of each symbol are as illustrated in (d) of FIG. 5B.

Meanwhile, in the bond prediction head, the probability of the bond type k of the bond transformation T according to (e) of FIG. 5B may be calculated as illustrated in (b) of FIG. 5B. In the bond prediction head, the Softmax regularization such as (f) of FIG. 5B may be applied to acquire the probability distribution of the bond transformation.

In the present invention, each bond type prediction may be understood as each head in the multi-head attention system. The decoder 420 may acquire a normalized probability distribution for the bond transformations of each atom pair by applying the Softmax normalization to the possible states of the bond transformation according to (e) of FIG. 5B. This may provide an efficient and scalable method of directly predicting the bond transformation in the embedding space.

Next, the atom prediction may be performed in the second projection layer 423b of the decoder 420. The second projection layer 423b may be composed of layers that constitute the atom prediction head. Since the features of some atoms may change during the chemical reaction, the decoder 420 according to the present invention may perform prediction on the atoms in addition to predicting the bond transformation between the atoms. The atom prediction may be performed in the second projection layer 423b. To this end, a standard Softmax output layer may be used for features of each atom. This includes the degree (i.e., the number of single bonds included in the atom) of the atom for each bond type, and the prediction may help ensure consistency between independent parallel samples of the bond transformations and between the atom transformations.In the atom prediction head, as illustrated in (c) of FIG. 5C, the probability that the feature Av of the atom v becomes a at step t of the diffusion process may be calculated. Here, (b) of FIG. 5C represents the embedding of the atom v, and (d) of FIG. 5C represents the weight matrix of the prediction head for the atomic feature a. In the atom prediction head, the probability that the feature Av of the atom becomes a may be calculated by multiplying embedding h (see (b) of FIG. 5C) of the atom v by a weight matrix W (see (d) of FIG. 5C) and then applying the Softmax function.

The decoder 420 may acquire a predicted chemical reaction product 425 (probability distribution) predicted from the chemical reaction of the plurality of molecular structures by combining the bond prediction probability and the atom prediction probability of the atoms output from the projection layers 423a and 423b. The chemical reaction product predicted from the projection layers may also be called an initial chemical reaction product.

In the present invention, the predicted chemical reaction products may be input to the graph diffusion module 430, and the graph diffusion module 430 may sample the initial chemical reaction products using the bond prediction result and the atom prediction result. The graph diffusion module 430 may stabilize the sampled initial chemical reaction products through the diffusion feedback process. The chemical reaction prediction model may acquire the final chemical reaction products stabilized through the diffusion feedback process.

In the diffusion feedback process, the graph diffusion module 430 may repeatedly evaluate each bond transformation of the initial chemical reaction products, and remove or change unstable bonds. Furthermore, the graph diffusion module 430 may evaluate the predicted bond transformation at each step repeatedly performed using the transformation probability matrix and the target transformation matrix to predict the change in the bonded state of the initial chemical reaction products, and generate the final chemical reaction products using the interpolation factor.

In the present invention, since the bond prediction is performed independently in the entire molecular graph, a method of finally acquiring a valid molecular structure is required. For example, in the prediction process, there may be cases in which the bonds to the plurality of atoms are predicted or the bonding rules are violated. In order to solve this problem, a process of denoising into a valid final state is required.

As illustrated in FIG. 5E, the stable final state may be generated through a plurality of sampling and denoising processes 432 for the predicted chemical reaction product.

FIG. 5E illustrates the diffusion process from the correct answer data (or target label (ground truth product) 440) to the initial distribution 425, and this process is based on the initial distribution derived from the dot product matrix of the bond transformation predictions between all the atom pairs. Here, the target label corresponding to the correct answer data may include actually bonded states and atom states corresponding to the plurality of molecular structures input to the encoder. Here, the initial distribution 425 means the initial chemical reaction product 425 output from the decoder, which may correspond to the probability distribution. Meanwhile, the diffusion process to the target label is performed in the learning process in the training unit 440, and in the actual inference step, the graph diffusion module 430 for which the learning has been completed may be used. In this case, the predicted chemical reaction product 425 may be input to the graph diffusion module 430 for which the learning has been completed, and the final chemical reaction product may be acquired through the denoising process.

The training unit 440 may calculate a loss function between the final chemical reaction product output from the decoder and label data (hereinafter, referred to as "target label") including the actually bonded states and atom states corresponding to the plurality of molecular structures, and may optimize a parameter of at least one of the encoder and the decoder to minimize the loss function.

Meanwhile, in the learning process, the target label may be sampled at random points in the interpolated space by interpolating with the initial distribution, and FIG. 5E illustrates a noise graph in which these random points are denoised with the target label. During the prediction, the results sampled from the initial distribution are updated at each step to lead to the stable final product molecule, and a similar process may be applied to the atomic features.

In the diffusion step, the predicted product may correspond to the initial distribution acquired through the dot product matrix of the bond transformation prediction for all the atom pairs in the decoder 420, as described above. This initial distribution represents the probability distribution of the bond formation between the atoms. The interpolated product is a product in which a target label (ground-truth product) is interpolated with an initial distribution, and in the present invention, a noisy graph may be acquired by sampling the random points in this interpolated space. These random points represent the noise graph denoised with the target label. The denoised product denoises the sampled noise graph to gradually approach the target label, and this process includes several steps, and the graph may change more stably at each step. The alternative product means that in some cases, an alternative product may come out through different paths, which may have a different combination configuration from the target label.

In this way, in the present invention, the decoder 420 acquires the initial distribution, and the graph diffusion module 430 samples the initial distribution to sample the random points. The graph diffusion module 430 interpolates the target label with the initial distribution through the interpolation and denoising process to create the intermediate state. During the learning process, the intermediate state v3 may be input back to the encoder. The graph diffusion module 430 samples the random points to acquire the noisy graph, and gradually approaches the target label through the denoising process. This process may be performed repeatedly, and at least one of the encoder, decoder, and graph diffusion module may be trained in the repetitive process.

To train the method of denoising the predicted chemical reaction product 425, the graph diffusion module 430 may be configured to train a method of diffusing the target label to a previous output distribution and mapping the state in the diffused interpolation space to the final target label.

To this end, a transformation probability matrix Mt and a desired target transformation matrix T may be defined, and the transformation matrix T may be derived from the bond prediction result of the atom. The result of the bond prediction of the atom may be acquired from the decoder and may be derived from the bond prediction head described above. This may be expressed as the atom-mapped label of the reaction. The transformation matrix T may identify whether the bond is formed or broken by finding the difference between the input and output adjacency matrices, and the transformation matrix T may be expressed as in (a) of FIG. 5D. This defines the difference between the adjacency matrix of the reactants and products, and the adjacency matrix may represent the entire bond type matrix including the single/double/triple/aromatic bonds.

Meanwhile, the reactants are time-dependent because they keep changing at each diffusion step. This means that the target label also changes at each step, and in the present invention, a method of predicting when and where the difference between the reactants and the target label occurred (reaction center) and what changed (final bond configuration) when there was a difference may be trained as in (b) of FIG. 5D. A parameter at is a randomly sampled interpolation factor that determines the weight between the current transformation matrix and the target transformation matrix. During the training, the diffusion process may include acquiring the transformation probability Mt from an initial reactant set {Gr} and sampling {Gr(t+1)} to be used as the input of the next step from Mt+1. Both steps may aim to directly predict the target transformation matrix T. Therefore, the training loss may be defined as in (c) of FIG. 5D. During the prediction, the input {Gr} may be simply encoded, the prediction head may be used to acquire the probability of the bond transformation Mt, and the sample may be performed from the distribution at each step through standard categorical Softmax sampling. In the learning process of the present invention, this process may be repeated continuously and the results may be fed back until the stable configuration, in which the bond no longer changes, is acquired.

Meanwhile, in the present invention, a Top-K ranking may be applied to a plurality of stabilized products in the diffusion module 430 to derive the final chemical reaction product. This may be performed in the Top-K sampled product module of the chemical reaction prediction model 400. The Top-K ranking process may be performed in an inference stage. That is, the Top-K ranking process may be utilized to extract products with a high probability in order to provide the final product to the user. In the present invention, at least one of the Top-k sampling and Top-K ranking may be performed, which may be used to acquire more diverse results in the inference process. In the Top-k sampling, the chemical reaction prediction model may generate a plurality of samples based on the trained contents, and may diversify the distribution by adjusting a temperature parameter τ. The Top-k ranking is used to select the most likely result among the generated samples, calculate the probability of each sample, and select the result with the highest probability as the final prediction.

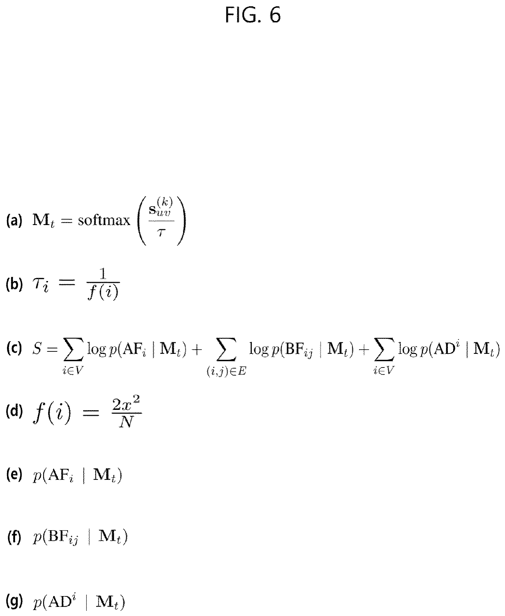

In the Top-k sampling process, when sampling the distribution Mt at time step t, logits may be readjusted by the temperature parameter τ. This may adjust the Softmax distribution illustrated in (a) of FIG. 6 more sharply or more uniformly.

Meanwhile, in order to acquire the plurality of samples, the temperature parameter τ may be used in the present invention. In the present invention, when N samples are to be extracted, τi may be set as in (b) of FIG. 6. Here, f(i) is a temperature sampling schedule, and in the present invention, f(i) may be defined as in (c) of FIG. 6. Here, x is a linspace between 0 and 1.

Next, the Top-K ranking will be described. When there are sampled candidates, a method is needed to rank the sampled candidates according to the probability. This may be calculated from the transformation matrix Mt. When the sampled state is considered as a sampled token for the bonds to each atom, the probability for each state may be derived from the sampled Softmax distribution Mt. Through this, the total probability for the final overall configuration (bonded state to atom) of the molecule may be assigned, and the sampled probability S may be calculated. This may be represented as in (d) of FIG. 6. Here, each of (e), (f), and (g) of FIG. 6 may be derived from the Softmax distribution for the atomic feature, the bond feature, and the atomic degree. In addition, the term of (d) of FIG. 6 may ensure consistency between the predicted atomic degree and the actual degree calculated from the bond. In the present invention, since the bonds are sampled independently, the plurality of bonds may be formed on the same atom. Therefore, the actual degree of each atom is directly calculated in the sampled graph, and compared with the probability distribution of the predicted degree, and in this way, a high probability score is assigned when the actual degree of the generated molecule matches the predicted degree.

In addition, since there is no guarantee that all solutions are unique when sampling independently from the distribution, many duplicate samples may occur. Therefore, in the present invention, during the prediction, the Top-k product may be selected by taking a simple unique graph.

Meanwhile, this is described based on FIGS. 4A and 4B, which include the learning process of the chemical reaction prediction model, but as described above, the chemical reaction prediction model may have the configuration of FIGS. 4C and 4D in the inference process. In this case, since all may be understood equally except for the learning process, the detailed description will be replaced with the above description.

In the inference process, as described above and as illustrated in FIG. 3, the final chemical reaction product may be obtained by a process (S310) of receiving the information related to the plurality of molecular structures including the first molecular structure and the second molecular structure as the input of the encoder, a process (S320) of acquiring the embedding vector corresponding to the first molecular structure and the second molecular structure using the information related to the plurality of molecular structures, in the embedding layer of the encoder, a process (S330) of performing the attention operation related to the interaction between the atoms of the first molecular structure and the second molecular structure and updating the embedding vector based on the operation, in the multi-head self-attention layer, and a process (S340) of storing the updated embedding vector through the update step in the memory and inputting the updated embedding vector stored in the memory to the decoder, a process (S350) of performing the bond prediction and the atom prediction predicted as the chemical reaction of the first molecular structure and the second molecular structure using the updated embedding vector in the decoder, and a process (S360) of obtaining the final chemical reaction product predicted from the chemical reaction of the first molecular structure and the second molecular structure using the result of the bond prediction and the result of the atom prediction.

The process for acquiring the final chemical reaction product may be performed through the answer generation system 100 as illustrated in FIG. 1. In this case, the answer generation system 100 may receive the user query, and the user query may include the information on the molecular structure that is the target of the chemical reaction prediction.

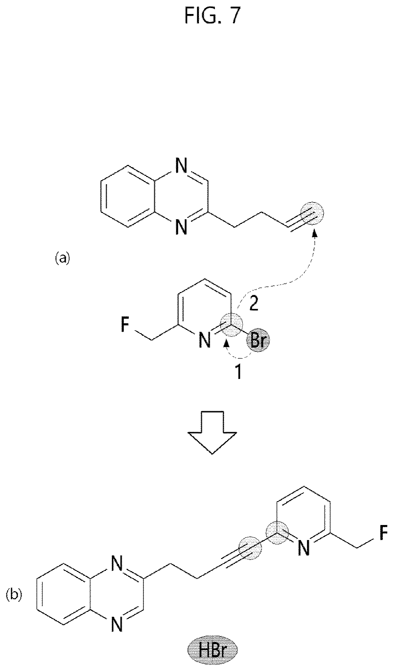

In the chemical reaction prediction model 400 according to the present invention, as illustrated in (b) of FIG. 7, the chemical reaction product may be acquired through the bond prediction and atom prediction for the plurality of molecular structures that are the targets of the chemical reaction as illustrated in (a) of FIG. 7.

As described above, according to the chemical reaction prediction system and the control method thereof, and the learning method of a chemical reaction prediction system according to the present invention, by modeling the movement of electrons through the graph structure of molecules, it is possible to better understand the chemical reaction mechanisms and accurately predict the results of the chemical reaction.

In addition, according to the chemical reaction prediction system and the control method thereof according to the present invention, and the learning method of a chemical reaction prediction system, by providing the chemical reaction prediction model that can accurately predict the results of the chemical reaction and better understand the chemical reaction mechanisms, it is possible for a user to reduce the time and cost required for experiments. Accordingly, it is possible to reduce the research and development costs and shorten the time to market for new products.

Meanwhile, according to the chemical reaction prediction system, the control method thereof, and the learning method of a chemical reaction prediction system according to the present invention, by performing both the input of the molecular structure and the output of the predicted product in the graph space, it is possible to perform the forward reaction prediction regardless of the SMILES permutation and order.

Furthermore, according to the chemical reaction prediction system, the control method thereof, and the learning method of a chemical reaction prediction system according to the present invention, by simultaneously sampling multiple interdependent transformations that occur in parallel within the molecular graph, it is possible to secure the consistency between the transformations.

Furthermore, according to the chemical reaction prediction system, the control method thereof, and the learning method of a chemical reaction prediction system according to the present invention, in order to solve problems that may occur due to the symmetrical structure, by breaking the symmetry and forming the valid output structure by including the noise or sampling mechanisms, it is possible to prevent the occurrence of the invalid configuration.

Next, the document understanding model 300 may extract various types of content that satisfy preset contents criteria from documents (e.g., papers, books, patent documents, reports, etc.). More specifically, the document understanding model 300 may be a model trained to understand structured data, unstructured data, linguistic data (or linguistic elements), non-linguistic data (or non-linguistic elements), etc., included in a document, and extract various content (or data) and knowledge based on the understood contents.

Here, the preset content criteria may be set in various ways and may be determined according to the purpose or utilization purpose of the answer generation system 100 according to the present invention.

For example, when the utilization purpose of the answer generation system 100 is the chemistry, the bio, the new materials, the new substances, and the new drug development, the document understanding model 300 may be trained to understand and extract the contents related to the chemistry, the bio, the new materials, the new substances, and the new drug development from the analysis target document.

In this case, the preset content criteria may include the contents related to the molecular structures that are related to at least one of the chemistry, the bio, the new materials, the new substances, and the new drug development. Here, the document understanding model 300 may extract the contents related to the chemistry, the bio, the new materials, the new substances, and the new drug development from the analysis target document based on the preset content criteria.

In this specification, for the convenience of description, the preset content criteria are described as being related to at least one of the chemistry, the bio, the new materials, the new substances, and the new drug development, but are not necessarily limited thereto.

Based on the preset content criteria, the document understanding model 300 may extract at least one of the text, molecular structure, formula, chart, table, and image, which satisfy the preset content criteria, from the analysis target document.

In an embodiment, as illustrated in FIG. 8, the document understanding model 300 may understand the chemical structure of the molecular structure formula 21 included in the analysis target document 20, and may be extracted by converting a molecular structure formula 21 into a SMILES string expression formula 22 based on the result of understanding. In addition, the document understanding model 300 may understand the chemical structure of the molecular structure formula 21 and perform graph transformation corresponding to the molecular structure formula 21 based on the result of understanding.

In other embodiments, the document understanding model 300 may understand text 23 related to the molecular structure 21 among the texts included in the analysis target document 20, and extract the text 23 as text datarelated to the molecular structure formula 21.

In another embodiment, the document understanding model 300 may recognize rows and columns that constitute a table 24 related to the molecular structure formula 21 from the analysis target document 20 and extract structured data 25 by converting the recognized rows and columns into the structured data 25 in a format such as HTML or Excel.

Furthermore, the document understanding model 300 may extract relationship information (or relationship) between the molecular structures included in the document.

In an embodiment, as illustrated in FIG. 7, among the plurality of molecular structures included in an analysis target document 600, a third molecular structure to which a third label M3 is assigned may be understood as the molecular structure (or compound) generated as the results of the chemical reaction between the first molecular structure to which a first label M1 is assigned and the second molecular structure to which a second label M2 is assigned. The document understanding model 300 may understand the relationship between the first molecular structure and the second molecular structure included in the analysis target document 600, and extract the relationship information that the third molecular structure is generated through the chemical reaction between the first molecular structure and the second molecular structure.

In this case, the relationship information between the molecular structures may be extracted by understanding the text included in the analysis target document, or extracted by understanding non-verbal data included in the analysis target document.

In an embodiment, the document understanding model 300 may understand the relationship between the first molecular structure and the second molecular structure through symbols (e.g., plus, arrow, etc.) present in one region where the first molecular structure and the second molecular structure are located among the plurality of regions included in the analysis target document 600, and extract the relationship information that the third molecular structure is generated through the chemical reaction between the first molecular structure and the second molecular structure.

As described above, the document understanding model 300 may extract various types of data included in a document by converting the data into data (e.g., machine-readable data) in a form that the ultra-large foundation model 200 may understand. The data extracted using the document understanding model 300 may be sorted in units of pages or documents and stored in the storage unit 140 (or memory). In the present invention, the document understanding model 300 may also be called a deep document understanding model.

Next, a molecular property prediction model 500 may be a model pre-trained based on various training data to predict properties of a substance (or molecule) or design a material structure.

For example, the training data used for training the molecular property prediction model 500 may be data including unique characteristic information of the substance and property information of the substance.

Here, the unique characteristic information of the substance may include the name of the substance, the molecular structural formula, and/or chemical the formula, etc. In addition, the property information of the substance may include property (i.e., domain) values such as boiling point, melting point, refractive index, solubility, viscosity, surface tension, density, strength, and/or thermal conductivity of the substance.

The molecular property prediction model 500 may predict the properties (or property information) of the substance or design a material having user's desired properties.

Specifically, the molecular property prediction model 500 may receive the unique characteristic information of the substance and/or the property information of the substance, and output predicted data based on the input information and trained knowledge.

In an embodiment, the molecular property prediction model 500 may receive unique characteristic information of a specific substance, and output property information of the specific substance predicted based on the input information and trained knowledge.

In other embodiments, the molecular property prediction model 500 may receive the property information of the specific substance, and output the unique characteristic information of the specific substance predicted based on the input information and trained knowledge.

In another embodiment, the molecular property prediction model 500 may receive the unique characteristic information of the specific substance and the property information of the specific substance, and output optimal unique characteristic information of the substance and property information of the substance predicted based on the input information and trained knowledge.

As described above, the answer generation system 100 based on the ultra-large foundation model 200 is intended to suggest optimal research methods to researchers in the field of natural science, minimize the risk of failure in natural science research, and increase the efficiency of natural science research. More specifically, the present invention is to solve the problem of time and cost required for material research and development and to increase the efficiency of material research and development. Hereinafter, the answer generation method of the ultra-large foundation model and the overall process of the system will be described.

The answer generation system 100 may specify an analysis target based on the user input received from the user terminal 10. Here, the user input may include at least one of a document, an image, a voice, a video, and a text. For example, when the user input for the document is received, the answer generation system 100 may specify the document corresponding to the user input as an analysis target. Hereinafter, it will be described on the premise of the process of receiving the user input for the document.

As illustrated in FIG. 9, the answer generation system 100 may specify an analysis target document 30 to be analyzed.

In the present invention, there may be various methods (or methods or criteria) for specifying the analysis target document 30.

In an embodiment, the answer generation system 100 may specify the input document as the analysis target document 30 based on the fact that at least one document corresponding to the user selection among documents stored (or embedded) in the storage (or memory or storage space or database) of the user terminal 10 is input to a document upload page (or interface) provided to a service page 1000.

In another embodiment, the answer generation system 100 may receive link information (e.g., URL) of a document or link information of external storage services (e.g., Google Drive, Dropbox, etc.) storing the document from the user terminal 10. The answer generation system 100 may directly access the document through the link information of the document or download the document to specify the analysis target document 30.

However, the method of specifying an analysis target document in the present invention is not necessarily limited to the above-described embodiment. Hereinafter, for the convenience of description, it will be described on the premise that the document received through the user terminal 10, to which the service page 1000 is output, is specified as the analysis target document 30.

When the analysis target document 30 is specified, the answer generation system 100 may extract various forms of content from the analysis target document 30 using the document understanding model 300. Here, various types of content extracted from the analysis target document 30 may be understood as content that satisfies the preset content criteria.

As described above, based on the fact that the purpose of using the answer generation system 100 is the chemistry, the bio, the new materials, the new substances, and the new drug development, the preset content criteria may be determined as contents related to at least one of the chemistry, the bio, the new materials, the new substances, and the new drug development.

Accordingly, the document understanding model 300 may extract a plurality of content 31 related to the chemistry, the bio, the new materials, the new substances, and the new drug development from the analysis target document 30 based on the preset content criteria. For example, the plurality of content 31 may include at least one of text, molecular structure, formula, chart, table, and image.

Furthermore, when the plurality of content 31 is extracted from the document understanding model 300, the processor may store the plurality of extracted content 31 in the storage unit 140 (or memory).

Meanwhile, the answer generation system 100 (or processor) may analyze the relationship between the plurality of content 31 stored in the storage unit 140 (or memory). Here, the relationship indicates the semantic association between different types of content (e.g., molecular structure, text, formula, table, etc.), and may mean the relationship based on semantic, thematic, and/or structural similarity that is connected between the different types of content. This relationship may be analyzed based on the meanings of each content.

Specifically, the answer generation system 100 may analyze 32 the relationship between the plurality of content 31 based on the meanings of each of the plurality of content 31. For example, it is assumed that the first molecular structure, the second molecular structure, a first text, a second text, a first formula, a second formula, a first table, and a second table are extracted as the plurality of content 31. The answer generation system 100 may specify that the first molecular structure, the first text, the first formula, and the first table have a mutual relationship through a relationship analysis 32 of the plurality of content 31, and may specify that the second molecular structure, the second text, the second formula, and the second table have the mutual relationship.

Furthermore, the answer generation system 100 may perform grouping of the plurality of content 31. In the present invention, the grouping may be performed between the plurality of content 31 that has the mutual relationship. The answer generation system 100 may group 33 related content among the plurality of content 31 based on the relationship between the plurality of content.More specifically, the answer generation system 100 may group 33 contents related to the same molecular structure among at least one text, molecular structure, formula, chart, table, and image included in the plurality of content 31 into related content to generate grouped content 34.

In an embodiment, the answer generation system 100 may group 33 the first text, the first formula, and the first table including contents related to the first molecular structure among the plurality of content 31 into the content related to the first molecular structure to generate the grouped first content.

In other embodiments, the answer generation system 100 may group the second text, the second formula, and the second table including the contents related to the second molecular structure among the plurality of content 31 into the content related to the second molecular structure to generate the grouped second content.

Furthermore, the answer generation system 100 (or processor) may store the grouped content in the storage unit 140 (or memory) by linking the grouped content to a user account.

Through the process described above, the content 34 grouped based on a specific molecular structure may include at least one of a molecular structure image of a specific molecular structure corresponding to the grouped content 34, a name of the molecular structure, a description of the molecular structure, a properties (e.g., molecular weight, density, melting point, boiling point, flash point, surface tension, etc.) of the molecular structure, and a string according to the SMILES notation of the molecular structure.

Meanwhile, at least some of the content included in the grouped content 34 for the specific molecular structure may include content generated by the pre-trained prediction model.

Specifically, at least some of the content included in the grouped content 34 for the specific molecular structure may include the content generated by at least one of the ultra-large foundation model 200, pre-trained chemical reaction prediction model 400, and pre-trained molecular property prediction model 500.

In an embodiment, it is assumed that the plurality of content 31 extracted from the analysis target document 30 includes the molecular structure image and name of the specific molecular structure, and no description of the specific molecular structure exists. The answer generation system 100 may generate a description of the specific molecular structure using the pre-trained chemical reaction prediction model 400. In addition, the answer generation system 100 may group 33 the molecular structure image and name of the specific molecular structure extracted from the analysis target document 30 and the description of the specific molecular structure generated by the chemical reaction prediction model 400 to generate the grouped content 34.

In other embodiments, it is assumed that the plurality of content 31 extracted from the analysis target document 30 includes the molecular structure image, name, and description of the specific molecular structure, and no properties of the specific molecular structure exists. The answer generation system 100 may generate the properties of the specific molecular structure using the pre-trained molecular property prediction model 500. In addition, the answer generation system 100 may group 33 the molecular structure image, name, and description of the specific molecular structure extracted from the analysis target document 30 and the properties of the specific molecular structure generated by the molecular property prediction model 500 to generate the grouped content 34.

That is, the answer generation system 100 may generate the content not included in the analysis target document using at least one of the ultra-large foundation model 200, chemical reaction prediction model 400, and molecular property prediction model 500, and generate the grouped content 34 including the content generated by at least one of the models 200, 400, and 500.

Meanwhile, the answer generation system 100 may perform labeling 35 so that labels are assigned to at least some of the plurality of content 31. Here, at least some of the content to which the label is assigned among the plurality of content 31 may correspond to the content 34 grouped through the grouping 33.

In this case, the grouped content 34 including the related content may be assigned the same label.

Specifically, the answer generation system 100 may assign the same label to the grouped content 34 through the labeling 35. For example, the answer generation system 100 may assign a first label to the first content grouped based on the first molecular structure, and assign a second label to the second content grouped based on the second molecular structure through the labeling 35.

In this regard, as described above, when there are the plurality of grouped content 34 in the present invention, each of the plurality of grouped content 34 may be assigned different labels (e.g., the first label may be assigned to the first grouped content, and the second label may be assigned to the second grouped content).

However, as discussed above, the present invention has described the labeled target to which the label is assigned as the grouped content, but it is not necessarily limited thereto. In the present invention, in addition to the grouped content, it is also possible to assign labels to each content by labeling each content having an independent meaning.

Furthermore, the grouped content to which different labels are assigned may be stored in the storage unit 140 in connection with the user account.

Meanwhile, the answer generation system 100 may provide the grouped content 34 stored in the storage unit 140 to the user terminal 10 to which the service page 1000 is output.

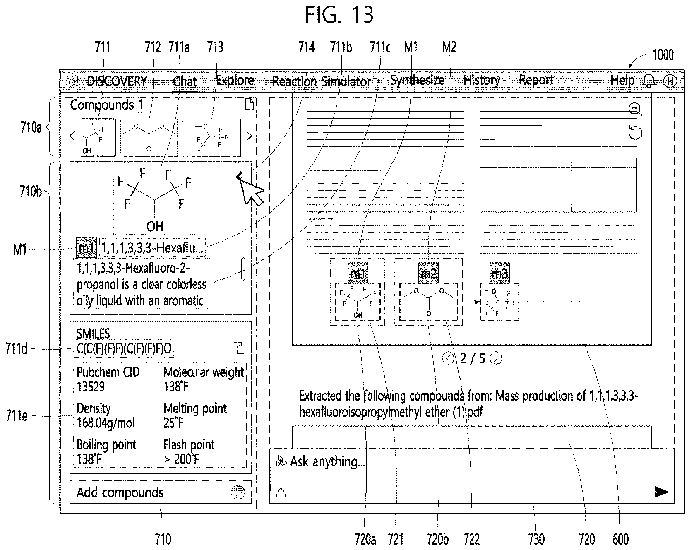

Specifically, the answer generation system 100 may provide a graphic object corresponding to each grouped content 34 assigned the label through the performance of the labeling 35 to a region of the service page 1000 on which the user query is received (e.g., see FIG. 13).

The answer generation system 100 may receive the user query corresponding to the user input through one region of the service page 1000.

Here, the user query 36 may include a label (or label information) assigned to the grouped content 34, or information (e.g., a name of the molecular structure, a chemical formula of the molecular structure, etc.) that may express the molecular structure.

Hereinafter, for the convenience of description, it is assumed that a user query 36 including label information (e.g., "Can you predict the chemical reaction of the first label M1 and the second label M2?") is received. However, the present invention does not limit the information included in the user query 36 to any one, and any information that may express the specific molecule (or compound or material) may be included in the user query.

Based on receiving the user query 36, the answer generation system 100 may process the user query 36 as the input of the ultra-large foundation model 200.

The ultra-large foundation model 200 may receive the user query 36 as the input, understand the contents included in the user query 36, and specify the specific content related to the user query 36.

More specifically, the ultra-large foundation model 200 may extract the label assigned to the grouped content 34 from the user query 36 through the analysis of the user query 36, and specify specific grouped content 37 corresponding to the extracted label. For example, based on the analysis result of the user query 36, which includes contents corresponding to the first label and the second label indicating the specific grouped content 37 in the user query 36, the ultra-large foundation model 200 may specify the first content corresponding to the first label and the second content corresponding to the second label as the specific grouped content 37 related to the user query 36.

Furthermore, the ultra-large foundation model 200 may process specific content (i.e., the molecular structure of the specific grouped content) as the input of the pre-trained chemical reaction prediction model 400.

In this case, the ultra-large foundation model 200 may change the name of the molecular structure included in the specific grouped content 37 to a language that the computer may understand.

More specifically, the ultra-large foundation model 200 may convert (or change) the name of the molecular structures included in the specific grouped content 37 into the string according to the SMILES notation which is the language that the computer may understand, and process the converted string and the information on the specific grouped content 37 as the inputs of the chemical reaction prediction model 400 that understands the chemical reaction mechanisms. For example, the ultra-large foundation model 200 may convert the names of the first molecular structure and the second molecular structure included in each of the specific grouped content 37 into the string according to the SMILES notation, and input the converted string and the information on the first molecular structure and the second molecular structure to the pre-trained chemical reaction prediction model 400.

The chemical reaction prediction model 400 may predict the intermolecular chemical reaction of the specific grouped content 37 and output the predicted results as the output data. As described above, the chemical reaction prediction model 400, which receives the string converted from the ultra-large foundation model 200 and the information on the first molecular structure and the second molecular structure, may predict the chemical reaction (or synthesis result) between the first molecule corresponding to the first molecular structure and the second molecule corresponding to the second molecular structure, and output a predicted result 38 of the chemical reaction between the first molecule and the second molecule.

In an embodiment, the predicted results of the chemical reaction may include a third molecular structure generated as the results of the chemical reaction between the first molecular structure and the second molecular structure, and may include at least one of the chemical characteristics, reaction conditions, expected yield, reaction energy, reaction path, and expected reaction time of the third molecular structure.

Meanwhile, the ultra-large foundation model 200 may generate an answer 39 to the user query 36 using the output data (predicted result 38 of a chemical reaction) of the chemical reaction prediction model 400 and the content (or specific grouped content) constituting the grouped content.

In this case, the ultra-large foundation model 200 may determine what procedure and tool to use to generate the answer 39 to the user query 36. More specifically, the ultra-large foundation model 200 may determine the answer generation procedure performed for prediction corresponding to the user query 36 and the tool used for the answer generation procedure.

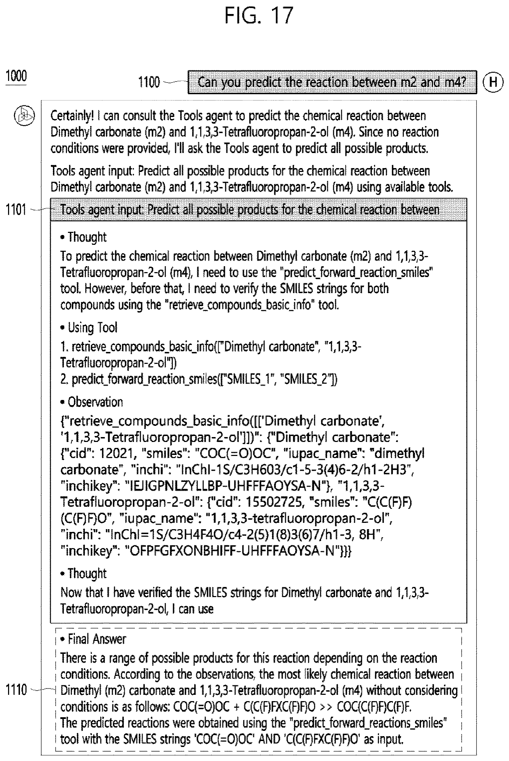

In this case, the answer generation system 100 may provide the information on the answer generation procedure determined from the ultra-large foundation model 200 and the tool used for the answer generation procedure to the service page 1000 (e.g., see FIG. 17).

The ultra-large foundation model 200 may execute an operation of generating the answer 39 to the user query 36 based on the determined answer generation procedure and tool. The ultra-large foundation model 200 may generate the answer 39 to the user query 36 using the output data of the chemical reaction prediction model 400 described above, the contents that constitutes the specific grouped content 37, and the determined answer generation procedure and tool.

In this case, the answer generation system 100 may assign a new label (e.g., the third label M3) through labeling the new molecular structure based on the fact that the answer 39 generated from the ultra-large foundation model 200 includes a new molecular structure (e.g., the third molecular structure).

Based on the generation of the answer 39 to the user query 36, the answer generation system 100 may provide the answer 39 generated from the ultra-large foundation model 200 through the user terminal 10 to which the service page 1000 is output.

Meanwhile, the answer generation system 100 may receive a new (or additional) user query through the service page 1000.

The answer generation system 100 may receive an input for a new user query 40 when the new user query 40 is input from the user terminal 10 after the answer 39 to the user query 36 is provided.

For example, the new user query 40 may be a query including contents related to at least one molecular structure to which a label is assigned, or a query including contents related to another molecule to which a label is not assigned. In the following, for the convenience of description, it will be described on the premise that the new user query 40 including the specific molecular structure (e.g., the third molecular structure 41) to which a new label (e.g., the third label M3) is assigned is received.

The answer generation system 100 may input the new user query 40 to the ultra-large foundation model 200 based on receiving the new user query 40 including the label M3 assigned to the third molecular structure 41.

The ultra-large foundation model 200 may utilize at least one prediction model to understand the new user query 40 and generate an answer to the new user query 40. In an embodiment, the ultra-large foundation model 200 may input information on the third molecular structure 41 to the molecular property prediction model 500 based on the fact that the user query 40 includes the contents "Can you predict surface tension of m3?"

The molecular property prediction model 500 may predict the properties (e.g., surface tension) for the third molecular structure 41 corresponding to the new user query 40. In addition, the molecular property prediction model 500 may output a property prediction result 42 of the third molecular structure 41.

In an embodiment, the property prediction result 42 may include at least one of surface tension, boiling point and melting point, density, solubility, viscosity, thermal characteristics, mechanical characteristics, and electrical characteristics for the third molecular structure 41.

The ultra-large foundation model 200 may determine the answer generation procedure performed for prediction corresponding to the new user query 40 and the tool used in the answer generation procedure, and may generate an answer 43 (e.g., "m3 surface tension is OO...,) to the user query 40 using the determined answer generation procedure and tool and the output data of the molecular property prediction model 500.

In addition, the answer generation system 100 may provide the answer 43 generated from the ultra-large foundation model 200 to the user terminal 10.

In this way, the present invention may generate an answer suitable for a user query and generate the answer, thereby allowing the user to receive an optimal research method and minimize the risk of research failure. In addition, by providing the prediction information for the user query using the pre-trained prediction model, the user's decision-making may be supported, and the user may receive the information that the user needs, thereby increasing the efficiency of research.

Hereinafter, based on the answer generation method of the ultra-large foundation model and the overall process of the system described above, the answer generation method of the ultra-large foundation model will be described in more detail.

First, in the present invention, a process of specifying the plurality of molecular structures to be predicted may be performed based on user input received through a service page (S1010, see FIG. 10).

The answer generation system 100 according to the present invention may be implemented in various platform forms such as applications, software, and websites. In this specification, for the convenience of description, the form in which the answer generation system 100 is implemented is not limited to any one. In the present invention, the answer generation system 100 may also be called an "answer generation platform."

As described above, the user may have a user account pre-registered in the answer generation system 100 according to the present invention. In this case, the account may be generated through a page (or screen) linked to the answer generation system 100. Alternatively, the account can also be generated in at least one other system linked to the answer generation system 100. However, in this specification, the system to which the user account is issued is not separately distinguished, and all accounts that may use various services (or functions) provided by the answer generation system 100 according to the present invention are called "accounts pre-registered in the answer generation system 100."

Meanwhile, in the present invention, receiving the "molecular structure" may be understood as receiving information that may specify a molecule. In this case, the information that may specify the molecular structure may be in various forms such as the molecular structure formula, a molecular graph, the chemical formula, the molecular structure formula based on the SMILES notation, the molecular structure image, etc.

As illustrated in FIG. 11, the answer generation platform based on the ultra-large foundation model 200 may provide the service page 1000 linked to the platform to the user terminal 10.

The answer generation system 100 may specify the plurality of molecular structures to be predicted based on the user input received through the service page 1000.

As described above, the user input may include at least one of a document, an image, a voice, a video, and text. Hereinafter, it will be described on the premise of the process of receiving the user input for the document.

The answer generation system 100 may receive the user input for at least one document from the user terminal 10 to which the service page 1000 is provided. In this case, the answer generation system 100 may receive one or more documents (i.e., a plurality of documents are also possible) from the user terminal 10, and in this specification, for the convenience of description, it will be described on the premise that one document is received.

In order to receive the user input for the document, the answer generation system 100 may provide (or display) a graphic object 601 linked to a document input function in one region of the service page 1000. For example, when the graphic object 601 is selected from the user terminal 10, the answer generation system 100 may activate (or output) a document upload page (or window) on the user terminal 10. The user may select a document through the document upload page or upload a document in a drag and drop manner. The answer generation system 100 may receive the user input for the document based on the input of the document corresponding to the user selection.

However, the user input for the document in the present invention is not necessarily limited to the above-described embodiments. As an example, a user may input link information (e.g., URL) of a document, or input link information of an external storage service (e.g., Google Drive, Dropbox, etc.) where the document is stored. In this case, the answer generation system 100 may directly access the document through the link information, or download the document, and receive the user input for the document.

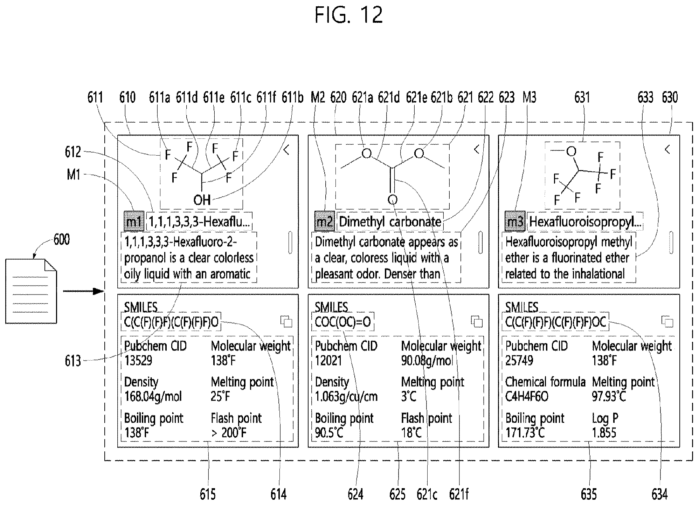

Furthermore, the answer generation system 100 may specify a document received from the user terminal 10 as an analysis target document. For example, as illustrated in FIG. 12, the answer generation system 100 may specify the document 600 corresponding to the user input as a target for analysis using the document understanding model 300.

When the analysis target document is specified, the answer generation system 100 may specify at least one molecular structure to be predicted from the analysis target document 600.

Specifically, the answer generation system 100 may extract the plurality of content (or various types of content related to the molecular structure or the information on the molecular structure) from the target document 600 for analysis using the document understanding model 300, and specify the molecular structure to be predicted based on the extracted contents.

As described above, the document understanding model 300 may extract plurality of content satisfying the preset contents criteria from at least one document. Here, the preset contents criteria may be understood as contents related to the molecular structure related to at least one of the chemistry, the bio, the new materials, the new substances, and the new drug development. Accordingly, the answer generation system 100 may extract contents related to the molecular structure related to at least one of the chemistry, the bio, the new materials, the new substances, and the new drug development from the document using the document understanding model 300.

The document understanding model 300 may understand the contents included in the analysis target document 600, and extract various types of content (or information) related to the molecular structure from the analysis target document 600 based on the result of understanding.

In an embodiment, as illustrated in FIG. 12, when the analysis target document 600 includes a first molecular structure 611 and a second molecular structure 621, the document understanding model 300 may understand the first molecular structure 611 and the second molecular structure 621, and extract the first molecular structure 611 and the second molecular structure 621 included in the analysis target document 600 based on the result of understanding. In this case, the answer generation system 100 may specify the first molecular structure 611 and the second molecular structure 621 extracted using the document understanding model 300 as the target molecular structures to be predicted.

In another embodiment, when the analysis target document 600 includes the first molecular graph for the first molecular structure 611 and the second molecular graph for the second molecular structure 621, the document understanding model 300 may understand the first molecular graph and the second molecular graph, and extract the first molecular graph and the second molecular graph included in the analysis target document 600 based on the result of understanding. In this case, the answer generation system 100 may specify the first molecular structure 611 for the first molecular graph and the second molecular structure 621 for the second molecular graph as the target molecular structures to be predicted.

In addition, the answer generation system 100 may extract at least one of the text, the formula, the chart, the table, and the image as the plurality of content from the analysis target document 600 using the document understanding model 300. For example, the document understanding model 300 may understand texts 612, 613, 614, 615, 622, 623, 624, 625, 632, 633, 634, and 635 included in the analysis target document 600, and extract texts 612, 613, 614, 615, 622, 623, 624, 625, 632, 633, 634, and 635 included in the analysis target document 600 based on the result of understanding.

Furthermore, in the present invention, a process of storing the information on the plurality of molecular structures in the memory may be performed (S 1020, see FIG. 10).

The plurality of content extracted from the document understanding model 300 may be stored in the storage unit 140 (or memory).

More specifically, the answer generation system 100 may store various types of information on the plurality of extracted molecular structures 611, 621 and 631 in the storage unit 140 (or memory). For example, the answer generation system 100 may store the plurality of extracted molecular structures 611, 621, and 631 and the extracted texts 612, 613, 614, 615, 622, 623, 624, 625, 632, 633, 634, and 635 together with the plurality of extracted molecular structures 611, 621 and 631 in the storage unit 140.

Meanwhile, the answer generation system 100 may analyze the relationship between the plurality of content based on the meanings of each of the plurality of content stored in the storage unit 140. That is, the answer generation system 100 or the processor of the system 100 may perform a series of processes described in the present invention using the content in the storage unit 140 (or memory).

Here, the relationship analysis can be understood as a process of identifying and understanding the mutual relationship (or relevance) between the plurality of content based on the meanings of each of the plurality of content. This may include a process of identifying similarity, mutual dependency, or linked meaning of the information expressed by each content and grouping the similarity, mutual dependency, or linked meaning, or deriving a specific pattern.

The answer generation system 100 may analyze the meanings of each of the plurality of content and specify (or extract) the meanings of each of the plurality of content. In this case, the analysis of the meanings of each of the plurality of content may also be performed by at least one of the ultra-large foundation model 200, the chemical reaction prediction model 400, and the molecular property prediction model 500. For example, the answer generation system 100 may analyze the meanings of each of the texts 612, 613, 614, 615, 622, 623, 624, 625, 632, 633, 634, and 635 extracted from the document understanding model 300, and based on the analysis results, determine that the meanings of each of the texts 612, 613, 614, 615, 622, 623, 624, 625, 632, 633, 634, and 635 has the name 612 of the first molecular structure, the description 613 of the first molecular structure, the SMILES notation 614 of the first molecular structure, the property 615 of the first molecular structure, the name 622 of the second molecular structure, the description 623 of the second molecular structure, the SMILES notation 624 of the second molecular structure, the property 625 of the second molecular structure, the name 632 of the third molecular structure, the description 633 of the third molecular structure, the SMILES notation 634 of the third molecular structure, and the property 635 of the third molecular structure.

Furthermore, the answer generation system 100 may specify the content having the mutual relationship based on the meanings of each of the plurality of specified content. More specifically, the answer generation system 100 may specify the relationship between the plurality of molecular structures 611, 621 and 631 extracted from the document understanding model 300, the name 612 of the first molecular structure with different meanings, the description 613 of the first molecular structure, the SMILES notation 614 of the first molecular structure, the property 615 of the first molecular structure, the name 622 of the second molecular structure, the description 623 of the second molecular structure, the SMILES notation 624 of the second molecular structure, the property 625 of the second molecular structure, the name 632 of the third molecular structure, the description 633 of the third molecular structure, the SMILES notation 634 of the third molecular structure, and the property 635 of the third molecular structure. For example, the answer generation system 100 may specify that there are the mutual relationship between the first molecular structure 611 and the name 612 of the first molecular structure, the description 613 of the first molecular structure, the SMILES notation 614 of the first molecular structure, and the property 615 of the first molecular structure, and that there is the mutual relationship between the second molecular structure 621 and the name 622 of the second molecular structure, the description 623 of the second molecular structure, the SMILES notation 624 of the second molecular structure, and the property 625 of the second molecular structure. In addition, the answer generation system 100 may specify that there is the mutual relationship between the third molecular structure 631, the name 632 of the third molecular structure, the description 633 of the third molecular structure, the SMILES notation 634 of the third molecular structure, and the property 635 of the third molecular structure.

Meanwhile, the answer generation system 100 may group contents about the same molecular structure among the plurality of content into content related to each other based on the relationship between the plurality of content. More specifically, the answer generation system 100 may group, based on the specified relationship, the name 612 of the first molecular structure, the description 613 of the first molecular structure, the SMILES notation 614 of the first molecular structure, and the property 615 of the first molecular structure, which include the contents related to the first molecular structure 611, into the content related to the first molecular structure 611. In addition, the answer generation system 100 may group, based on the specified relationship, the name 622 of the second molecular structure, the description 623 of the second molecular structure, the SMILES notation 624 of the second molecular structure, and the property 625 of the second molecular structure, and the property 625 of the second molecular structure, which include the contents related to the second molecular structure 621, into the content related to the second molecular structure 621. Furthermore, the answer generation system 100 may group, based on the specified relationship, the name 632 of the third molecular structure, the description 633 of the third molecular structure, and the SMILES notation 634 of the third molecular structure, and the property 635 of the third molecular structure, which include the contents related to the third molecular structure 631, into the contents related to the third molecular structure 631.

Through the grouping process described above, at least one grouped content may be generated (or extracted). Referring to FIG. 12, the first molecular structure 611, the name 612 of the first molecular structure, the description 613 of the first molecular structure, the SMILES notation 614 of the first molecular structure, and the property 615 of the first molecular structure may be grouped to generate the grouped first content 610. In addition, the second molecular structure 621, the name 622 of the second molecular structure, the description 623 of the second molecular structure, the SMILES notation 624 of the second molecular structure, and the property 625 of the second molecular structure may be grouped to generate the grouped second content 620. Furthermore, the third molecular structure 631, the name 632 of the third molecular structure, the description 633 of the third molecular structure, the SMILES notation 634 of the third molecular structure, and the property 635 of the third molecular structure may be grouped to generate the grouped third content 630.

The content 610, 620 and, 630 grouped based on each of the plurality of molecular structures 611, 621 and 631 may include at least one of the molecular structure images of the specific molecular structures corresponding to each of the grouped content 610, 620 and, 630, the names 612, 622, and 632 of the molecular structure, the descriptions 613, 623, and 633 of the molecular structure, the strings 614, 624, and 634 according to the SMILES notation of the molecular structure, and the properties 615, 625, and 635 of the molecular structure.

Furthermore, the grouped content 610, 620 and, 630 may be stored in the storage unit 140 in connection with the user account.

Meanwhile, in the present invention, a process of acquiring the molecular graph for the plurality of molecular structures by converting the atoms into the nodes and the bonds between the atoms into the edges based on the plurality of molecular structures stored in the memory may be performed (S1030, see FIG. 10).

Here, the molecular graph may include the nodes and edges corresponding to each of the atoms included in the molecular structure and the bonds between the atoms. That is, in the molecular graph, the atoms may be expressed as the nodes and the bonds may be expressed as the edges.

The answer generation system 100 may acquire the molecular graph for the molecular structure by converting the molecular structure. More specifically, the answer generation system 100 may acquire the molecular graph for each of the plurality of molecular structures by converting the atoms included in each of the plurality of molecular structures into the nodes and the bonds between the atoms into the edges based on the plurality of molecular structures stored in the storage unit 140.

In an embodiment, as illustrated in FIG. 12, the answer generation system 100 may convert the atoms constituting the first molecular structure 611 stored in the storage unit 140 into nodes 611a, 611b, and 611c and the bond relationship between the atoms constituting the first molecular structure 611 into edges 611d, 611e, and 611f, thereby acquiring the first molecular graph including the nodes 611a, 611b, and 611c and the edges 611d, 611e, and 611f corresponding to the first molecular structure 611.

In another embodiment, the answer generation system 100 may convert the atoms constituting the second molecular structure 621 stored in the storage unit 140 into nodes 621a, 621b, and 621c and the bond relationship between the atoms constituting the second molecular structure 621 into edges 621d, 621e, and 621f, thereby acquiring the second molecular graph including the nodes 621a, 621b, and 621c and edges 621d, 621e, and 621f corresponding to the second molecular structure 621.

However, in the present invention, the molecular graph may be acquired using the document understanding model 300, or may be acquired (or extracted) from various types of information related to the molecular structure stored in the storage unit 140.

In an embodiment, when the analysis target document 600 includes the first molecular structure 611 and the second molecular structure 621, the document understanding model 300 understands the first molecular structure 611 and the second molecular structure 621, and converts the atoms included in each of the first molecular structure 611 and the second molecular structure 621 into the nodes 611a, 611b, 611c, 621a, 621b, and 621c and the bonds between the atoms into the edges 611d, 611e, 611f, 621d, 621e, and 621f based on the result of understanding, thereby acquiring the molecular graph for each of the first molecular structure 611 and the second molecular structure 621.

In another embodiment, when the text extracted from the document understanding model 300 includes the descriptions of the first molecular structure 611 and the second molecular structure 621, the answer generation system 100 may extract the molecular graphs for the first molecular structure 611 and the second molecular structure 621, respectively, from the storage unit 140 based on the descriptions of the first molecular structure 611 and the second molecular structure 621, respectively.

That is, as described above, there may be various methods (or schemes) for acquiring the molecular graph in the present invention, and for the convenience of description, the following description will assume that the molecular graph is acquired through the conversion process by the answer generation system 100.

Meanwhile, the answer generation system 100 may perform the labeling on each of the grouped content 610, 620 and, 630 stored in the storage unit 140 so that the labels are assigned to each of the grouped content 610, 620 and, 630.

The answer generation system 100 may perform the labeling on each of the molecular structures 611, 621 and 631 so that different labels are assigned to each of the molecular structures 611, 621 and 631.

In an embodiment, the answer generation system 100 may assign the first label M1 to the first molecular structure 611 and the second label M2 to the second molecular structure 621 by labeling the first molecular structure 611 and the second molecular structure 621.

In another embodiment, the answer generation system 100 may assign the first label M1 to the first content 610 grouped based on the first molecular structure 611 and the second label M2 different from the first label M1 to the second content 620 grouped based on the second molecular structure 621 by labeling the plurality of molecular structures 611, 621 and 631. In addition, the answer generation system 100 may assign the third label M3 different from the first label M1 and the second label M2 to the third content 630 grouped based on the third molecular structure 631. In this case, the labeling for molecular structure may be understood as assigning the same label to the content grouped based on the specific molecular structure (i.e., all the content included in the grouped content has the same label).

The grouped content 610, 620 and, 630 to which different labels are assigned as described above may be stored in the storage unit 140 in connection with the user account. More specifically, the answer generation system 100 may store the information M1 on the first molecular graph acquired through the process of acquiring the molecular graph and the label assigned to the first molecular graph, and the information M2 on the second molecular graph acquired through the process of acquiring the molecular graph and the label assigned to the second molecular graph in the storage unit 140 (or memory).

Meanwhile, the answer generation system 100 may provide the grouped content 610, 620, and 630 stored in the storage unit 140 to the user terminal 10 to which the service page 1000 is output. The answer generation system 100 may provide a graphic object corresponding to each content to which the label is assigned to one region of the service page 1000 where the user query is received.

Here, the content to which the label is assigned may correspond to the content (e.g., the image of the molecular structure, the name of the molecular structure, the description of the molecular structure, the SMILES notation of the molecular structure, the properties of the molecular structure, etc.) related to the molecular structure (e.g., the first molecular structure 611, the second molecular structure 621, the third molecular structure 631) extracted from the analysis target document 600 described above.

In this regard, the answer generation system 100 may provide the plurality of graphic objects corresponding to each of the plurality of molecular structures 611, 621 and 631, to which different labels are assigned, to one region of the service page 1000 where the user query is received.

As illustrated in FIG. 13, the service page 1000 may include at least one of a first region 710 in which the information related to the plurality of molecular structures specified from the analysis target document 600 is provided, a second region 720 in which at least a portion of the document is provided or the answer to the user query is provided, and a third region 730 that receives the user query.

First, the first region 710 of the service page 1000 may include at least one grouped content to which the label is assigned. The first region 710 may display information on the specified (or extracted) molecular structure, and such information may be provided in various forms such as the molecular graph, text, or image.

Specifically, the first region 710 may include at least one graphic object 711, 712, and 713 corresponding to the extracted molecular structure (e.g., the first molecular structure 611 to which the first label M1 is assigned, the second molecular structure 621 to which the second label M2 is assigned, and the third molecular structure 631 to which the third label M3 is assigned) to which different labels are respectively assigned through the labeling, and at least one of detailed information on the extracted molecular structures 611, 621 and 631.

The first region 710 of the service page 1000 may include a first sub-region 710a including the graphic objects 711, 712, and 713 and a second sub-region 710b including the detailed information.

In this regard, when the plurality of molecular structures 611, 621 and 631 are specified from document 600, the first sub-region 710a may include the plurality of graphic objects 711, 712, and 713 corresponding to each of the plurality of molecular structures. More specifically, the first graphic object 711 among the plurality of graphic objects may include a graph of the first molecular structure 611 corresponding to the first graphic object 711 among the plurality of molecular structures 611, 621 and 631, and the second graphic object 712 may include a graph of the second molecular structure 621 corresponding to the second graphic object 712. In this case, at least some of the graphic objects may include the molecular structure image of the molecular structure.

In addition, the detailed information on the molecular structure corresponding to one graphic object selected by the user input among the plurality of graphic objects 711, 712, and 713 may be provided to the second sub-region 710b. The answer generation system 100 may provide the detailed information on the graphic object selected according to the user input based on the user input for selecting one of the plurality of graphic objects 711, 712, and 713.

In this regard, the detailed information on the plurality of molecular structures 611, 621 and 631 corresponding to each of the plurality of graphic objects 711, 712, and 713 may exist in each of the plurality of graphic objects 711, 712, and 713 included in the first sub-region 710a in a linked (or connected) manner. For example, it is assumed that the first graphic object 711 corresponding to the first molecular structure 611 is selected from the user terminal 10. The answer generation system 100 may provide detailed information 711a, 711b, 711c, 711d, and 711e on the first molecular structure 611 linked to the first graphic object 711 and information on the first label M1 assigned to the first molecular structure to the second sub-region 710b from the user terminal 10, based on the selection of the first graphic object 711 included in the first sub-region 710a.

Here, the detailed information on the molecular structure may include at least one of the molecular structure image 711a of the molecular structure, the name 711b of the molecular structure, the description 711c of the molecular structure 711c, the string 711d according to the SMILES notation, and the property 711e of the molecular structure. In this case, the molecular structure image of the molecular structure may also be provided (or displayed) in the form of the molecular graph acquired through the process of acquiring the molecular graph.

Meanwhile, the detailed information (or at least one content included in the grouped content) may be extracted from the document or acquired from at least one pre-trained prediction model. As described above, the pre-trained prediction model may include at least one of the chemical reaction prediction model 400 that predicts the chemical reaction between the molecular structures and the molecular property prediction model 500 that predicts the properties of the molecular structure.

As an example, when the plurality of content extracted from the analysis target document 600 includes the molecular structure image, the name, the description, and the string according to the SMILES notation of the first molecular structure 611, and there are no properties of the first molecular structure 611, the answer generation system 100 may predict the property of the first molecular structure 611 using the pre-trained molecular property prediction model 500. The molecular property prediction model 500 outputs the property prediction result of the first molecular structure 611 as the output data, and the answer generation system 100 may acquire the property prediction result of the first molecular structure 611 and generate the detailed information on the first molecular structure 611. In this case, the molecular structure image 711a, name 711b, description 711c and SMILES notation 711d of the first molecular structure 611 included in the first region 710 of the service page 1000 are extracted from the document 600, and the property 711e of the first molecular structure 611 may be understood to have been generated by the molecular property prediction model 500.

Next, in the second region 720 distinguished from the first region 710 of the service page 1000, the document 600 received from the user terminal 10 may be provided.

In the second region 720, highlighting objects corresponding to each of the molecular structures 611, 621 and 631 may overlap with one region of the document provided on the service page 1000 so that it is identifiable that the plurality of molecular structures 611, 621 and 631 have been extracted from the document 600. More specifically, in the second region 720, highlighting objects 721 and 722 may be overlapped and displayed in the first region (or first sub-region 720a including the first molecular structure 611 of the document provided on the service page 1000 and the second region (or second sub-region 720b) including the second molecular structure 621, respectively, so that it is identifiable that the first molecular structure 611 and the second molecular structure 621 have been extracted from the document 600.

Here, in the first region 720a including the first molecular structure 611, the first label M1 assigned to correspond to the first molecular structure 611 may be provided around the first highlighting object 721 overlapping with the first region 720a. In addition, in the second region 720b including the second molecular structure 621, the second label M2 assigned to correspond to the second molecular structure 621 may be provided around the second highlighting object 722 overlapping with the second region 720b.

Furthermore, the highlighting object 721 and 722 may be visually highlighted in a user interface (e.g., service page 1000) and thus displayed so as to be distinguished from other objects. For example, the answer generation system 100 may display the highlighting object 721 and 722 so as to be distinguished from other objects in the service page 1000 by at least one of changing colors, adding borders, and changing background colors of the highlighting object 721 and 722.

Meanwhile, when one highlighting object is selected by the user input, information on a specific molecular structure corresponding to the selected highlighting object may be provided in another region that is distinguished from one region of the service page 1000 where the highlighting object is displayed.

Specifically, the answer generation system 100 may provide detailed information on the specific molecular structure linked to the highlighted object selected according to the user input to the first region 710 of the service page 1000 based on receiving the user input for selecting one of the plurality of highlighted objects 721 and 722. For example, it is assumed that the user input for the first highlighted object 721 of the first region 720a including the first molecular structure 611 is received. The answer generation system 100 may provide detailed information 711a, 711b, 711c, 711d, and 711e on the first molecular structure 611 linked to the first highlighted object 721 to the first region 710 based on receiving the user input for selecting the first highlighted object 721.

In this case, the display of the plurality of graphic objects 711, 712, and 713 included in the first region 710 of the service page 1000 may also be changed according to the selected highlighting object. More specifically, when the first highlighting object 721 is selected, the first graphic object 711 corresponding to the first highlighting object 721 may be highlighted and displayed in the first sub-region 710a of the first region 710 so that the user can identify that the first highlighting object 721 has been selected.

That is, when the highlighting object is selected, the graphic object corresponding to the selected highlighting object may be displayed in the first region 710 with a first visual appearance so that the user may intuitively recognize the graphic object. In contrast, the graphic object corresponding to the unselected highlighting object may be displayed with a second visual appearance.

In this way, as a highlighting object is selected by the user, pieces of information on the specific molecular structure linked to the highlighting object may be provided (or displayed) in another region (first region) that is distinguished from the region (second region) where the highlighting object is displayed.

That is, in the present invention, by visually providing information through graphic objects and labels, the user may easily understand and utilize data related to a complex molecular structure. This may increase the convenience and comprehension of the user and increase the efficiency of research.

Meanwhile, the answer generation system 100 may receive an editing request for the plurality of molecular structures through the service page 1000 where the answer to the user query is provided.

The answer generation system 100 may provide a graphic object linked to a function of receiving the editing request for the plurality of molecular structures to one region of the service page 1000. For example, as illustrated in FIG. 13, the answer generation system 100 may provide a graphic object 714 linked to the function of receiving the editing request for the molecular structure (first molecular structure 611) corresponding to the molecular structure image (or graph) to the first region 710 of the service page 1000 where the molecular structure image corresponding to the selected graphic object (first graphic object 711) is displayed.

As another example, although not illustrated, the answer generation system 100 may provide the graphic object linked to the function of receiving the editing request for the plurality of molecular structures to each of the plurality of graphic objects 711, 712, and 713 provided to the first region 710. The answer generation system 100 may receive an editing request for a specific molecular structure corresponding to the selected graphic object based on receiving the user input selecting one of the graphic objects provided to each of the plurality of graphic objects 711, 712, and 713.

As another example, although not illustrated, the answer generation system 100 may provide the graphic object linked to the function of receiving the editing request for the plurality of molecular structures to each of the plurality of highlighting objects 721 and 722 provided to the second region 720. The answer generation system 100 may receive the editing request for the specific molecular structure corresponding to the selected highlighting object based on receiving the user input selecting one of the graphic objects provided to each of the plurality of graphic objects 721 and 722.

That is, the present invention does not limit the method of receiving the editing request for the molecular structure to any one. For the convenience of description, the following description will be given on the assumption that the graphic object 714 is selected.

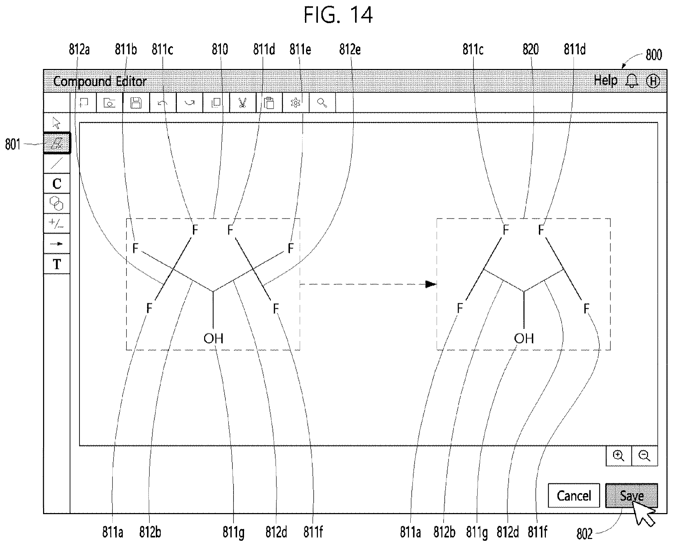

The answer generation system 100 may receive the editing request for the plurality of molecular structures (e.g., the first molecular structure) from the user terminal 10 based on the selection of the graphic object 714 included in the first region 710. In addition, as illustrated in FIG. 14, the answer generation system 100 may provide an editing interface 800 providing the editing function for the first molecular structure 611 from the user terminal 10 based on the reception of the editing request for the first molecular structure 611.

The editing interface 800 may provide, as an editable state, the molecular graph corresponding to one of the first molecular structure and the second molecular structure acquired through the molecular graph transformation among the plurality of molecular structures. For example, the answer generation system 100 may provide the molecular structure graph 810 of the first molecular structure 611 to the editing interface 800 activated on the user terminal 10 from the user terminal 10 based on receiving the editing request for the first molecular structure 611.

Here, the molecular structure image 810 may also be understood as the molecular graph that includes nodes 811a, 811b, 811c, 811d, 811e, 811f, and 811g corresponding to each of the atoms constituting the first molecular structure 611 and edges 812a, 812b, 812c, 812d, and 812e indicating the bond relationship between the atoms. The first molecular structure 611 may be configured to be edited based on the user input for at least one of nodes 811a, 811b, 811c, 811d, 811e, 811f, and 811g and edges 812a, 812b, 812c, 812d, 812e.

Specifically, the editing for the first molecular structure 611 may be a deletion or a change in position of at least one of the nodes 811a, 811b, 811c, 811d, 811e, 811f, and 811g corresponding to each of the atoms constituting the first molecular structure 611 and the edges 812a, 812b, 812c, 812d, 812e indicating the bond relationship between the atoms, or may be an addition of a new node corresponding to a new atom or an addition of a new edge generating a new bond relationship between the atoms.

For example, the answer generation system 100 may activate the node and edge deletion mode based on a selection of a graphic object 801 linked to the node and edge deletion function included in one region of the editing interface 800. The answer generation system 100 may edit the first molecular structure 611 such that the specific nodes 811b and 811e at positions corresponding to the user input are deleted based on user input received for selecting the specific nodes 811b and 811e from among the plurality of nodes 811a, 811b, 811c, 811d, 811e, 811f, and 811g included in the image 810 of the first molecular structure.

In this case, when the editing is performed on the first molecular structure 611 as the editing target based on the user input, the molecular structure image 820 corresponding to the edited molecular structure, which is different from the molecular structure image 810 of the first molecular structure 611 before the editing, may be continuously displayed on the editing interface 800. For example, based on the deletion of the specific nodes 811b and 811e corresponding to the user selection, the molecular structure image 820 in which the specific nodes 811b and 811e are deleted may be displayed on the editing interface 800.

Furthermore, the answer generation system 100 may store the molecular structure (or molecular structure image or molecular graph 820) for which the editing has been performed (or edited) in a predetermined storage (e.g., storage unit 140 or memory) by linking the molecular structure to the user account. For example, the answer generation system 100 may generate the molecular structure 820 in which the first molecular structure 611 is edited based on a selection of a graphic object 802 linked to an editing save (or completion) function included in the editing interface 800 from the user terminal 10, and store the edited molecular structure 820 in the storage unit 140 (or memory) by linking the edited molecular structure 820 to the user account.

In this way, the present invention may provide a user environment in which a user may design a desired molecule through the editing interface.

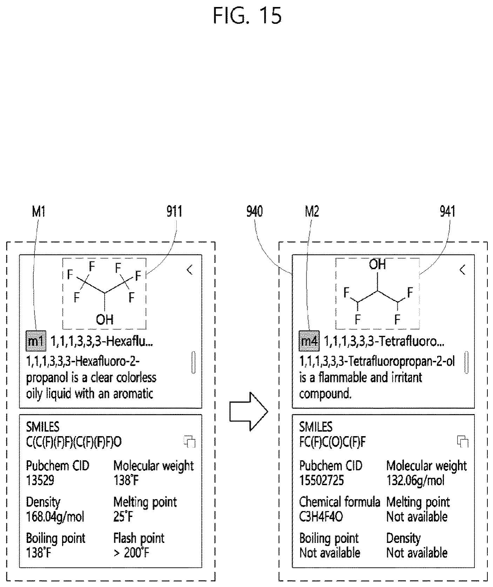

Meanwhile, a new label specifying the edited molecular structure may be assigned to the edited molecular structure. The answer generation system 100 may perform labeling on the edited molecular structure stored in the storage unit 140 so that a new label for specifying the edited molecular structure may be assigned. For example, as illustrated in FIG. 15, the answer generation system 100 may perform labeling on an edited molecular structure 941 so that the edited molecular structure 941 is assigned a fourth label M4, which is different from the first label M1 assigned to the molecular structure (e.g., the first molecular structure 911) before the editing.

The answer generation system 100 may generate a fourth graphic object corresponding to the edited molecular structure 941 and provide the generated fourth graphic object to one region of the service page 1000.

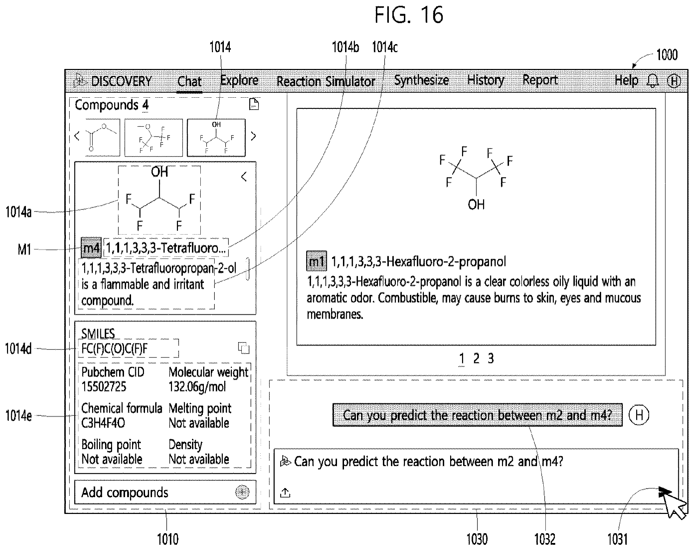

Specifically, as illustrated in FIG. 16, a graphic object (or a fourth graphic object, 1014) corresponding to the edited molecular structure 941 may be provided to the first region (or a first sub-region, 1010) of the service page 1000. Here, the graphic object 1014 corresponding to the edited molecular structure 941 may include the molecular structure image of the edited molecular structure 941.

In addition, the first region (or the first sub-region 1010) of the service page 1000 may include a molecular structure image 1014a of the edited molecular structure 941. In addition, the fourth label M4 assigned to the edited molecular structure 941 may also be provided to the surrounding region of the molecular structure image 1014a.

Furthermore, the graphic object 1014 corresponding to the edited molecular structure 941 may further include detailed information on the edited molecular structure 941. For example, the graphic object 1014 may include at least one of a name 1014b of the edited molecular structure 941, a description 1014c of the edited molecular structure 941, a SMILES notation 1014d of the edited molecular structure 941, and a property 1014e of the edited molecular structure 941.

In this case, at least one of the molecular structure image 1014a of the edited molecular structure 941, the name 1014b of the edited molecular structure 941, the description 1014c of the edited molecular structure 941, the SMILES notation 1014d of the edited molecular structure 941, and the property 1014e of the edited molecular structure 941 may be generated by at least one of the pre-trained chemical reaction prediction model 400 and molecular property prediction model 500. For the convenience of description, the edited molecular structure 941 is named "a fourth molecular structure 941", and the detailed information on the edited molecular structure 941 is named "the grouped fourth content 940" (see FIG. 15).

Meanwhile, in the present invention, a process of receiving the user query for the chemical reaction prediction related to the plurality of molecular structures may be performed through the service page (S1040, see FIG. 10).

The answer generation system 100 may receive a user query including at least one of the labels assigned by the labeling through the service page 1000. More specifically, the answer generation system 100 may receive the user query for the chemical reaction prediction related to the plurality of molecular structures to which the labels are assigned.

As illustrated in FIG. 16, a third region 1030 of the service page 1000 may be configured to receive the user query. The third region 1030 may include a graphic object 1031 linked to the function of receiving the user query.

The answer generation system 100 may receive a user query including a label assigned to a specific molecular structure through the third region 1030 of the service page 1000.

Specifically, the answer generation system 100 may receive the user query including the plurality of molecular structures to which different labels are assigned. For example, the answer generation system 100 may receive a user query 1032 (e.g., "Can you predict the reaction between m2 and m4?") including the second label M2 assigned to the second molecular structure 621 and the fourth label M4 assigned to the edited molecular structure 941 from the user terminal 10, based on the selection of the graphic object 1031 included in the third region 1030. In this case, the user query 1032 may be a query related to predicting the chemical reaction between the second molecular structure 621 to which the second label M2 is assigned and the fourth molecular structure 941 to which the fourth label M4 is assigned.

In this way, the user may input a query more intuitively and simply by utilizing the label assigned to the specific molecular structure without having to input complex information on the specific molecular structure.

When the user query is received, the answer generation system 100 may specify the specific content (or molecular structure) related to the user query among the plurality of content.

The answer generation system 100 may input the user query 1032 to the ultra-large foundation model 200 based on receiving the user query 1032.

The ultra-large foundation model 200 may receive the user query 1032 as the input, understand the contents included in the user query 1032, and specify the specific content (or molecular structure) related to the user query 1032.

The ultra-large foundation model 200 may analyze the user query 1032 and extract the label indicating the grouped content from user query 1032. For example, the ultra-large foundation model 200 may extract the second label M2 and the fourth label M4 as the result of analyzing the user query 1032, based on the fact that the text corresponding to the second label M2 indicating the grouped second content 620 and the fourth label M4 indicating the grouped fourth content 940 are included in the user query 1032.

Furthermore, the ultra-large foundation model 200 may specify the specific grouped content corresponding to the extracted label. For example, the ultra-large foundation model 200 may specify the grouped second content 620 corresponding to the extracted second label M2 and the grouped fourth content 940 corresponding to the extracted fourth label M4 as the specific grouped content.

Here, the fact that the grouped second content 620 and the grouped fourth content 940 are specified may also be understood to mean that the second molecular structure 621 corresponding to the grouped second content 620 and the fourth molecular structure 941 corresponding to the grouped fourth content 940 are specified.

In this way, in the present invention, the time required to identify the specific content corresponding to the user query may be shortened through the label assigned to the extracted molecular structure.

When the content related to the user query is specified, in the present invention, a process of processing the molecular graph for the plurality of molecular structures as the input to the chemical reaction prediction model may be performed so that a chemical reaction corresponding to the user query is predicted (S1050, see FIG. 10).

The ultra-large foundation model 200 may process the molecular structure of the specific grouped content as the input to the pre-trained prediction model. More specifically, the ultra-large foundation model 200 may process the grouped second content 620 to which the second label M2 is assigned and the grouped fourth content 940 to which the fourth label M4 is assigned as the inputs to the pre-trained prediction model.

Here, processing the specific grouped content as the input to the pre-trained prediction model may also be understood to mean processing the molecular structure corresponding to the grouped content as the input.

In this regard, the answer generation system 100 may process the second molecular structure 621 corresponding to the specific grouped second content 620 and the fourth molecular structure 941 corresponding to the specific grouped fourth content 940 as the inputs to the pre-trained prediction model.

More specifically, the answer generation system 100 may process the second molecular graph for the second molecular structure 621 and the fourth molecular graph for the fourth molecular structure 941 as the inputs to the pre-trained prediction model. The second molecular graph for the second molecular structure 621 may be confirmed with reference to FIG. 12, and the fourth molecular graph for the fourth molecular structure 941 may be confirmed with reference to FIG. 15. In this case, it may be determined based on the user query 1032 which of the multiple pre-trained prediction models to process the molecular graph (or specified content) as input. For example, the ultra-large foundation model 200 may understand the content included in the user query 1032, and may determine that the user query 1032 is related to the chemical reaction prediction for the plurality of molecular structures based on the fact that the user query 1032 includes the content "Can you predict the chemical reaction between m2 and m4?". Based on the determination result, the ultra-large foundation model 200 may determine the prediction model to which the plurality of molecular graphs (e.g., the second molecular graph and the fourth molecular graph) is input as the chemical reaction prediction model 400.

Furthermore, the ultra-large foundation model 200 may process the second molecular graph and the fourth molecular graph as the inputs to the chemical reaction prediction model 400 which understands the chemical reaction mechanisms.

When the molecular graph for the molecular structure is input to the chemical reaction prediction model, a process of performing the chemical reaction prediction for the plurality of molecular structures in the chemical reaction prediction model may be performed (S1060, see FIG. 10).

Furthermore, in the present invention, a process of acquiring the product of the chemical reaction prediction for the plurality of molecular structures from the chemical reaction prediction model may be performed (S1070, see FIG. 10).

Since the chemical reaction prediction process of the chemical reaction prediction model 400 has been described in more detail above, it will be briefly described below to avoid the duplication of contents.

When the second molecular graph and the fourth molecular graph are input, the chemical reaction prediction model 400 may acquire the embedding vector corresponding to the second molecular graph and the fourth molecular graph from the embedding layer 411 of the encoder 410 using the information related to the plurality of molecular structures (the second molecular structure 621 and the fourth molecular structure 941). The chemical reaction prediction model 400 may perform an attention operation related to the interaction between the atoms of the plurality of molecular structures 621 and 941 using the multi-head self-attention layer 413, and update the embedding vector based on the operation. Then, the chemical reaction prediction model 400 may perform the bond and atom predictions predicted as the chemical reaction of the plurality of molecular structures 621 and 641 using the updated embedding vector through the update process, and output the final chemical reaction product predicted from the chemical reaction of plurality of molecular structures 621 and 641 using the result of the bond prediction and the result of the atom prediction.

As described above, the updated embedding vector may be understood as being updated by adding different biases according to the bond type between the nodes constituting the second molecular graph and the fourth molecular graph to the attention score calculated in each of the plurality of heads of the multi-head self-attention layer 413. Furthermore, the bond prediction may be performed by performing the dot-product using the updated embedding vector, the atom prediction may predict the atom properties of the atoms corresponding to the updated embedding vector using the atom property probability distribution of each of the atoms corresponding to the updated embedding vector, and the atom properties may include the charge state of the atoms that may change during the chemical reaction process of the plurality of molecular structures.

That is, the chemical reaction prediction model 400 may output the final chemical reaction product predicted from the chemical reaction between the second molecular structure 621 and the fourth molecular structure 941 through the process described above. Here, the product of the chemical reaction prediction acquired from the chemical reaction prediction model 400 may correspond to the final product stabilized through the diffusion feedback process.

In an embodiment, the output data (or the product of the chemical reaction prediction) of the chemical reaction prediction model 400 may include the specific molecular structure generated as the predicted results of the chemical reaction between the plurality of molecular structures and at least one piece of information (or content) related to the specific molecular structure. For example, the output data may include at least one of a fifth molecular structure generated as the predicted results of the chemical reaction between the second molecular structure 621 and the fourth molecular structure 941, the molecular structure image of the fifth molecular structure, a name of the fifth molecular structure, a description of the fifth molecular structure, and a SMILES notation of the fifth molecular structure.

Meanwhile, in the present invention, a process of generating the answer to the user query using the product of the chemical reaction prediction may be performed (S1080, see FIG. 10).

The ultra-large foundation model 200 may determine the answer generation procedure performed for prediction corresponding to the user query 1032 and the tool used for the answer generation procedure. For example, as illustrated in FIG. 17, the ultra-large foundation model 200 may determine what procedure and tool to use to generate the answer to the user query 1032.

The answer generation system 100 may provide the information on the answer generation procedure and tool determined from the ultra-large foundation model 200 to the service page 1000. For example, the answer generation system 100 may provide information 1101 on the answer generation procedure and tool determined from the ultra-large foundation model 200 through the service page 1000 to perform a prediction corresponding to a user query 1100 (e.g., "Can you predict the reaction between m2 and m4?").

Furthermore, the ultra-large foundation model 200 may generate the answer to the user query 1032 using the plurality of molecular structures (e.g., the second molecular structure 621 and the fourth molecular structure 941) corresponding to the specific labels (the second label M2 and the fourth label M4) included in the user query 1032. More specifically, the ultra-large foundation model 200 may generate an answer 1110 to the user query 1100 using the prediction products of the chemical reaction of the chemical reaction prediction model 400 and the contents (e.g., contents related to the second molecular structure 621 and the fourth molecular structure 941) constituting the grouped content and the information 1101 on the determined answer generation procedure and tool.

Meanwhile, the answer generation system 100 may predict the properties of the specific molecular structure using the pre-trained molecular property prediction model 500, and also provide the information on the properties of the specific molecular structure predicted from the molecular property prediction model 500 as the answer 1110 to the user query 1100.

As described above, the molecular property prediction model 500 may be a model built for material structure design. The molecular property prediction model 500 may be configured to predict the properties from the molecular structure or design a molecule having the user's desired characteristics (or new characteristics).

Specifically, the answer generation system 100 may process the fifth molecular structure as the input to the molecular property prediction model 500. The molecular property prediction model 500 may receive the fifth molecular structure as an input and output the property prediction result of the fifth molecular structure as output data. The answer generation system 100 may acquire the property prediction result of the fifth molecular structure output from the molecular property prediction model 500.

Furthermore, the answer generation system 100 may input the property prediction result of the fifth molecular structure to the ultra-large foundation model 200. The ultra-large foundation model 200 may generate the answer 1110 to the user query 1100 using the property prediction result of the fifth molecular structure. In this case, the answer generation system 100 may provide the information on the properties of the fifth molecular structure predicted from the molecular property prediction model 500 as the answer 1110 to the user query 1100.

Meanwhile, when the answer 1110 to the user query 1100 includes the specific molecular structure (or a new molecular structure) generated through the pre-trained prediction model, a label may be assigned to the specific molecular structure.

The answer generation system 100 may perform labeling on the specific molecular structure so that a new label for specifying the specific molecular structure is assigned. For example, the answer generation system 100 may perform the labeling on the fifth molecular structure so that the fifth label M5 is assigned to the fifth molecular structure generated through the chemical reaction prediction model 400.

Here, the specific molecular structure (the fifth molecular structure) and the label assigned to the specific molecular structure (the fifth label M5) may be stored in the storage unit 140 by being linked to the user account, together with the extracted molecular structure and the label assigned to the extracted molecular structure.

In addition, the answer generation system 100 may generate a specific graphic object corresponding to the specific molecular structure based on the fact that the specific molecular structure is generated from the pre-trained prediction model. For example, the answer generation system 100 may generate the fifth graphic object corresponding to the fifth molecular structure using the fifth molecular structure stored in the storage unit 140.

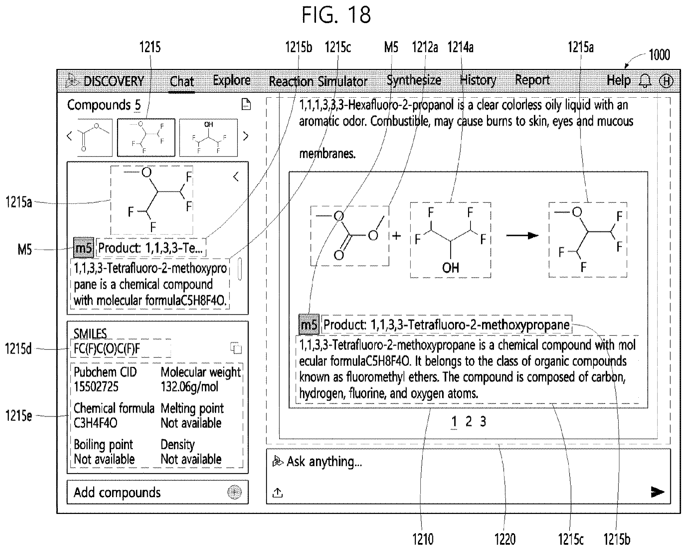

Furthermore, the answer generation system 100 may perform an update on the service page 1000 so that the specific graphic object corresponding to the specific molecular structure is included in the region of the service page 1000. More specifically, as illustrated in FIG. 18, the answer generation system 100 may perform an update on a first region 1210 so that a graphic object (or a fifth graphic object 1215) corresponding to the fifth molecular structure is included in the first region 1210 of the service page 1000.

Based on the update, the detailed information on the molecular structure corresponding to the specific graphic object may be provided to the first region 1210 of the service page 1000 together with the specific graphic object. For example, the first region 1210 may include a molecular structure image 1215a of the fifth molecular structure corresponding to the fifth graphic object 1215, a name 1215b of the fifth molecular structure, a description 1215c of the fifth molecular structure, a SMILES notation 1215d of the fifth molecular structure, and a property 1215e of the fifth molecular structure.

Meanwhile, the answer generation system 100 may provide an answer 1221 (e.g., "The product generated through the chemical reaction between m2 and m4 is m5...,") to the user query 1100 generated from the ultra-large foundation model 200 to one region (or the second region, 1220) of the service page 1000.

The answer 1221 provided to the second region 1220 of the service page 1000 may include contents indicating that a new specific molecular structure (e.g., the fifth molecular structure) is generated as the predicted results of the chemical reaction between the plurality of molecular structures (e.g., the second molecular structure 621 and the fourth molecular structure 941).

Specifically, the answer 1221 may include a molecular structure image 1212a of the second molecular structure 621 and a molecular structure image 1214a of the fourth molecular structure 941, and may include a molecular structure image 1215a of the fifth molecular structure generated as the results of the chemical reaction between the second molecular structure 621 and the fourth molecular structure 941.

In addition, graphic objects (e.g., a plus sign, an arrow, etc.) indicating the relationship between the molecular structures corresponding to each image may also be displayed between the image 1212a of the second molecular structure, the image 1214a of the fourth molecular structure, and the image 1215a of the fifth molecular structure that are included in the answer 1221.

Furthermore, the answer 1221 may include at least one of the detailed information (e.g., a name 1215b of the fifth molecular structure, a description 1215c of the fifth molecular structure, etc.) on the fifth molecular structure generated through the chemical reaction prediction model 400.

Furthermore, the information on the properties of the fifth molecular structure predicted from the property prediction model 500 may be also provided to the answer 1221, and the information on the fifth label M5 assigned to the fifth molecular structure may also be displayed to the surrounding region of the molecular structure image 1215a of the fifth molecular structure.

Meanwhile, the answer generation method of the ultra-large foundation model described above was described on the premise that the document was received, but the answer generation method of the ultra-large foundation model will be described below in more detail on the premise that the text was received.

First, the answer generation system 100 may receive the user query in the form of the text from the user terminal 10 to which the service page 1000 is provided.

Specifically, the answer generation system 100 may receive the user query including the information on at least one molecular structure through the region of the service page 1000. Here, the information on the molecular structure included in the user query may exist variously. For example, the information on the molecular structure may include the name of the specific molecular structure, the description of the specific molecular structure, the SMILES notation of the specific molecular structure, the formula of the specific molecular structure, etc. However, the information on the molecular structure described above is only an example, and the information on the molecular structure included in the user query in the present invention is not necessarily limited thereto, and it is obvious that it may further include various types of information related to the molecular structure.

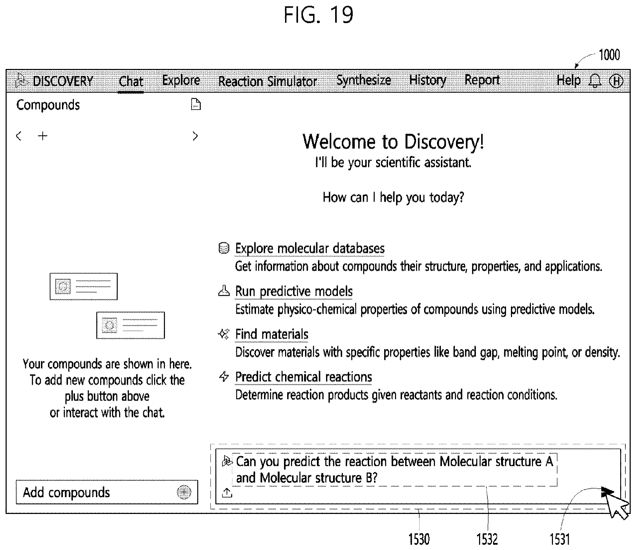

For example, as illustrated in FIG. 19, the answer generation system 100 may receive a user query 1532 (e.g., "Can you predict the reaction between Molecular structure A and Molecular structure B?") including a name (e.g., "Molecular structure A," "Molecular structure B") of a specific molecular structure based on a selection of a graphic object 1531 included in a third region 1530 from the user terminal 10.

The answer generation system 100 may input the user query 1532 to the ultra-large foundation model 200 based on receiving the user query 1532.

The ultra-large foundation model 200 may specify the molecular structure to be predicted based on the name of the specific molecular structure included in the user query 1532. For example, the ultra-large foundation model 200 may specify the first molecular structure and the second molecular structure corresponding to each of the specific molecular structures from the name (e.g., "Molecular structure A", "Molecular structure B") of the specific molecular structure included in the user query 1532.

When the molecular structure is specified, the answer generation system 100 may extract the information on the specified molecular structure. In this case, the information on the specified molecular structure may also be extracted by the ultra-large foundation model 200.

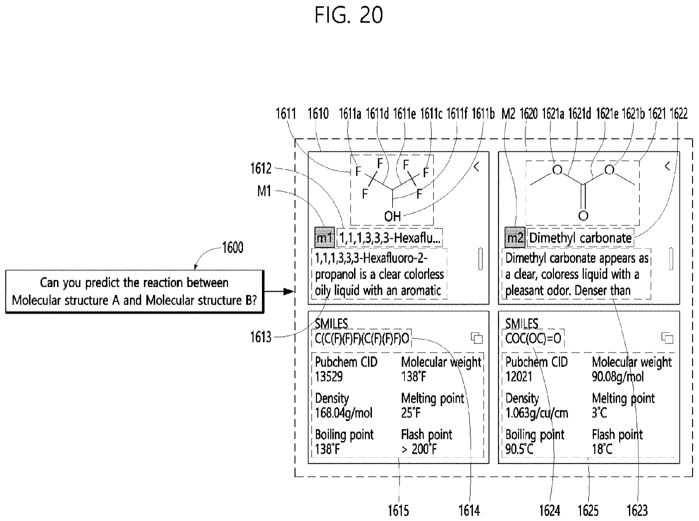

In an embodiment, as illustrated in FIG. 20, the answer generation system 100 may extract at least one of a molecular structure image of a first molecular structure 1611, a name 1612 of the first molecular structure, a description 1613 of the first molecular structure, a SMILES notation 1614 of the first molecular structure, and a property 1614 of the first molecular structure that are related to the first molecular structure 1611 specified from a user query 1600.

In another embodiment, the answer generation system 100 may extract at least one of a molecular structure image of a second molecular structure 1621, a name 1622 of the second molecular structure, a description 1623 of the second molecular structure, a SMILES notation 1624 of the second molecular structure, and a property 1625 of the first molecular structure that are related to the second molecular structure 1621 specified from the user query 1600.

Here, various types of information (e.g., detailed information, plurality of content, etc.) related to the specific molecular structure may be i) extracted from contents (or information or data) related to various molecular structures stored in the storage unit 140, or ii) generated by at least one of the chemical reaction prediction model 400 and the molecular property prediction model 500.

In an embodiment, the information related to various molecular structures stored in the storage unit 140 may include contents related to molecular structures related to at least one of the chemistry, the bio, the new materials, the new substances, and the new drug development extracted from each of the plurality of documents using the document understanding model 300. The ultra-large foundation model 200 may extract, as content, at least one contents related to the specified molecular structure from among the contents related to the molecular structure stored in the storage unit 140.

In another embodiment, when the specific molecular structure is specified from the user query, the answer generation system 100 may generate at least one content related to the specified molecular structure using at least one of the pre-trained chemical reaction prediction model 400 and molecular property prediction model 500.

Meanwhile, the answer generation system 100 may group contents about the same molecular structure among the plurality of content into content related to each other based on the relationship between the plurality of content. More specifically, the answer generation system 100 may group, into the content related to the first molecular structure, a molecular structure image of the first molecular structure 1611, a name 1612 of the first molecular structure, a description 1613 of the first molecular structure, a SMILES notation 1614 of the first molecular structure, and a property 1615 of the first molecular structure, which include contents related to the first molecular structure 1611. In addition, the answer generation system 100 may group, into the content related to the second molecular structure 1621, a molecular structure image of the second molecular structure 1621, a name 1622 of the second molecular structure, a description 1623 of the second molecular structure, a SMILES notation 1624 of the second molecular structure, and a property 1625 of the first molecular structure, which include contents related to the second molecular structure 1621.

Through the grouping process described above, the content grouped based on the molecular structure may be generated. For example, the first molecular structure 1611, the molecular structure image of the first molecular structure 1611, the name 1612 of the first molecular structure, the description 1613 of the first molecular structure, the SMILES notation 1614 of the first molecular structure, and the property 1615 of the first molecular structure may be grouped to generate the grouped first content 1610. In addition, the second molecular structure 1621, the molecular structure image of the second molecular structure 1621, the name 1622 of the second molecular structure, the description 1623 of the second molecular structure, the SMILES notation 1624 of the second molecular structure, and the property 1625 of the first molecular structure may be grouped to generate the grouped second content 1620.

Furthermore, the grouped content 1610 and 1620 may be stored in the storage unit 140 by being linked to the user account.

Meanwhile, the answer generation system 100 may acquire the molecular graph for the plurality of molecular structures by converting the atoms into the nodes and the bonds between the atoms into the edges based on the plurality of molecular structures stored in the storage unit 140 (or memory).

As described above, the answer generation system 100 may acquire the molecular graph for the molecular structure by converting the atoms included in the molecular structure into the nodes and the bonds between the atoms into the edges based on the molecular structure.

In an embodiment, as illustrated in FIG. 16, the answer generation system 100 may convert the atoms constituting the first molecular structure 1611 stored in the storage unit 140 into nodes 1611a, 1611b, and 1611c and the bond relationship between the atoms constituting the first molecular structure 1611 into the edges 1611d, 1611e, and 1611f, thereby acquiring the first molecular graph including the nodes 1611a, 1611b, and 1611c and the edges 1611d, 1611e, and 1611f corresponding to the first molecular structure 1611.

In another embodiment, the answer generation system 100 may convert the atoms constituting the second molecular structure 1621 stored in the storage unit 140 into nodes 1621a, 1621b, and 1621c and the bond relationship between the atoms constituting the second molecular structure 1621 into edges 1621d, 621e, and 621f, thereby acquiring the second molecular graph including the nodes 1621a, 1621b, and 1621c and the edges 1621d, 1621e, and 1621f corresponding to the second molecular structure 1621.

Furthermore, the answer generation system 100 may store the information on the plurality of specified molecular structures in the storage unit 140. More specifically, the answer generation system 100 may store various types of information (e.g., the first molecular graph and the second molecular graph) on the first molecular structure 1611 and the second molecular structure 1621 in the storage unit 140 (or memory).

Meanwhile, the answer generation system 100 may perform the labeling on the plurality of molecular structures 1611 and 1621 so that different labels are assigned to each of the plurality of molecular structures 1611 and 1621. For example, the answer generation system 100 may assign the first label M1 to the first molecular structure 1611 and the second label M2 to the second molecular structure 1621 by performing the labeling on the first molecular structure 1611 (or the first molecular structure graph) and the second molecular structure 1621 (or the second molecular structure graph).

Further, the answer generation system 100 may store the information M1 on the first molecular graph acquired through the process of acquiring the molecular graph and the label assigned to the first molecular graph, and the information M2 on the second molecular graph acquired through the process of acquiring the molecular graph and the label assigned to the second molecular graph in the storage unit 140 (or memory).

Furthermore, the ultra-large foundation model 200 understands the contents included in the user query 1600, and may process the first molecular structure 1611 to which the first label M1 is assigned and the second molecular structure 1621 to which the second label M2 is assigned as the inputs to the chemical reaction prediction model 400 based on the fact that the user query 1032 includes the contents "Can you predict the reaction between molecular structure A and molecular structure B?"

Meanwhile, since the chemical reaction prediction process of the chemical reaction prediction model 400 has been described in more detail above, it will be briefly described below to avoid the duplication of contents.

When the first molecular graph and the second molecular graph are input, the chemical reaction prediction model 400 may acquire the embedding vector corresponding to the first molecular graph and the second molecular graph from the embedding layer 411 of the encoder 410 using the information related to the plurality of molecular structures (the first molecular structure 1611 and the second molecular structure 1621). The chemical reaction prediction model 400 may perform an attention operation related to the interaction between the atoms of the plurality of molecular structures 1611 and 1621 using the multi-head self-attention layer 413, and update the embedding vector based on the operation. Then, the chemical reaction prediction model 400 may perform the bond and atom predictions predicted as the chemical reaction of the plurality of molecular structures 1611 and 1621 using the updated embedding vector through the update process, and output the final chemical reaction product predicted from the chemical reaction of plurality of molecular structures 1611 and 1621 using the result of the bond prediction and the result of the atom prediction.

In addition, the ultra-large foundation model 200 may acquire the predicted product of the chemical reaction for the plurality of molecular structures 1611 and 1621 from the chemical reaction prediction model 400, and may generate an answer 1721 (e.g., "The product generated through the chemical reaction between m1 and m2 is m3...") to the user query 1600 using the acquired results.

Meanwhile, the answer generation system 100 may generate the third content (or detailed information) grouped based on the specific molecular structure based on the fact that the answer 1721 to the user query 1600 includes the specific molecular structure (or a new molecular structure). For the convenience of description, the specific molecular structure will be described below by naming "the third molecular structure."

In this case, at least some of the information included in the grouped third content may be generated by at least one of the chemical reaction prediction model 400 and the molecular property prediction model 500. As another example, at least some of the information included in the grouped third content may be generated using the information related to various molecular structures stored in the storage unit 140.

In addition, the answer generation system 100 may perform the labeling on the grouped third content based on the third molecular structure so that a new label for specifying the third molecular structure is assigned. For example, the answer generation system 100 may perform the labeling on the third molecular structure so that the third label M3 is assigned to the third molecular structure generated through the chemical reaction prediction model 400.

Here, the third molecular structure and the third label assigned to the third molecular structure may be stored in the storage unit 140 by being linked to the user account, together with the extracted molecular structure (e.g., the first molecular structure and the second molecular structure) and the label (e.g., the first label and the second label) assigned to the extracted molecular structure.

In addition, the answer generation system 100 may generate the specific graphic object corresponding to the third molecular structure. For example, the answer generation system 100 may generate a third graphic object corresponding to the third molecular structure generated through the chemical reaction prediction model 400.

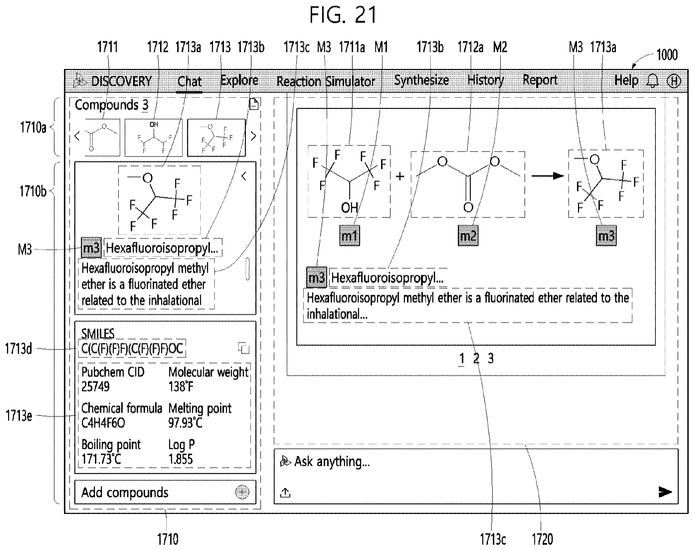

Meanwhile, as illustrated in FIG. 21, the answer generation system 100 may provide the graphic objects corresponding to each content to which the labels are assigned, together with the answer 1721 generated from then ultra-large foundation model 200, to the service page 1000. Here, the content to which the label is assigned may include the grouped first content 1610 and second content 1620, and the third content grouped based on the third molecular structure included in the answer 1721 to the user query 1600.

First, the first region 1710 of the service page 1000 may include at least one grouped content to which the label is assigned.

Specifically, the first region 1710 may include graphic objects 1711, 1712, and 1713 corresponding to each of the plurality of molecular structures (e.g., the first molecular structure to which the first label M1 is assigned, the second molecular structure to which the second label M2 is assigned, and the third molecular structure to which the third label M3 is assigned) to which different labels are assigned through the labeling, and at least one of the detailed information on the plurality of molecular structures.

The first region 710 of the service page 1000 may include a first sub-region 1710a including the graphic objects 1711, 1712, and 1713 and a second sub-region 1710b including the detailed information.

The first sub-region 1710a may include the plurality of graphic objects 1711, 1712, and 1713 corresponding to each of the plurality of molecular structures. More specifically, the first graphic object 1711 among the plurality of graphic objects may include the image of the first molecular structure corresponding to the first graphic object 1711 among the plurality of molecular structures, and the third graphic object 1713 may include the image of the third molecular structure corresponding to the third graphic object 1713.

In addition, the detailed information on the molecular structure corresponding to one graphic object selected by the user input among the plurality of graphic objects 1711, 1712, and 1713 may be provided to the second sub-region 1710b. The answer generation system 100 may provide the detailed information on the selected graphic object selected to the user input based on receiving that the user input for selecting one of the plurality of graphic objects 1711, 1712, and 1713.

In this regard, the detailed information of the molecular structures corresponding to each of the plurality of graphic objects 1711, 1712, and 1713 may exist in the each of the plurality of graphic objects 1711, 1712, and 1713 included in the first sub-area 1710a by being linked (or associated). For example, it is assumed that the third graphic object 1713 corresponding to the third molecular structure is selected from the user terminal 10. The answer generation system 100 may provide detailed information 1711a, 1711b, 1711c, 1711d, and 1711e on the third molecular structure linked to the third graphic object 1713 to the second sub-region 1710b from the user terminal 10, based on the selection of the third graphic object 1713 included in the first sub-region 1710a. Furthermore, the information on the third label M3 assigned to the third molecular structure may also be displayed in the second sub-region 1710b.

As another example, when a new molecular structure (e.g., the third molecular structure) is included in the answer to the user query, the answer generation system 100 may automatically select the graphic object corresponding to the new molecular structure included in the first sub-region 1710a to provide the detailed information on the new molecular structure to the second sub-region 1710b.

Next, an answer 1721 to the user query 1600 may be provided to the second region 1720 that is distinguished from the first region 1710 of the service page 1000.

The answer 1721 provided to the second region 1720 of the service page 1000 may include contents indicating that a new specific molecular structure (e.g., the fifth molecular structure) is generated as the predicted results of the chemical reaction between the plurality of molecular structures (e.g., the first molecular structure and the second molecular structure).

Specifically, the answer 1721 may include the molecular structure image 1711a of the second molecular structure and a molecular structure image 1712a of the second molecular structure, and may include a molecular structure image 1713a of the third molecular structure generated as the results of the chemical reaction between the first molecular structure and the second molecular structure.

In addition, the graphic objects (e.g., a plus sign, an arrow, etc.) indicating the relationship between the molecular structures corresponding to each image may also be displayed between the image 1711a of the first molecular structure, the molecular structure image 1712a of the second molecular structure and the molecular structure image 1713a of the third molecular structure that are included in the answer 1721.

Furthermore, the answer 1721 may include at least one of the detailed information (e.g., a name 1713b of the third molecular structure, a description 1713c of the third molecular structure, etc.) on the third molecular structure generated through the chemical reaction prediction model 400.

Furthermore, the information on the property of the third molecular structure predicted from the property prediction model 500 may be also provided to the answer 1221, and the information on the third label M3 assigned to the third molecular structure may also be displayed to the surrounding region of the molecular structure image 1713a of the third molecular structure.

Meanwhile, the answer generation system 100 may receive a new user query including the third label M3 assigned to the third molecular structure through the service page 1000.

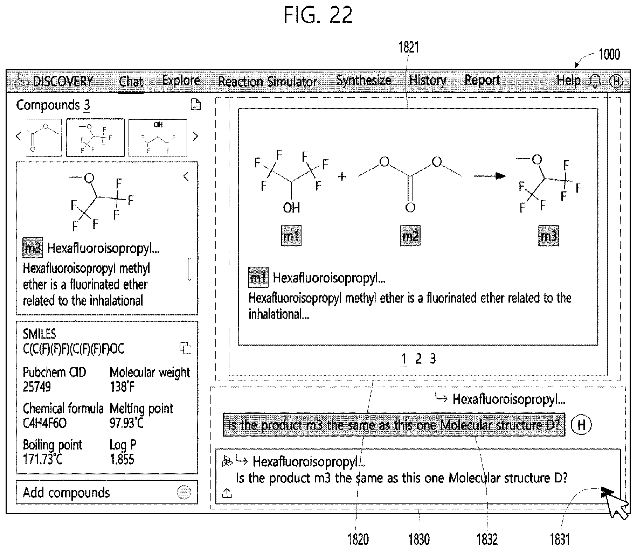

Specifically, the answer generation system 100 may receive a new user query including the third label M3 assigned to the third molecular structure through the third region of the service page 1000. For example, as illustrated in FIG. 22, the answer generation system 100 may receive a new user query 1832 (e.g., "Is the product m3 the same as this one Molecular structure D?") including the third label M3 assigned to the third molecular structure from the user terminal 10 based on a selection of a graphic object 1831 included in a third region 1830.

The answer generation system 100 may input the new user query 1832 to the ultra-large foundation model 200 based on receiving the new user query 1832 including the label M3 assigned to the third molecular structure.

Here, the ultra-large foundation model 200 may specify the fourth molecular structure corresponding to the name of the specific molecular structure and extract the contents related to the fourth molecular structure based on the new user query 1832 including the name (e.g., "Molecular structure D") of the new specific molecular structure instead of the content to which the label is assigned. Then, the answer generation system 100 may group the contents related to the fourth molecular structure, generate fourth content grouped based on the fourth molecular structure, and assign the fourth label M4 to the grouped fourth content. More specific details related to this have been described above, and therefore, it will be described briefly.

The ultra-large foundation model 200 may generate the answer to the new user query 1832 using at least some of the detailed information of the third molecular structure corresponding to the third label M3 and the fourth molecular structure corresponding to the fourth label M4. For example, the ultra-large foundation model 200 may generate an answer to the new user query 1832 using at least some of the information included in the grouped third content to which the third label M3 is assigned and the grouped fourth content to which the fourth label M4 is assigned.

In this case, the ultra-large foundation model 200 may utilize at least one prediction model to generate the answer to the new user query 1832. For example, the ultra-large foundation model 200 may understand the contents included in the new user query 1832 and input the information on the third content grouped based on the third molecular structure and the fourth content grouped based on the fourth molecular structure to the chemical reaction prediction model 400 based on the fact that the user query 1832 includes the contents "Do m3 and molecular structure D have the same structure?"

The chemical reaction prediction model 400 may compare the connection structures of the third molecular structure and the fourth molecular structure corresponding to the new user query 1832. The chemical reaction prediction model 400 may output the comparison results for the third molecular structure and the fourth molecular structure as the output data.

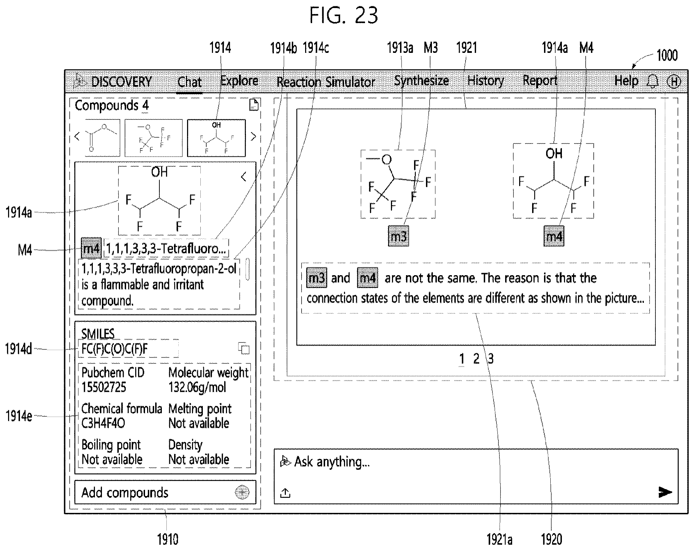

Meanwhile, the ultra-large foundation model 200 may determine the answer generation procedure performed for prediction corresponding to the new user query 1832 and the tool used in the answer generation procedure, and generate an answer 1921 (e.g., "m3 and m4 are connected in different relationships...") to the user query 1832 using the determined answer generation procedure and tool, the output data of the chemical reaction prediction model 400, and the information on the third molecular structure and the fourth molecular structure (see FIG. 23).

When the answer 1921 is generated, as illustrated in FIG. 23, the answer generation system 100 may provide the answer 1921 to the user query 1832 generated from the ultra-large foundation model 200 to a second region 1920 of the service page 1000.

In this case, the answer 1921 provided to the second region 1920 of the service page 1000 may include contents indicating the results of comparison of the connection structures between the plurality of molecular structures (e.g., the third molecular structure and the fourth molecular structure). For example, the answer 1921 may include at least one of labels M3 and M4 assigned to each of the third molecular structure and the fourth molecular structure, molecular structure images 1913a and 1914a of each of the third molecular structure and the fourth molecular structure, and contents 1921a describing the results of comparison of connection structures between the third molecular structure and the fourth molecular structure.

Furthermore, the first region 1910 of the service page 1000 may include at least one of a fourth graphic object 1914 corresponding to the fourth molecular structure, the molecular structure image 1914a of the fourth molecular structure linked to the fourth graphic object 1914, a name 1914b of the fourth molecular structure, a description 1914c of the fourth molecular structure, a SMILES notation 1914d for the fourth molecular structure, and a property 1914e of the fourth molecular structure.

Meanwhile, the service pages provided by the answer generation system 100 may be provided to a user in various forms in addition to the form of the service page 1000 described above. Hereinafter, the service pages of the answer generation system 100 provided in various forms will be described in more detail.

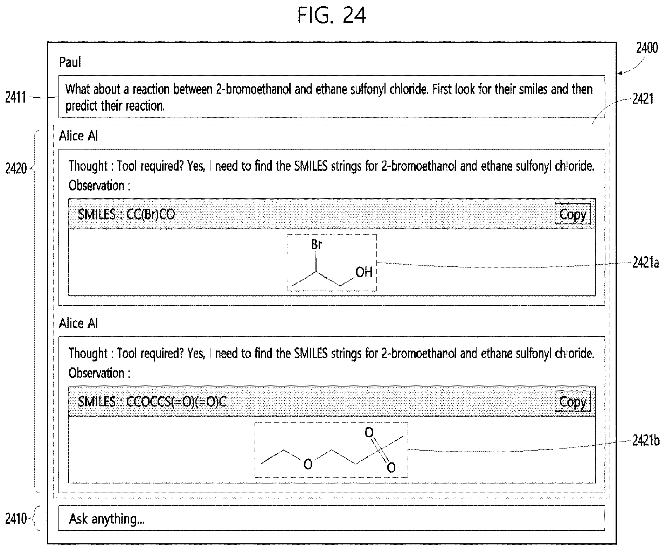

In an embodiment, as illustrated in FIG. 24, the answer generation system 100 may provide a service page 2400 linked to the system 100 to the user terminal 10.

The service page 2400 may include at least one of a first region 2410 for receiving an answer to a user query and a second region 2420 for providing an answer to a user query. The answer generation system 100 may receive a user query 2411 (e.g., "What about a reaction between 2-bromoethanol and ethane sulfonyl chloride. First look for their smiles and then predict their reaction.") including information on at least one molecule input through the first region 2410 of the service page 2400.

Based on the fact that user query 2411 includes the contents "What is a reaction between a first compound (2-bromoethanol) and a second compound (ethane sulfonyl chloride)? First, find their SMILES strings," the ultra-large foundation model 200 may extract SMILES strings for each of the first compound and the second compound from the storage unit 140 and input information on the first compound and the second compound to the chemical reaction prediction model 400. The chemical reaction prediction model 400 may predict the results of the chemical reaction between the first molecular structure and the second molecular structure under specific conditions and output the predicted results.

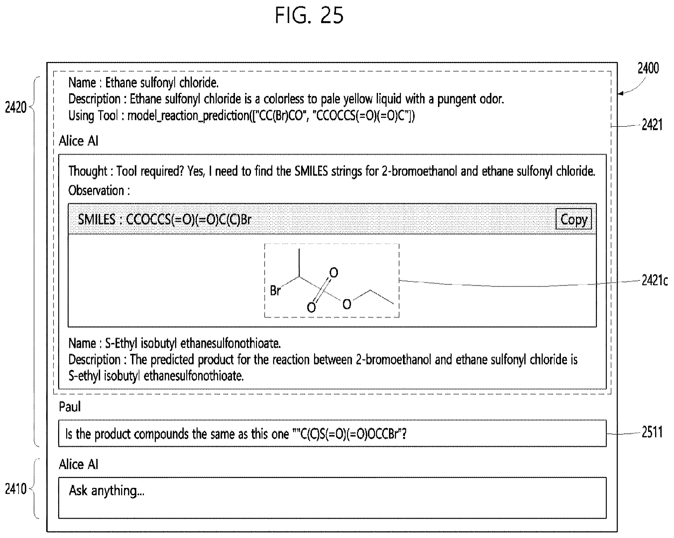

Furthermore, as illustrated in FIGS. 24 and 25, the ultra-large foundation model 200 may generate an answer 2421 (e.g., "The predicted product for the reaction of 2-bromoethanol and ethane sulfonyl chloride is S-ethyl isobutyl ethanesulfonothioate. This organic compound is commonly used as a pesticide. This reaction is a process in which a sulfonyl chloride group of ethane sulfonyl chloride substitutes a hydroxyl group of 2-bromoethanol, thereby forming sulfonothioester.") to the user query 2411 using the predicted results of the chemical reaction between the first molecular structure and the second molecular structure output from the chemical reaction prediction model 400. For example, the answer 2421 may include the molecular structure image (or molecular graphs 2421a and 2421b) of each of the specified first molecular structure and second molecular structure, and detailed information (e.g., SMILES string) for each of the molecular structures. In addition, the answer 2421 may include a molecular structure image 2421c of a product generated as the predicted results of the chemical reaction, and detailed information (e.g., SMILES string) for the product (or molecular structure).

Next, the answer generation system 100 may receive a new user query including the information on the product generated as the predicted results of the chemical reaction. For example, as illustrated in FIG. 25, the answer generation system 100 may receive a user query 2511 (e.g., "Is the generated compound identical to this compound?") including the information on the new product input through the first region 2410 of the service page 2400.

The ultra-large foundation model 200 may input the information on the product included in the new user query 2511 and a target compound for comparison to the chemical reaction prediction model 400 based on the contents included in the new user query 2511. The chemical reaction prediction model 400 may compare the molecular structure of the new product corresponding to the new user query 2511 and the connection structure of the molecular structure of the target compound for comparison, and output the comparison result as the output data.

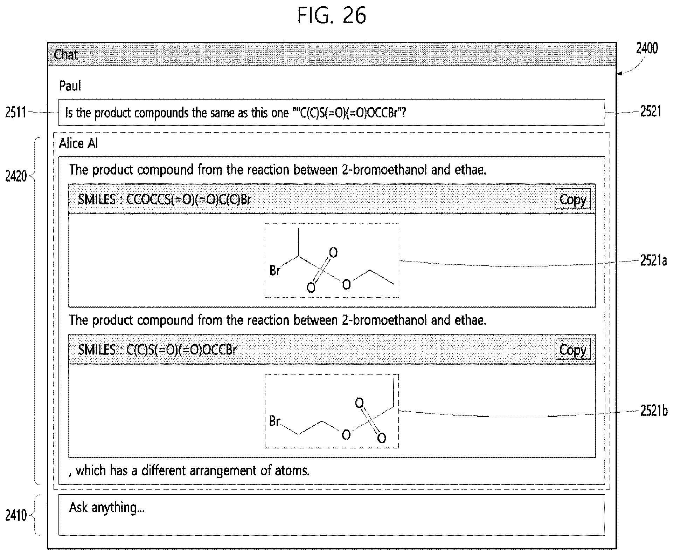

The ultra-large foundation model 200 may generate the answer to the user query 2511 using the output data of the chemical reaction prediction model 400. For example, as illustrated in FIG. 26, the ultra-large foundation model 200 may generate an answer 2521 (e.g., "The two compounds have different arrangements of atoms.") to the user query 2511 and provide the generated answer 2521 through the service page 2400. In this case, the answer 2521 may include contents (e.g., the molecular structure image (or molecular graph 2521a) of the new product and the molecular structure image 2521b of the comparison compound, the SMILES strings of each of the new product and the comparison compound, etc.) indicating the comparison result of the molecular structure of the new product and the molecular structure of the comparison target compound.

Meanwhile, the service page linked to the answer generation system 100 may include various functions and be provided to the user.

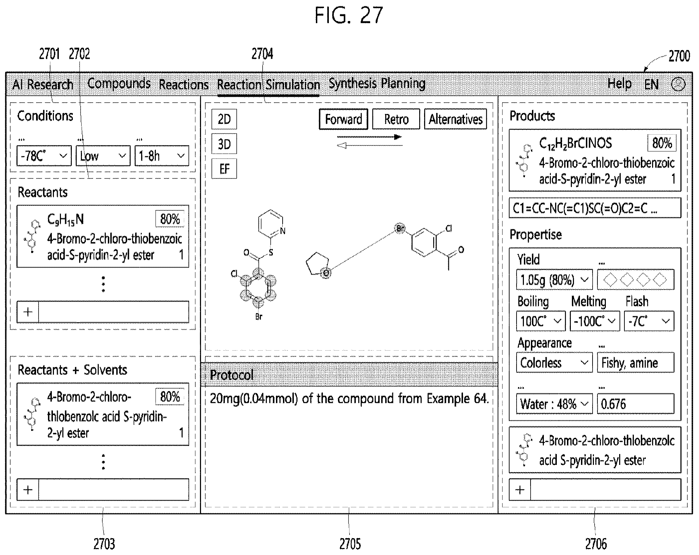

In an embodiment, as illustrated in FIG. 27, a service page 2700 may include at least one of a first region 2701 that includes a function for setting various reaction conditions (or experimental conditions) such as temperature and pressure, a second region 2702 that includes a function for selecting a reactant to be used for predicting the chemical reaction, and a third region 2703 that includes a function for selecting a reagent and/or solvent required for the reaction.

In another embodiment, the service page 2700 may include a fourth region 2704 that includes a function of deriving a direction in which a reaction proceeds or a starting material when a user selects at least one of a forward reaction simulation or a reverse reaction simulation. In addition, the service page 2700 may include a fifth region 2705 that comprehensively provides experimental protocols, conditions, compound information, reaction types, etc.

In another embodiment, the service page 2700 may include a sixth region 2706 that provides information on main products predicted as the simulation result, information on a yield predicted according to the reaction conditions, and information on the predicted by-products and impurities.

In this way, the present invention may input reactants, reagents, and experimental conditions, predict the reaction results using text data, and provide the predicted products, yields, and by-products in a visualized manner. As a result, according to the present invention, it is possible to contribute to users discovering efficient synthetic routes and setting optimal experimental conditions.

Meanwhile, as described above, the user may have the user account pre-registered in the answer generation system 100 according to the present invention.

Accordingly, various types of information related to the user account may be stored in the storage unit 140 of the answer generation system 100. Here, the information related to the user account may include at least one of the user's (or user account's) history information and user's metadata (e.g., name, gender, age, major, occupation, workplace (or company), etc.).

More specifically, the user's history information may include information related to various events that are performed in the user account. For example, the events that have been performed in the user account may include at least one of: i) inputting a user query to acquire an answer from the ultra-large foundation model 200, ii) inputting (or selecting) a document, iii) inputting a new (or additional) query for an answer generated from the ultra-large foundation model 200.

Based on these events, the user's history information may include at least one of: i) the user query input by the user, ii) the document information (or user's document input history) input by the user, iii) content (e.g., a specific molecular structure and information related to the specific molecular structure) extracted from a document input by the user, and iv) an answer from the ultra-large foundation model 200 to the user query.

Accordingly, the storage unit 140 of the answer generation system 100 may store at least one of the analysis target document, the extracted molecular structure, the label for (or assigned to) the extracted molecular structure, the user query, and the answer to the user query by being linked with the user account.

Here, the information related to the extracted molecular structure may be i) the information extracted from the analysis target document, or ii) the information extracted from the user query. Alternatively, the information related to the extracted molecular structure may be i) the information generated from the chemical reaction prediction model 400, or ii) the information generated from the molecular property prediction model 500.

Furthermore, the storage unit 140 of the answer generation system 100 may store various types of information related to at least one of the chemistry, the bio, the new materials, the new substances, and the new drug development. For example, the storage unit 140 may store data such as text, molecular structure, formula, chart, table, and image related to at least one of the chemistry, the bio, the new materials, the new substances, and the new drug development.

Accordingly, the present invention may provide a user environment that allows a user to use various functions by using the DB constructed in the answer generation system 100.

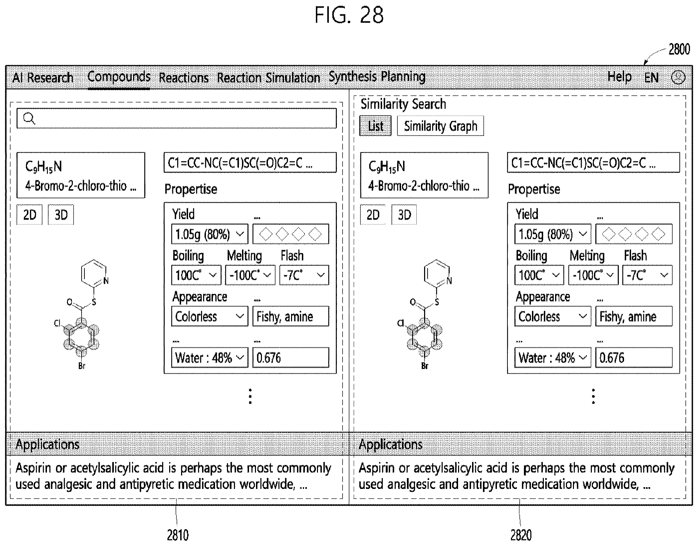

As illustrated in FIG. 28, the answer generation system 100 may provide, to the user terminal 10, a service page 2800 which is configured to explore physical and chemical properties of a specific compound (or product or molecule) or to predict missing properties, by using various types of information stored in the storage unit 140.

In an embodiment, detailed information on a compound selected from the user terminal 10 may be provided to a first region 2810 of the service page 2800. For example, physical and chemical properties, etc., along with the structure of the compound may be provided to the first region 2810 of the service page 1000.

In another embodiment, the second region 2820 of the service page 2800 may include a function that allows a user to search for similar compounds. For example, the answer generation system 100 may extract information on a compound structurally similar to a specific compound from the storage unit 140 based on receiving a search request for the compound structurally similar to the specific compound from the user terminal 10 through the second region 2820, and provide information on the extracted similar compound to the second region 2820.

In addition, the present invention may provide a user environment that allows a user to explore various chemical reaction data or explore chemical reaction data similar to a specific chemical reaction based on various chemical reaction data stored in the storage unit 140.

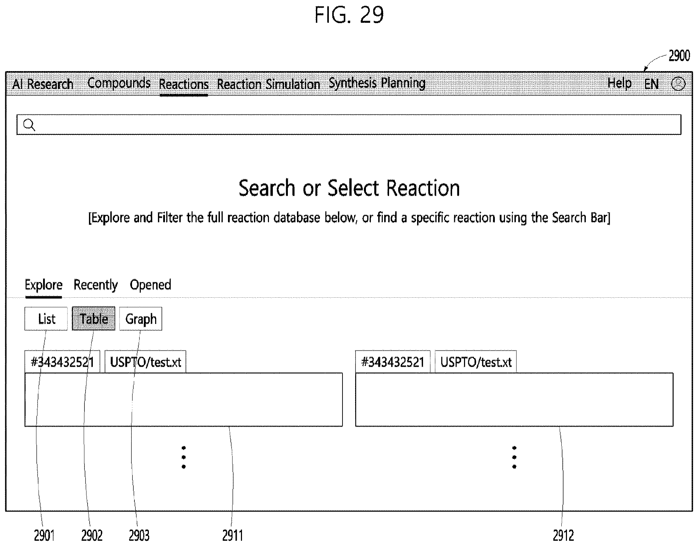

In an embodiment, as illustrated in FIG. 29, the answer generation system 100 may provide a service page 2900 configured to allow a user to explore various chemical reaction data to the user terminal 10. The user may explore data on various chemical reactions through the service page 2900, and the chemical reaction data may include, for example, at least one of reactants, products, and reaction conditions.

In this case, the answer generation system 100 may provide various chemical reaction data by visualizing the various chemical reaction data as options according to the user selection. For example, one region of the service page 2900 may include graphic objects 2901, 2902, and 2903 linked to various visualization options.

In an embodiment, when the first graphic object 2901 is selected from the user terminal 10, the answer generation system 100 may provide various chemical reaction data 2911 and 2912 stored in the storage unit 140 in a list format.

In another embodiment, when the second graphic object 2902 is selected from the user terminal 10, the answer generation system 100 may provide various chemical reaction data 2911 and 2912 stored in the storage unit 140 in a table format.

In another embodiment, when the third graphic object 2903 is selected from the user terminal 10, the answer generation system 100 may provide various chemical reaction data 2911 and 2912 stored in the storage unit 140 in a graph format.

Furthermore, the present invention may provide a user environment that allows a user to perform chemical synthesis research and experiments more efficiently and systematically.

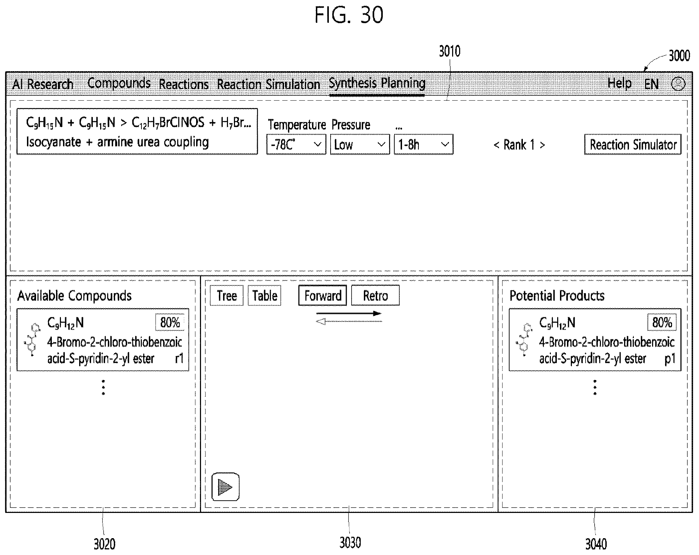

As illustrated in FIG. 30, the answer generation system 100 may provide a service page 3000 that allows a user to conduct research on various synthetic paths related to forward and reverse synthesis. In this case, the service page 3000 may support both forward and reverse simulations, and may include functions that provide information on various paths by which compounds may be synthesized, provide information on synthesizable compounds having specific properties, or allow a user to select a compound having the desired physical and chemical properties, and plan a synthetic path.

In an embodiment, a reaction path between the selected compounds may be provided to a first region 3010 of the service page 3000. A reaction mechanism including major products and by-products may be provided to the first region 3010 of the service page 3000 in a visualized manner.

In another embodiment, a second region 3020 of the service page 3000 may arrange and provide a list of synthesizable compounds, and may include a function for adding reagents and solvents required for the reaction.

In another embodiment, various reaction paths may be provided to a third region 3030 of the service page 3000 in a visualized manner. In this case, compounds generated along a specific reaction path may be visualized step by step and provided to the third region 3030. Furthermore, the information on the major products and by-products predicted as the simulation results may be provided to a fourth region 3040 of the service page 3000, and information on predicted yields, detailed information on major products and reaction conditions, expected impurities, and the amount of impurities may be provided according to each reaction path.

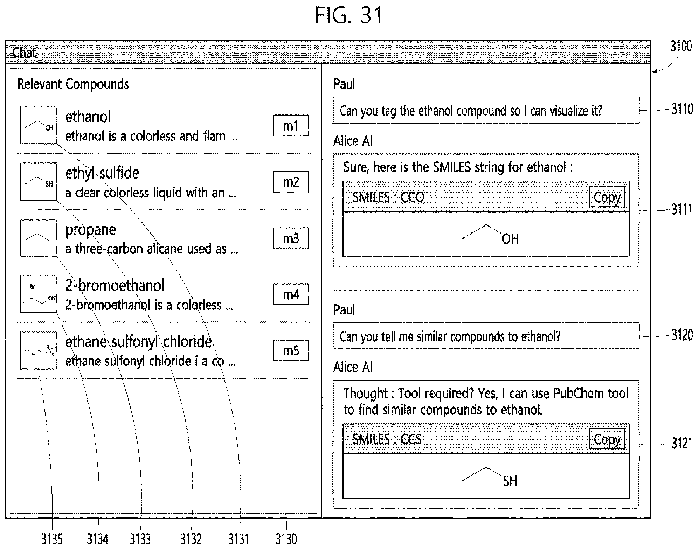

Meanwhile, the service page in which the user query is received may provide information on at least one molecular structure to which the label is assigned. For example, as illustrated in FIG. 31, a plurality of graphic objects 3131, 3132, 3133, 3134, and 3135 corresponding to the plurality of molecular structures each including different labels M1, M2, M3, M4, and M5 may be provided to a third region 3130 of a service page 3100. In this case, the plurality of graphic objects 3131, 3132, 3133, 3134, and 3135 may include the molecular structure image of the molecular structure or the molecular graph for the molecular structure.

In an embodiment, the answer generation system 100 may visualize and provide the first compound as an answer 3111 to a user query 3110 based on the user query 3110 "Please visualize the first compound (e.g., ethanol)." received from the user terminal 10. In this case, the answer generation system 100 may assign the first label M1 to the molecular structure 3131 of the first compound based on the fact that the molecular structure of the first compound is included in the answer 3111, and update the molecular structure 3131 of the first compound to which the first label M1 is assigned in the third region 3130.

In another embodiment, the answer generation system 100 may visualize and provide a molecular structure of a compound similar to the molecular structure of the first compound as an answer 3121 to the user query 3120 based on a user query 3120 "Please visualize and provide a compound similar to the molecular structure of the first compound" received from the user terminal 10. In this case, the answer generation system 100 may assign the second label M2 to the molecular structure 3132 of the new compound based on the fact that the answer 3121 includes the molecular structure of the new compound (or the second compound), and update the molecular structure 3132 of the new compound to which the second label M2 is assigned in the third region 3130.

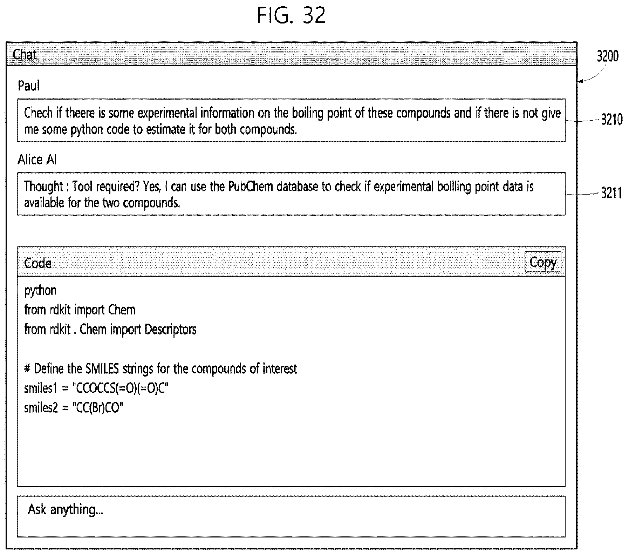

Furthermore, as illustrated in FIG. 32, the answer generation system 100 may generate a Python code corresponding to the user query based on the user query 3210 including the contents "Please check whether there is experimental information on boiling points of compounds, and if not, provide a Python code for estimating the boiling points of the two compounds," received through the service page 3200, and provide an answer 3211 including the Python code.

That is, the user may receive the Python code to inquire about the experimental information on the boiling point of the compound or to acquire the estimated value when there is no experimental data.

As described above, according to the answer generation method and system according to the present invention, by generating and providing the answer suitable for the user's query based on the data extracted from the document, it is possible for the user to minimize the risk of research failure by receiving suggestions for the optimal research method.

In addition, according to the answer generation method and system according to the present invention, it is possible to provide the answer to the user query using the data that is extracted from the document or generated from the pre-trained prediction model. Accordingly, the user can quickly and accurately be provided with his/her required information and reduce the time and/or cost of research and/or development.

Furthermore, according to the answer generation method and system according to the present invention, it is possible to generate the answer to the user query using the predicted results from the pre-trained prediction model and provide the generated answer to the user. Accordingly, it is possible for the user to shorten the time required for research and/or development and reduce the number of trial and errors in research and/or development.

Furthermore, according to the answer generation method and system according to the present invention, by visualizing and providing the extracted molecular structure and related data through the user interface, it is possible for the user to intuitively recognize his/her required information and understand the information more quickly, thereby increasing the accuracy and efficiency of the research.

Meanwhile, the present invention described above may be implemented as a program that is executed by one or more processes on a computer and can be stored on a computer-readable medium (or recording medium).

Furthermore, the present invention described above can be implemented as a computer-readable code or instructions on a medium in which a program is recorded. The present invention may be provided in the form of a program.

Meanwhile, the computer-readable medium may include all kinds of recording devices in which data that may be read by a computer system are stored. Examples of the computer-readable medium include a hard disk drive (HDD), a solid state disk (SSD), a silicon disk drive (SDD), a read only memory (ROM), a random access memory (RAM), a compact disk (CD)-ROM, a magnetic tape, a floppy disk, an optical data storage device, and the like.

Furthermore, the computer-readable medium may include a storage and may be a server or a cloud storage that an electronic device may access through communication. In this case, the computer may download the program according to the present invention from the server or cloud storage through wired or wireless communication.

Furthermore, in the present invention, the computer described above is an electronic device equipped with a processor, that is, a central processing unit (CPU), and the type of electronic device is not particularly limited.

Meanwhile, the above-described detailed description is to be interpreted as being illustrative rather than being restrictive in all aspects. The scope of the present invention is to be determined by reasonable interpretation of the claims, and all modifications within an equivalent range of the present invention fall in the scope of the present invention.

## Claims

1. An answer generation method performed by cooperation by a memory and at least one processor, the answer generation method comprising:
specifying a plurality of molecular structures to be predicted based on user input received through a service page;
storing information related to the plurality of molecular structures in the memory;
acquiring molecular graphs for the plurality of molecular structures by converting atoms into nodes and bonds between the atoms into edges based on the plurality of molecular structures stored in the memory;
receiving a user query for chemical reaction prediction related to the plurality of molecular structures through the service page;
processing the molecular graphs for the plurality of molecular structures as an input of a chemical reaction prediction model so that a chemical reaction corresponding to the user query is predicted;
performing chemical reaction prediction on the plurality of molecular structures in the chemical reaction prediction model;
acquiring a product of the chemical reaction prediction for the plurality of structures from the chemical reaction prediction model; and
generating an answer to the user query using the product of the chemical reaction prediction.

2. The answer generation method of claim 1, wherein the chemical reaction prediction model includes:
a first module that receives the molecular structure and predicts a graph-based chemical reaction; and
a second module that analyzes information related to chemical reaction prediction related to the plurality of molecular structures from text data, and
in the performing of the chemical reaction prediction, the product according to the chemical reaction prediction is generated using the prediction result of the first module and the analysis result of the second module.

3. The answer generation method of claim 2, wherein in the performing of the chemical reaction prediction, the product predicted through the first module is verified using the output data analyzed in the second module.

4. The answer generation method of claim 1, wherein the plurality of molecular structures include a first molecular structure and the second molecular structure,
in the acquiring of the molecular graphs for the plurality of molecular structures, atoms constituting the first molecular structure are converted into nodes and a bond relationship between atoms constituting the first molecular structure is converted into edges to acquire a first molecular graph including the nodes and edges corresponding to the first molecular structure, and
atoms constituting the second molecular structure are converted into nodes and a bond relationship between atoms constituting the second molecular structure is converted into edges to acquire the second molecular graph including the nodes and edges corresponding to the second molecular structure.

5. The answer generation method of claim 4, further comprising:
performing labeling so that different labels are assigned to each of the plurality of molecular structures; and
storing, in the memory, information related to the first molecular graph acquired through the process of acquiring the molecular graph and a label assigned to the first molecular graph, and information related to the second molecular graph acquired through the process of acquiring the molecular graph and a label assigned to the second molecular graph.

6. The answer generation method of claim 5, wherein when the user query including the plurality of molecular structures to which the different labels are assigned is received, in the generating of the answer,
the plurality of molecular structures to which the different labels are assigned are processed as an input of the chemical reaction prediction model,
the product of the chemical reaction prediction acquired from the chemical reaction prediction model is used to generate the answer to the user query, and
when the answer to the user query includes a specific molecular structure generated through the chemical reaction prediction model, the label is assigned to the specific molecular structure.

7. The answer generation method of claim 5, further comprising:
providing, to a region of the service page where the user query is received, a plurality of graphic objects corresponding to each of the plurality of molecular structures to which the different labels are assigned,
wherein each of the plurality of graphic objects includes the first molecule graph and the second molecule graph.

8. The answer generation method of claim 7, wherein based on receiving the user input for selecting one of the plurality of graphic objects, the service page provides detailed information corresponding to a graphic object selected according to the user input.

9. The answer generation method of claim 4, wherein in the performing of the chemical reaction prediction on the plurality of molecular structures,
an embedding vector corresponding to the molecular graph is acquired using the information on the plurality of molecular structures,
an attention operation related to an interaction between the atoms of the plurality of molecular structures is performed, and the embedding vector is updated based on the operation,
bond prediction and atom prediction predicted as a chemical reaction of the plurality of molecular structures are performed using the updated embedding vector, and
the product of the chemical reaction prediction predicted from the chemical reaction of the plurality of molecular structures is acquired using a result of the bond prediction and a result of the atom prediction.

10. The answer generation method of claim 9, wherein the updated embedding vector is updated by adding different biases according to a bond type between the nodes constituting the first molecular graph and the second molecular graph to an attention score calculated according to the result of performing the attention operation.

11. The answer generation method of claim 9, wherein the bond prediction is performed by performing a dot-product using the updated embedding vector,
the atom prediction predicts atom properties of the atoms corresponding to the updated embedding vector by using an atom property probability distribution of each of the atoms corresponding to the updated embedding vector, and
the atom properties include a charge state of the atoms that change during a chemical reaction process of the plurality of molecular structures.

12. The answer generation method of claim 11, wherein the product of the chemical reaction prediction corresponds to a final product stabilized through a diffusion feedback process.

13. The answer generation method of claim 7, wherein the service page includes at least one of:
a first region in which the plurality of graphic objects corresponding to each of the plurality of molecular structures assigned with the different labels and detailed information on the plurality of molecular structures are provided,
a second region in which the answer to the user query is provided, and
a third region in which the user query is received, and
the detailed information on the plurality of molecular structures includes at least one of a molecular structure image, a name, a property, and a string according to a SMILES notation of each of the plurality of molecular structures,
the detailed information is extracted from the user input or acquired from at least one pre-trained prediction model, and
the pre-trained prediction model includes at least one of a chemical reaction prediction reaction model that predicts the chemical reaction between the molecular structures and a molecular property prediction model that predicts a property of the molecular structure.

14. The answer generation method of claim 4, further comprising:
receiving an editing request about the plurality of molecular structures through the service page to which the answer to the user query is provided; and
providing an editing interface that provides an editing function for the plurality of molecular structures to the service page.

15. The answer generation method of claim 14, wherein the molecular graphs corresponding to at least one of the first molecular structure and second molecular structure acquired through the molecular graph transformation are provided to the editing interface in an editable state.

16. The answer generation method of claim 15, wherein the editing of the plurality of molecular structures is a deletion or position change of at least one of the nodes corresponding to each of the atoms constituting each of the plurality of molecular structures and the edges indicating the bond relationship of the atoms, or
an addition of a new node corresponding to a new atom or an addition of a new edge that generates a new bond relationship between the atoms.

17. The answer generation method of claim 16, wherein the edited molecular structure among the plurality of molecular structures is stored in the memory,
a new label specifying the edited molecular structure is assigned to the edited molecular structure, and
when a user query including the new label is input to the ultra-large foundation model, the ultra-large foundation model generates an answer using the edited molecular structure corresponding to the new label.

18. An answer generation system, comprising:
a memory and at least one processor,
wherein the memory and the processor cooperate to
specify a plurality of molecular structures to be predicted based on user input received through a service page,
store information related to the plurality of molecular structures in the memory,
acquire molecular graphs for the plurality of molecular structures by converting atoms into nodes and bonds between the atoms into edges based on the plurality of molecular structures stored in the memory,
receive a user query for chemical reaction prediction related to the plurality of molecular structures through the service page,
process the molecular graphs for the plurality of molecular structures as an input of a chemical reaction prediction model so that a chemical reaction corresponding to the user query is predicted,
perform chemical reaction prediction on the plurality of molecular structures in the chemical reaction prediction model,
acquire a product of the chemical reaction prediction for the plurality of structures from the chemical reaction prediction model, and
generate an answer to the user query using the product of the chemical reaction prediction.

19. A program stored on a computer-readable recording medium, executable by one or more processes on an electronic device, the program comprising instructions to execute:
specifying a plurality of molecular structures to be predicted based on user input received through a service page;
storing information on the plurality of molecular structures in the memory;
acquiring molecular graphs for the plurality of molecular structures by converting atoms into nodes and bonds between the atoms into edges based on the plurality of molecular structures stored in the memory;
receiving a user query for chemical reaction prediction related to the plurality of molecular structures through the service page;
processing the molecular graphs for the plurality of molecular structures as an input of a chemical reaction prediction model so that a chemical reaction corresponding to the user query is predicted;
performing chemical reaction prediction on the plurality of molecular structures in the chemical reaction prediction model;
acquiring a product of the chemical reaction prediction for the plurality of structures from the chemical reaction prediction model; and
generating an answer to the user query using the product of the chemical reaction prediction
